# EUROPEAN PATENT SPECIFICATION

(11) **EP 3 924 349 B1**
(45) Date of publication and mention of the grant of the patent: **25.06.2025**
(21) Application number: 20708296.7
(22) Date of filing: 22.01.2020
(51) Int. Cl.: C07D 401/14, C07D 401/04, C07D 403/14, A61K 31/4439, A61K 31/444, A61K 31/4709, A61K 31/497, A61P 29/00, A61P 37/02

(54) **AMINO ACID DERIVATIVES FOR THE TREATMENT OF INFLAMMATORY DISEASES**
AMINOSÄUREDERIVATE ZUR BEHANDLUNG VON ENTZÜNDUNGSKRANKHEITEN
DÉRIVÉS D'ACIDES AMINÉS POUR LE TRAITEMENT DE MALADIES INFLAMMATOIRES

(30) Priority: 22.01.2019 US 201962795549 P; 09.07.2019 US 201962871951 P
(43) Date of publication of application: 22.12.2021
(73) Proprietor: The Roskamp Institute, Sarasota FL 34243 (US)
(72) Inventor: JIN, Chao, Sarasota, FL 34231 (US); PARIS, Daniel, Sarasota, FL 34243 (US); MULLAN, Michael, Sarasota, FL 34236 (US)
(74) Representative: Murgitroyd & Company
(86) International application number: PCT/US2020/014636
(87) International publication number: WO 2020/154420

(56) References cited:
- WO-A1-00/35891
- WO-A1-2010/127058
- KORMOS CHAD M ET AL: "Design, synthesis, and pharmacological evaluation of JDTic analogs to examine the significance of replacement of the 3-hydroxyphenyl group with pyridine or thiophene bioisosteres", BIOORGANIC & MEDICINAL CHEMISTRY, ELSEVIER, NL, vol. 24, no. 16, 15 June 2016 (2016-06-15), pages 3842 - 3848, XP029642323, ISSN: 0968-0896, DOI: 10.1016/J.BMC.2016.06.029

## Description

### Field of the Disclosure

The present disclosure provides certain amino acid derivatives that inhibit nuclear factor-kappa B (NF-kB) activation induced by inflammatory stimuli. Therefore, the compounds of the present disclosure are useful for the treatment of inflammatory disorders such as rheumatoid arthritis, multiple sclerosis, asthma, and inflammatory bowel disease. Also provided are pharmaceutical compositions containing such compounds.

### Background

Inflammation is an important host response to infection or injury. However, dysregulation of this response, with resulting persistent or inappropriate inflammation leads to a broad range of pathological processes. Inflammatory disorders include autoimmune diseases, allergies, asthma, chronic obstructive pulmonary disease and sepsis which are a major cause of illness and death. It is also becoming apparent that low-grade chronic inflammation underlies many diseases, including diabetes, cancer, cardiovascular disease and neurodegenerative disorders. Therefore, identifying new drugs to suppress inflammation is an area of intense interest.

NF-kB is a ubiquitously expressed transcription factor which regulates the expression of genes involved in inflammation and pain. Activation of NF-kB plays a central role in inflammation through its ability to induce transcription of proinflammatory genes (see Tak et al. NF-kB: a key role in inflammatory diseases. J Clin Inv 2001; 107:7-11; and Liu et al. NF-κB signaling in inflammation. Signal Transduct Target Ther. 2017;2. pii: 17023). Synthesis of proinflammatory cytokines, such as TNF-α, IL-1β, IL-6, and IL-8, is mediated by NF-kB, as is the expression of cyclooxygenase 2 (COX-2), inducible nitric oxide synthase (iNOS), adhesion molecules (ICAM-1, E-selectin, and VCAM-1), chemokines (MCP-1, KC, MIP-1) and metalloproteinases. Reciprocally, cytokines such as TNF-α and IL-1β or prostaglandins produced by COX-2 trigger NF-kB activation generating an inflammatory cascade.

NF-kB is activated at sites of inflammation in diverse diseases including rheumatoid arthritis, osteoarthritis, atherosclerosis, multiple sclerosis, asthma, inflammatory bowel disease, diabetes, Parkinson's disease, Alzheimer's disease, amyotrophic lateral sclerosis, osteoporosis, systemic lupus erythematosus, chronic obstructive pulmonary disease, stroke, acute kidney injury, glomerulonephritis, psoriasis, atopic dermatitis, etc (Pai et al. Immune deficiency or hyperactivity-Nf-kappab illuminates autoimmunity. J Autoimmun 2008; 31:245-251; Zhang et al. NFkB in inflammation and renal diseases. Cell Biosci 2015; 5:63; Roman-Blas et al. NF-kB as a potential therapeutic target in osteoarthritis and rheumatoid arthritis. OsteoArthritis and Cartilage 2006; 14:839-848). WO2010/127058 discloses inhibitors of NF-kB.

Corticosteroids have been developed to treat inflammatory conditions and have many side effects that can be mild or serious, especially when corticosteroids are used for extended periods of time. For instance, corticosteroids can cause weight gain, edema, high blood pressure, glaucoma, hypokalemia, cataract, psychiatric disturbances, osteoporosis, and weakening of the immune system. Non-steroidal anti-inflammatory drugs (NSAIDs) which act via inhibition of the cyclooxygenase (COX) isozymes are widely prescribed but the chronic use of NSAIDs is associated with common side effects including cardiovascular events (hypertension, myocardial infarction, stroke, and heart failure), gastrointestinal side effects (diffuse gastritis and discrete ulcers both gastric and duodenal) which can be fatal, renal side effects (interstitial nephritis) as well as hepatic adverse reactions (transaminitis, hepatitis). Biologics for targeting inflammatory cytokines have been developed. Although biologics have been shown to ameliorate inflammatory diseases pathology and progression, their use has been limited due to severe adverse reactions and to the fact that they abrogate host defense against infection (Rider et al. Biologics for Targeting Inflammatory Cytokines, Clinical Uses, and Limitations. Int J Cell Biol. 2016; 2016:9259646).

Therefore, there is a need to develop other anti-inflammatory medications with fewer side effects for chronic usage. The present invention fulfills this and related needs.

### Summary

In one aspect, provided is a compound of Formula (I): 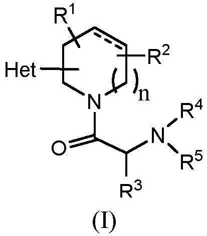 wherein:
n is 0, 1, or 2;
dashed line is an optional bond;
Het is pyridiny-2-yl or pyridin-3-yl;
R¹ is hydrogen or alkyl;
R² is hydrogen or alkyl;
R³ is hydrogen, alkyl, cycloalkyl, cycloalkylalkyl, hydroxyalkyl, alkoxyalkyl, thioalkyl, alkylthioalkyl, aminoalkyl, acylaminoalkyl, carboxyalkyl, alkoxycarbonylalkyl, aminocarbonylalkyl, aryl, aralkyl, heteroaryl, or heteroaralkyl, wherein aryl or heteroaryl by itself or as part of aralkyl or heteroaralkyl is optionally substituted with R^{d}, R^{e}, and/or Rfindependently selected from alkyl, alkoxy, hydroxy, halo, haloalkyl, haloalkoxy, cyano, nitro, carboxy, alkoxycarbonyl, amino, alkylamino, and dialkylamino;
R⁴ is hydrogen or alkyl; and
R⁵ is -C(O)R⁶ where R⁶ is alkyl, aryl, aralkyl, heteroaryl, heteroaralkyl, cycloalkyl, cycloalkylalkyl, heterocycloalkyl, or heterocycloalkylalkyl wherein aryl or heteroaryl by itself or as part of aralkyl or heteroaralkyl is optionally substituted with R^{g}, R^{h}, and/or Rⁱ wherein R^{g}, R^{h}, and/or Rⁱ are each independently selected from alkyl, alkoxy, aminoalkoxy, hydroxyalkoxy, alkoxyalkoxy, cycloalkyloxy, cycloalkylalkyloxy, optionally substituted aryloxy, optionally substituted aralkyloxy, optionally substituted heteroaryloxy, optionally substituted heteroaralkyloxy, optionally substituted heterocycloalkyloxy, optionally substituted heterocycloalkylalkyloxy, halo, haloalkyl, haloalkoxy, cyano, nitro, hydroxy, carboxy, alkoxycarbonyl, amino, alkylamino, dialkylamino, acylamino, and sulfonylamino; or
R⁴ and R⁵ together with the nitrogen atom to which they are attached form a 5 to 7 membered heterocycloamino ring; or
a pharmaceutically acceptable salt thereof;
wherein "alkyl" means a linear saturated monovalent hydrocarbon radical of one to six carbon atoms or a branched saturated monovalent hydrocarbon radical of three to six carbon atoms;
wherein "heteroaryl" means a monovalent monocyclic or bicyclic aromatic radical of 5 to 10 ring atoms, where one or more ring atoms are heteroatom selected from N, O, or S, the remaining ring atoms being carbon;
wherein "optionally substituted aryloxy" means -O-alkylene-R radical where R is optionally substituted aryl;
wherein "aryl" means a monocyclic or bicyclic aromatic hydrocarbon radical of 6 to 10 ring atoms;
wherein "optionally substituted aryl" means aryl as defined above that is optionally substituted with one, two, or three substituents independently selected from alkyl, hydroxyl, cycloalkyl, carboxy, alkoxycarbonyl, hydroxy, alkoxy, alkylthio, alkylsulfonyl, amino, alkylamino, dialkylamino, halo, haloalkyl, haloalkoxy, and cyano;
wherein "optionally substituted aralkyloxy" means -O-alkylene-R radical where R is optionally substituted aryl as defined above;
wherein "optionally substituted heteroaryloxy" means -O-R radical where R is optionally substituted heteroaryl;
wherein "optionally substituted heteroaryl" means heteroaryl as defined above that is optionally substituted with one, two, or three substituents independently selected from alkyl, alkylthio, alkylsulfonyl, hydroxyl, cycloalkyl, carboxy, alkoxycarbonyl, hydroxy, alkoxy, halo, haloalkyl, haloalkoxy, amino, alkylamino, dialkylamino, and cyano;
wherein "optionally substituted heteroaralkyloxy" means -O-alkylene-R radical where R is optionally substituted heteroaryl as defined above;
wherein "optionally substituted heterocycloalkyloxy" means -O-R radical where R is optionally substituted heterocycloalkyl;
wherein "optionally substituted heterocycloalkyl" means heterocycloalkyl that is optionally substituted with one, two, or three substituents independently selected from alkyl, alkylthio, alkylsulfonyl, hydroxyl, cycloalkyl, carboxy, alkoxycarbonyl, hydroxy, hydroxyalkyl, alkoxy, alkoxyalkyl, aminoalkyl, halo, haloalkyl, haloalkoxy, and cyano;
wherein"heterocycloalkyl" means a saturated or unsaturated monovalent monocyclic group of 4 to 8 ring atoms in which one, two, or three ring atoms are heteroatom selected from N, O, or S(O)ₙ, where n is an integer from 0 to 2, the remaining ring atoms being C; wherein one or two ring carbon atoms in the heterocyclyl ring can optionally be replaced by a -CO- group and heterocycloalkyl is optionally fused to phenyl or 5- or 6-membered heteroaryl as defined above;
wherein "optionally substituted heterocycloalkylalkyloxy" means -O-alkylene-R radical where R is optionally substituted heterocycloalkyl as defined above.

In a second aspect, this disclosure is directed to a pharmaceutical composition comprising a compound Formula (I) (or any of the embodiments thereof described herein), or a pharmaceutically acceptable salt thereof; and a pharmaceutically acceptable excipient.
In a third aspect, there is provided a compound of Formula (I) or a pharmaceutically acceptable salt thereof (and any embodiments thereof disclosed herein) for use in treating a disease treatable by inhibiting
(i) NF-kB activation, wherein (i) the disease is an inflammatory disease, optionally wherein said inflammatory disease is selected from the group consisting of autoimmune disease, pain, allergies, asthma, chronic obstructive pulmonary disease and sepsis; or (ii) the diseaseis selected from the group consisting of rheumatoid arthritis, osteoarthritis, atherosclerosis, multiple sclerosis, asthma, inflammatory bowel disease, diabetes, Parkinson's disease, Alzheimer's disease, amyotrophic lateral sclerosis, osteoporosis, systemic lupus erythematosus, chronic obstructive pulmonary disease, cystic fibrosis, stroke, acute kidney injury, glomerulonephritis, psoriasis, atopic dermatitis, Behcet's disease, tuberculosis, Crohn's disease, colitis, Pagett's disease, pancreatitis, periodonitis, inflammatory lung disease, and lupus nephritis.

Also described herein is an intermediate of Formula (II): 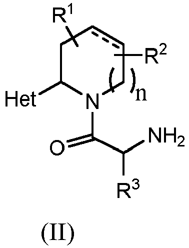 where dashed line, n, Het, R¹, R², and R³ are as defined for Formula (I) above (including embodiments thereof disclosed herein). In one aspect, the intermediate has the structure (IIB): 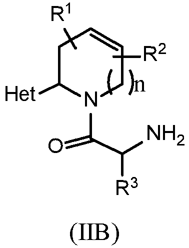 where n, Het, R¹, R², and R³ are as defined for Formula (I) (including embodiments thereof disclosed herein). In another aspect, the intermediate has the structure (IIB'): 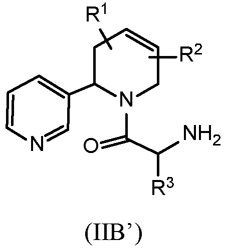 where R¹, R², and R³ are as defined for Formula (I) (including embodiments thereof disclosed herein) and pyridyl is optionally substituted with R^{a}, R^{b}, and/or R^{c}.

In a seventh aspect, provided is a process of making a compound of Formula (IB): 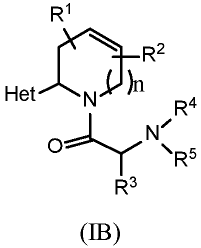 where, n, Het, R¹, R², and R³ are as defined above, R⁴ is hydrogen or alkyl and R⁵ is - COR⁶ where R⁶ is as defined above, comprising:
reacting a compound of formula (IIB): 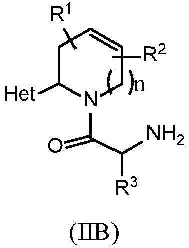 where dashed line, n, Het, R¹, R², and R³ are as defined above;
(i) with a compound of formula R⁶COLG where R⁶ is as defined in Formula (I) above and LG is a leaving group under acylating reaction conditions; or
(ii) with a compound of formula R⁶COOH under amino acid coupling reaction conditions;
(iii) optionally converting the compound of Formula (IB) obtained from step (i) or (ii) to an acid addition salt; or
(iv) optionally converting the compound of Formula (IB) obtained from step (i) or (ii) to the free base.

### Detailed Description

### Definitions:

Unless otherwise stated, the following terms used in the specification and claims are defined for the purposes of this Application and have the following meaning:
"Alkyl" means a linear saturated monovalent hydrocarbon radical of one to six carbon atoms or a branched saturated monovalent hydrocarbon radical of three to six carbon atoms, e.g., methyl, ethyl, propyl, 2-propyl, butyl, pentyl, and the like.
"Alkylene" means a linear saturated divalent hydrocarbon radical of one to six carbon atoms or a branched saturated divalent hydrocarbon radical of three to six carbon atoms unless otherwise stated e.g., methylene, ethylene, propylene, 1-methylpropylene, 2-methylpropylene, butylene, pentylene, and the like.
"Alkylthio" means a -SR radical where R is alkyl as defined above, e.g., methylthio, ethylthio, and the like.
"Alkylsulfonyl" means a -SO₂R radical where R is alkyl as defined above, e.g., methylsulfonyl, ethylsulfonyl, and the like.
"Amino" means a -NH₂.
"Alkylamino" means a -NHR radical where R is alkyl as defined above, e.g., methylamino, ethylamino, propylamino, or 2-propylamino, and the like.
"Aminoalkyl" means a linear monovalent hydrocarbon radical of one to six carbon atoms or a branched monovalent hydrocarbon radical of three to six carbons substituted with -NR'R" where R'and R" are independently hydrogen or alkyl as defined above, e.g., aminomethyl, aminoethyl, methylaminomethyl, and the like.
"Alkoxy" means an -OR radical where R is alkyl as defined above, e.g., methoxy, ethoxy, propoxy, or 2-propoxy, *n-, iso-,* or *tert*-butoxy, and the like.
"Alkoxyalkyl" means a linear monovalent hydrocarbon radical of one to six carbon atoms or a branched monovalent hydrocarbon radical of three to six carbons substituted with at least one alkoxy group, such as one or two alkoxy groups, as defined above, e.g., 2-methoxyethyl, 1-, 2-, or 3-methoxypropyl, 2-ethoxyethyl, and the like.
"Alkoxyalkyloxy" means a -(O)R radical where R is alkoxyalkyl as defined above, e.g., methoxyethoxy, ethoxyethoxy, and the like.
"Aminoalkyloxy" means a -O-alkylene-R radical where R is -NR'R" where R'and R" are independently hydrogen or alkyl as defined above, e.g., aminoethyloxy, methylaminoethyloxy, dimethylaminoethyloxy, diethylaminoethyloxy, and the like.
"Thioalkyl" means a linear monovalent hydrocarbon radical of one to six carbon atoms or a branched monovalent hydrocarbon radical of three to six carbons substituted with a -SH group, e.g., thioethyl, 1-, 2-, or 3-thiopropyl, and the like.
"Alkylthioalkyl" means a linear monovalent hydrocarbon radical of one to six carbon atoms or a branched monovalent hydrocarbon radical of three to six carbons substituted with an alkylthio group as defined above, e.g., 2-methythioethyl, 1-, 2-, or 3-ethylthiopropyl, and the like.
"Alkoxycarbonyl" means a -C(O)OR radical where R is alkyl as defined above, e.g., methoxycarbonyl, ethoxycarbonyl, and the like.
"Alkoxycarbonylalkyl" means a linear monovalent hydrocarbon radical of one to six carbon atoms or a branched monovalent hydrocarbon radical of three to six carbons substituted with an alkoxycarbonyl group as defined above, e.g., 2-methylcarboxyethyl, methylcarboxymethyl, 1-, 2-, or 3-ethylcarboxypropyl, and the like.
"Acyl" means a -C(O)R radical where R is alkyl as defined above, e.g., methylcarbonyl, ethylcarbonyl, and the like.
"Acylamino" means a -NHC(O)R radical where R is alkyl as defined above, e.g., methylcarbonylamino, ethylcarbonylamino, and the like, or R may be optionally substituted with one, two, or three substituents independently selected from substituents as disclosed herein, e.g. alkyl, hydroxyl, cycloalkyl, heterocycloalkyl, carboxy, alkoxycarbonyl, hydroxy, alkoxy, alkylthio, alkylsulfonyl, amino, alkylamino, dialkylamino, halo, haloalkyl, haloalkoxy, and cyano.
"Acylaminoalkyl" means a linear monovalent hydrocarbon radical of one to six carbon atoms or a branched monovalent hydrocarbon radical of three to six carbons substituted with an acylamino group as defined above, e.g., 2-acetylaminoethyl, 1-, 2-, or 3-ethanoylaminopropyl, and the like.
"Aminocarbonylalkyl" means a linear monovalent hydrocarbon radical of one to six carbon atoms or a branched monovalent hydrocarbon radical of three to six carbons substituted with an -CONRR' group where R is hydrogen or alkyl and R' is hydrogen, alkyl, optionally substituted aryl, optionally substituted heteroaryl, or R and R' together with the nitrogen atom to which they are attached form heterocycloamino, e.g., aminocarbonylethyl, methylaminocarbonylethyl, methylaminocarbonylmethyl, 1-, 2-, or 3-ethylaminocarbonylpropyl, pyrrolidinylmethyl, piperidinylethyl, and the like.
"Aryl" means a monocyclic or bicyclic aromatic hydrocarbon radical of 6 to 10 ring atoms e.g., phenyl or naphthyl.
"Aralkyl" means a -(alkylene)-R radical where R is aryl as defined above, e.g., benzyl, phenethyl, and the like.
"Cycloalkyl" means a cyclic saturated monovalent hydrocarbon radical of three to ten carbon atoms, e.g., cyclopropyl, cyclobutyl, cyclopentyl, or cyclohexyl, and the like.
"Cycloalkyloxy" means -O- R where R is cycloalkyl as defined above, e.g., cyclopropyloxy, cyclobutyloxy, cyclopentyloxy, and the like.
"Cycloalkylalkyl" means a -(alkylene)-R radical where R is cycloalkyl as defined above, e.g., cyclopropylmethyl, cyclohexylmethyl, and the like.
"Cycloalkylalkyloxy" means -O-cycloalkylalkyl radical as defined above, e.g., cyclopropylmethyloxy, 2-cyclopropylethyloxy, 1-, 2-, 3-cyclobutylpropoxy, and the like.
"Carboxy" means -COOH.
"Carboxyalkyl" means a linear monovalent hydrocarbon radical of one to six carbon atoms or a branched monovalent hydrocarbon radical of three to six carbons substituted with a carboxy group as defined above, e.g., 2-carboxyethyl, 1-, 2-, or 3-carboxypropyl, and the like.
"Dialkylamino" means a -NRR' radical where R and R' are alkyl as defined above, e.g., dimethylamino, methylethylamino, and the like.
"Halo" means fluoro, chloro, bromo, or iodo, preferably fluoro or chloro.
"Haloalkyl" means alkyl radical as defined above, which is substituted with one or more halogen atoms, such as one to five halogen atoms, such as fluorine or chlorine, including those substituted with different halogens, e.g., -CH₂Cl, -CF₃, -CHF₂, -CH₂CF₃, -CF₂CF₃, -CF(CH₃)₂, and the like. When alkyl is substituted with only fluoro, it may be referred to in this Application as fluoroalkyl.
"Haloalkoxy" means a -OR radical where R is haloalkyl as defined above e.g., -OCF₃, -OCHF₂, and the like. When R is haloalkyl where the alkyl is substituted with only fluoro, may be referred to in this Application as fluoroalkoxy.
"Hydroxyalkyl" means a linear hydrocarbon radical of one to six carbon atoms or a branched monovalent hydrocarbon radical of three to six carbons substituted with one or two hydroxy groups, provided that if two hydroxy groups are present they are not both on the same carbon atom. Representative examples include, but are not limited to, hydroxymethyl, 2-hydroxyethyl, 2-hydroxypropyl, 3-hydroxypropyl, 1-(hydroxymethyl)-2-methylpropyl, 2-hydroxybutyl, 3-hydroxybutyl, 4-hydroxybutyl, 2,3-dihydroxypropyl, 1-(hydroxymethyl)-2-hydroxyethyl, 2,3-dihydroxybutyl, 3,4-dihydroxybutyl and 2-(hydroxymethyl)-3-hydroxypropyl, preferably 2-hydroxyethyl, 2,3-dihydroxypropyl, and 1-(hydroxymethyl)-2-hydroxyethyl.
"Hydroxyalkyloxy" means -O-R radical where R is hydroxyalkyl as defined above, e.g., hydroxymethyloxy, 2-hydroxyethyloxy, 1-, 2-, 3-hydroxypropoxy, and the like.
"Heterocycloalkyl" or "heterocyclyl" means a saturated or unsaturated monovalent monocyclic group of 4 to 8 ring atoms in which one, two, or three ring atoms are heteroatom selected from N, O, or S(O)ₙ, where n is an integer from 0 to 2, the remaining ring atoms being C. Additionally, one or two ring carbon atoms in the heterocyclyl ring can optionally be replaced by a -CO- group and heterocycloalkyl is optionally fused to phenyl or 5- or 6-membered heteroaryl as defined above. More specifically the term heterocyclyl includes, but is not limited to, pyrrolidino, piperidino, homopiperidino, 2-oxopyrrolidinyl, 2-oxopiperidinyl, morpholino, piperazino, tetrahydro-pyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. Unless otherwise stated, the heterocycloalkyl ring is optionally be substituted with one, two, or three substituents independently selected from alkyl, hydroxyl, alkoxy, amino, alkylamino, and dialkylamino.
"Heterocycloalkylalkyl" means a -(alkylene)-R radical where R is heterocycloalkyl ring as defined above e.g., tetraydrofuranylmethyl, piperazinylmethyl, morpholinylethyl, and the like.
"Heterocycloamino" means a saturated or unsaturated monovalent monocyclic group of 4 to 8 ring atoms in which one or two ring atoms are heteroatom selected from N, O, or S(O)ₙ, where n is an integer from 0 to 2, the remaining ring atoms being C provided that at least one of the ring atoms is N unless stated otherwise. Additionally, one or two ring carbon atoms in the heterocycloamino ring can optionally be replaced by a -CO- group. Heterocycloamino is optionally fused to phenyl or 5- or 6-membered heteroaryl as defined above. More specifically the term heterocyclyl includes, but is not limited to, pyrrolidino, piperidino, homopiperidino, 2-oxopyrrolidinyl, 2-oxopiperidinyl, morpholino, piperazino, thiomorpholino, and the like. Unless otherwise stated, the heterocycloamino ring can optionally be substituted with one, two, or three substituents independently selected from alkyl, halo, hydroxyl, alkoxy, amino, alkylamino, dialkylamino, hydroxyalkyl, alkoxyalkyl, or aminoalkyl, each as defined herein.
"Heteroaryl" means a monovalent monocyclic or bicyclic aromatic radical of 5 to 10 ring atoms, unless otherwise stated, where one or more, (in one embodiment, one, two, or three), ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like. As defined herein, the terms "heteroaryl" and "aryl" are mutually exclusive. When the heteroaryl ring contains 5- or 6 ring atoms it is also referred to herein as 5-or 6-membered heteroaryl.
"Heteroaralkyl" means a -(alkylene)-R radical where R is heteroaryl as defined above, e.g., pyridinylmethyl, and the like. When the heteroaryl ring in heteroaralkyl contains 5- or 6 ring atoms it is also referred to herein as 5-or 6-membered heteroaralkyl.
"Patient" means a mammal, preferably a human.

The present disclosure also includes protected derivatives of compounds of the present disclosure (I). For example, when compounds of the present disclosure contain groups such as hydroxy, carboxy, thiol or any group containing a nitrogen atom(s), these groups can be protected with a suitable protecting groups. A comprehensive list of suitable protective groups can be found in T.W. Greene, Protective Groups in Organic Synthesis, John Wiley & Sons, Inc. (1999). The protected derivatives of compounds of the present disclosure can be prepared by methods well known in the art.

The present disclosure also includes polymorphic forms and deuterated forms of the compound of the present disclosure or a pharmaceutically acceptable salt thereof.

A "pharmaceutically acceptable salt" of a compound means a salt that is pharmaceutically acceptable and that possesses the desired pharmacological activity of the parent compound. Such salts include:
acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or
salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, N-methylglucamine, and the like. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985.

The compounds of the present disclosure may have asymmetric centers. Compounds of the present disclosure containing an asymmetrically substituted atom may be isolated in optically active or racemic forms. It is well known in the art how to prepare optically active forms, such as by resolution of materials. All chiral, diastereomeric, all mixtures of chiral or diasteromeric forms, and racemic forms are within the scope of this disclosure, unless the specific stereochemistry or isomeric form is specifically indicated. It will also be understood by a person of ordinary skill in the art that when a compound is denoted as (R) or (S) stereoisomer, it may contain the corresponding (S) or (R) stereoisomer as an impurity preferably the undesired enantiomer is present in less than about 10%, preferably 5% w/w. About means + or - 10% of initial value.

Certain compounds of the present disclosure can exist as tautomers and/or geometric isomers. All possible tautomers and *cis* and *trans* isomers, as individual forms and mixtures thereof are within the scope of this disclosure. Additionally, as used herein the term alkyl includes all the possible isomeric forms of said alkyl group albeit only a few examples are set forth. Similarly, when the cyclic groups such as aryl, heteroaryl, heterocyclyl are substituted, they include all the positional isomers albeit only a few examples are set forth. Furthermore, all hydrates of a compound of the present disclosure are within the scope of this disclosure.

"Oxo" or "carbonyl" means =(O) group.

"Optionally substituted alkyl" means alkyl as defined above that is optionally substituted with one, two, or three substituents independently selected from, e.g. alkyl, hydroxyl, cycloalkyl, heterocycloalkyl, carboxy, alkoxycarbonyl, hydroxy, alkoxy, alkylthio, alkylsulfonyl, amino, alkylamino, dialkylamino, halo, haloalkyl, haloalkoxy, and cyano.

"Optionally substituted aryl" means aryl as defined above that is optionally substituted with one, two, or three substituents independently selected from alkyl, hydroxyl, cycloalkyl, carboxy, alkoxycarbonyl, hydroxy, alkoxy, alkylthio, alkylsulfonyl, amino, alkylamino, dialkylamino, halo, haloalkyl, haloalkoxy, and cyano.

"Optionally substituted aryloxy" means -O-alkylene-R radical where R is optionally substituted aryl as defined above, e.g., benzyloxy, methoxybenzyloxy, halobenzyloxy, 2-phenethyloxy, and the like

"Optionally substituted aralkyl" means -alkylene-R where R is optionally substituted aryl, each as defined above. "Optionally substituted aralkyloxy" means -O-alkylene-R radical where R is optionally substituted aryl as defined above, e.g., benzyloxy, methoxybenzyloxy, halobenzyloxy, 2-phenethyloxy, and the like.

"Optionally substituted heteroaryl" means heteroaryl as defined above that is optionally substituted with one, two, or three substituents independently selected from alkyl, alkylthio, alkylsulfonyl, hydroxyl, cycloalkyl, carboxy, alkoxycarbonyl, hydroxy, alkoxy, halo, haloalkyl, haloalkoxy, amino, alkylamino, dialkylamino, and cyano.

"Optionally substituted heteroaryloxy" means -O-R radical where R is optionally substituted heteroaryl as defined above, e.g., pyridinyloxy, furanyloxy, thienyloxy, and the like.

"Optionally substituted heteroaralkyloxy" means -O-alkylene-R radical where R is optionally substituted heteroaryl as defined above.

"Optionally substituted heterocycloalkyl" means heterocycloalkyl as defined above that is optionally substituted with one, two, or three substituents independently selected from alkyl, alkylthio, alkylsulfonyl, hydroxyl, cycloalkyl, carboxy, alkoxycarbonyl, hydroxy, hydroxyalkyl, alkoxy, alkoxyalkyl, aminoalkyl, halo, haloalkyl, haloalkoxy, and cyano.

"Optionally substituted heterocycloalkylalkyl" means -alkylene-R radical where R is optionally substituted heterocycloalkyl as defined above, e.g., piperidinylmethyl, pyrrolidinylethyl, piperazin-1-ylethyl, and the like.

"Optionally substituted heterocycloalkyloxy" means -O-R radical where R is optionally substituted heterocycloalkyl as defined above, e.g., piperidinyloxy, pyrrolidinyloxy, tetrahydrofuranyloxy, and the like.

"Optionally substituted heterocycloalkylalkyloxy" means -O-alkylene-R radical where R is optionally substituted heterocycloalkyl as defined above, e.g., piperidinylmethyloxy, pyrrolidinylethyloxy, piperazin-1-ylethyloxy, and the like.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes situations where the heterocyclyl group is substituted with an alkyl group and situations where the heterocyclyl group is not substituted with alkyl.

A "pharmaceutically acceptable carrier or excipient" means a carrier or an excipient that is useful in preparing a pharmaceutical composition that is generally safe, non-toxic and neither biologically nor otherwise undesirable, and includes a carrier or an excipient that is acceptable for veterinary use as well as human pharmaceutical use. "A pharmaceutically acceptable carrier/excipient" as used in the specification and claims includes both one and more than one such excipient.

"Sulfonylamino" means a -NRSO₂R' radical where R is hydrogen or alkyl and R' is alkyl, optionally substituted aryl, optionally substituted aralkyl, optionally substituted heteroaryl, or optionally substituted heteroaralkyl, each as defined above.

"Treating" or "treatment" of a disease includes:
(1) preventing the disease, i.e. causing the clinical symptoms of the disease not to develop in a mammal that may be exposed to or predisposed to the disease but does not yet experience or display symptoms of the disease;
(2) inhibiting the disease, i.e., arresting or reducing the development of the disease or its clinical symptoms; or
(3) relieving the disease, i.e., causing regression of the disease or its clinical symptoms.

A "therapeutically effective amount" means the amount of a compound of the present disclosure and/or a pharmaceutically acceptable salt thereof that, when administered to a patient for treating a disease, is sufficient to effect such treatment for the disease. The "therapeutically effective amount" will vary depending on the compound, the disease and its severity and the age, weight, etc., of the mammal to be treated.

### Embodiments:

### Embodiment 1:

In embodiment 1, the compounds of Formula (I) as defined in the Summary above.

### Embodiment 2:

In embodiment 2, the compounds of Formula (I) have structure (IA): 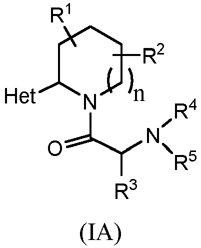 where Het, R¹, R², R³, R⁴, R⁵, and n are as defined in the Summary.

### Embodiment 3:

In embodiment 3, the compounds of Formula (I) have structure (IB): 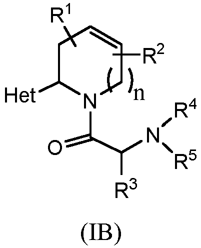 where Het, R¹, R², R³, R⁴, R⁵, and n are as defined in the Summary.

### Embodiment 4:

In embodiment 4, the compounds of any one of embodiments 1, 2 and 3 are those wherein n is 1 or 2.

### Embodiment 5:

In embodiment 5, the compounds of any one of embodiments 1, 2, 3, and 4 are those wherein n is 1.

### Embodiment 6:

In embodiment 6, the compounds of any one of embodiments 1, 2 and 3 are those wherein n is 0 or 2. In one subembodiment of embodiment 6, the compounds of any one of embodiments 1, 2 and 3 are those wherein n is 0. In a second subembodiment of embodiment 6, the compounds of any one of embodiments 1, 2 and 3 are those wherein n is 2.

### Embodiment 7:

In embodiment 7, the compounds of any one of embodiments 1 to 6 and subembodiments contained therein, are those wherein R⁴ is hydrogen or alkyl and R⁵ is -C(O)R⁶ where R⁶ is alkyl, aryl, aralkyl, heteroaryl, heteroaralkyl, cycloalkyl, cycloalkylalkyl, heterocycloalkyl, or heterocycloalkylalkyl wherein aryl or heteroaryl by itself or as part of aralkyl or heteroaralkyl is optionally substituted with R^{g}, R^{h}, and/or Rⁱ independently selected from alkyl, alkoxy, aminoalkoxy, hydroxyalkoxy, alkoxyalkoxy, cycloalkyloxy, cycloalkylalkyloxy, optionally substituted aryloxy, optionally substituted aralkyloxy, optionally substituted heteroaryloxy, optionally substituted heteroaralkyloxy, optionally substituted heterocycloalkyloxy, optionally substituted heterocycloalkylalkyloxy, halo, haloalkyl, haloalkoxy, cyano, nitro, hydroxy, carboxy, alkoxycarbonyl, amino, alkylamino, dialkylamino, acylamino, and sulfonylamino. In one subembodiment of embodiment 7, the compounds of any one of embodiments 1 to 6 are those wherein R⁶ is aryl or heteroaryl (e.g., thienyl, furanyl, pyrrolyl, pyrazolyl, pyridinyl, pyrazinyl, pyrimidinyl, quinolinyl, isoquinolinyl, indolyl, or indazolyl) optionally substituted with R^{g}, R^{h}, and/or Rⁱ as defined above (above as used herein means in embodiment 7). In a second subembodiment of embodiment 7, the compounds of any one of embodiments 1 to 6 are those wherein R⁶ is aralkyl or heteroaralkyl optionally substituted with R^{g}, R^{h}, and/or Rⁱ as defined above. In a third subembodiment of embodiment 7, the compounds of any one of embodiments 1 to 6 are those wherein R⁶ is phenyl optionally substituted with R^{g}, R^{h}, and/or Rⁱ as defined above. In a fourth subembodiment of embodiment 7, the compounds of any one of embodiments 1 to 6 are those wherein R⁶ is heteroaryl (e.g., thienyl, furanyl, pyrrolyl, pyrazolyl, pyridinyl, pyrazinyl, pyrimidinyl, quinolinyl, isoquinolinyl, indolyl, or indazolyl), optionally substituted with R^{g}, R^{h}, and/or Rⁱ as defined above. In a fifth subembodiment of embodiment 7, the compounds of any one of embodiments 1 to 6 and the compounds within subembodiment one to four within embodiment 7, are those wherein R^{g} is alkyl, alkoxy, halo, haloalkyl, haloalkoxy, hydroxy, or cyano and R^{h} and Rⁱ are independently selected from alkyl, alkoxy, aminoalkoxy, hydroxyalkoxy, alkoxyalkoxy, cycloalkyloxy, cycloalkylalkyloxy, optionally substituted aralkyloxy, optionally substituted heteroaryloxy, optionally substituted heterocycloalkyloxy, optionally substituted heterocycloalkylalkyloxy, halo, haloalkyl, haloalkoxy, cyano, hydroxy, carboxy, alkoxycarbonyl, amino, alkylamino, dialkylamino, acylamino, and sulfonylamino. In a sixth subembodiment of embodiment 7, the compounds of any one of embodiments 1 to 6 and the compounds within subembodiments first to fifth within embodiment 7, are those wherein R^{g}, R^{h} and/or Rⁱ are independently selected from alkyl, alkoxy, halo, haloalkyl, haloalkoxy, cyano, hydroxy, amino, acylamino, preferably, methyl, ethyl, methoxy, ethoxy, chloro, fluoro, trifluoromethyl, trifluoromethoxy, hydroxy, acetylamino, butanoylamino, and pentanoylamino.

### Embodiment 8:

In embodiment 8, the compounds of any one of embodiments 1 to 6 and subembodiments contained therein, are those wherein R⁴ and R⁵ together with the nitrogen atom to which they are attached form a 5 to 7 membered heterocycloamino ring. In one subembodiment of embodiment 8, the heterocycloamino ring is pyrrolidino, piperidino, homopiperidino, 2-oxopyrrolidinyl, 2-oxopiperidinyl, morpholino, piperazino, thiomorpholino, isoindolinyl, or 1,3-dioxoisoindolin-2-yl optionally be substituted with one, two, or three substituents independently selected from alkyl, hydroxyl, alkoxy, hydroxyalkyl, alkoxyalkyl, and aminoalkyl.

### Embodiment 9:

In embodiment 9, the compounds of any one of embodiments 1 to 8 and subembodiments contained therein, are those wherein R¹ and R² are independently hydrogen or methyl, preferably hydrogen.

### Embodiment 10:

In embodiment 10, the compounds of any one of embodiments 1 to 9 and subembodiemtns contained therein, are those wherein when both R¹ and R² are alkyl, they are not bound to the same ring carbon.

### Embodiment 11:

In embodiment 11, the compounds of any one of embodiments 1 to 10 and subembodiments contained therein, are those wherein Het is pyridin-2-yl.

### Embodiment 12:

In embodiment 12, the compounds of any one of embodiments 1 to 11 and subembodiments contained therein, are those wherein R³ is hydrogen, alkyl, cycloalkyl, cycloalkylalkyl, hydroxyalkyl, alkoxyalkyl, thioalkyl, alkylthioalkyl, aminoalkyl, acylaminoalkyl, carboxyalkyl, alkoxycarbonylalkyl, aminocarbonylalkyl, aryl, aralkyl, heteroaryl, or heteroaralkyl wherein aryl or heteroaryl by itself or in aralkyl and heteroaralkyl is optionally substituted with R^{d}, R^{e}, and/or R^{f} independently selected from alkyl, alkoxy, hydroxy, halo, haloalkyl, haloalkoxy, cyano, nitro, carboxy, alkoxycarbonyl, amino, alkylamino, or dialkylamino. In one subembodiment of embodiment 12, the compounds of any one of embodiments 1 to 11 are those wherein R³ hydrogen or alkyl, preferably methyl, ethyl, propyl, isopropyl, *sec*-propyl, *n-, sec-, iso-,* or *tert*-butyl.

In a second subembodiment of embodiment 12, the compounds of any one of embodiments 1 to 11 are those wherein R³ is aralkyl optionally substituted with R^{d}, R^{e}, and/or R^{f} as defined above, preferably R³ is benzyl or phenethyl, more preferably benzyl optionally substituted with R^{d}, R^{e}, and R^{f} as defined above, even more preferably benzyl.

In a third subembodiment of embodiment 12, the compounds of any one of embodiments 1 to 11 are those wherein R³ is cycloalkylalkyl optionally substituted with R^{d}, R^{e}, and/or R^{f} as defined above, preferably cyclopropylmethyl, cyclobutylmethyl, cyclopentylmethyl, or cyclohexylmethyl optionally substituted with R^{d}, R^{e}, and R^{f} as defined above.

In a fourth subembodiment of embodiment 12, the compounds of any one of embodiments 1 to 11 are those wherein R³ is heteroaralkyl (e.g., thienylmethyl, furanylmethyl, pyridinylmethyl, quinolinylmethyl, isoquinolinylmethyl, indolylmethyl, or indazolylmethyl) optionally substituted with R^{d}, R^{e}, and/or R^{f} as defined above.

In a fifth subembodiment of embodiment 12, the compounds of any one of embodiments 1 to 11, are those wherein R³ is hydroxyalkyl, alkoxyalkyl, or aminoalkyl, preferably hydroxymethyl, hydroxyethyl, methoxymethyl, methoxyethyl, aminomethyl, or aminobutyl.

In one embodiment, the stereochemistry at the carbon to which R³ is attached is (S).

In another embodiment, the stereochemistry at the carbon to which R³ is attached is (R).

Representative compounds of Formula (I) where R¹ and R² are H, n is 1 and other groups are as indicated in Table 1 below are:

**Table 1**

| 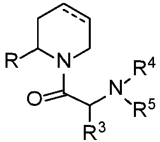 | | | | | | |
|---|---|---|---|---|---|---|
| **Cpd No.** | **R** | **R³** | **R⁴** | **R⁵ = -COR⁶ where R⁶ is:** | 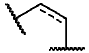 | **NR⁴R⁵** |
| 1 | pyridin-3-yl | S-methyl | | | 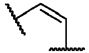 | 1,3-dioxoisoindolin-2-yl |
| 2 | S-pyridin-3-yl | S-methyl | | | 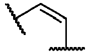 | 1,3-dioxoisoindolin-2-yl |
| 3 | S-pyridin-3-yl | S-isopropyl | | | 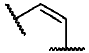 | 1,3-dioxoisoindolin-2-yl |
| 4 | R-pyridin-3-yl | S-isopropyl | | | 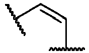 | 1,3-dioxoisoindolin-2-yl |
| 5 | mixture of S-pyridin-3-yl and R-pyridin-3-yl | S-ethyl and R-ethyl | | | 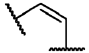 | 1,3-dioxoisoindolin-2-yl |
| 6 | mixture of S-pyridin-3-yl and R-pyridin-3-yl | R-ethyl and S-ethyl | | | 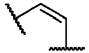 | 1,3-dioxoisoindolin-2-yl |
| 7 | pyridin-3-yl | benzyl | | | 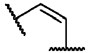 | 1,3-dioxoisoindolin-2-yl |
| 8 | pyridin-3-yl | hydrogen | | | 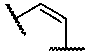 | 1,3-dioxoisoindolin-2-yl |
| 9 | pyridin-3-yl | S-methyl | H | 3-nitrophenyl | 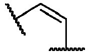 | |
| 10 | pyridin-3-yl | S-methyl | H | 3-methoxyphenyl | 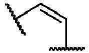 | |
| 11 | pyridin-3-yl | S-methyl | H | 3,4-diethoxyphenyl | 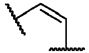 | |
| 12 | pyridin-3-yl | S-methyl | H | 3,4-dimethoxyphenyl | 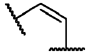 | |
| 13 | pyridin-3-yl | S-methyl | H | 4-nitrophenyl | 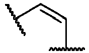 | |
| 14 | pyridin-3-yl | S-methyl | H | 4-chlorophenyl | 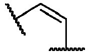 | |
| 15 | pyridin-3-yl | benzyl | H | 3-nitrophenyl | 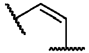 | |
| 16 | pyridin-3-yl | benzyl | H | 3-methoxyphenyl | 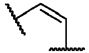 | |
| 17 | pyridin-3-yl | R-methyl | | | 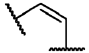 | 1,3-dioxoisoindolin-2-yl |
| 18 | pyridin-3-yl | benzyl | H | 3-chlorophenyl | 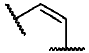 | |
| 19 | pyridin-3-yl | benzyl | H | 3-fluorophenyl | 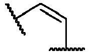 | |
| 20 | pyridin-3-yl | benzyl | H | 3-ethoxyphenyl | 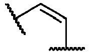 | |
| 21 | pyridin-3-yl | benzyl | H | 3,4-dimethoxyphenyl | 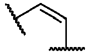 | |
| 23 | pyridin-3-yl | benzyl | H | 3-trifluoromethylphenyl | 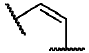 | |
| 24 | pyridin-3-yl | benzyl | H | 3-fluoro-5-methoxyphenyl | 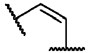 | |
| 25 | pyridin-3-yl | R-benzyl | H | 3-methoxyphenyl | 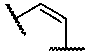 | |
| 26 | pyridin-3-yl | R-benzyl | H | 3-ethoxy-4-methoxyphenyl | 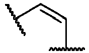 | |
| 27 | pyridin-3-yl | R-benzyl | H | 2-chloro-5-methoxyphenyl | 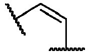 | |
| 28 | pyridin-3-yl | R-benzyl | H | 3,5-dimethoxyphenyl | 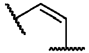 | |
| 29 | pyridin-3-yl | R-benzyl | H | 5-methoxy-2-methylphenyl | 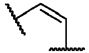 | |
| 30 | pyridin-3-yl | R-benzyl | H | 4-chloro-2-methoxyphenyl | 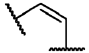 | |
| 31 | pyridin-3-yl | R-benzyl | H | 4-fluoro-3-methoxyphenyl | 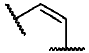 | |
| 32 | pyridin-3-yl | R-benzyl | H | 2-methoxyphenyl | 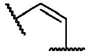 | |
| 33 | pyridin-3-yl | S-benzyl | H | 3-ethoxyphenyl | 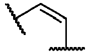 | |
| 34 | pyridin-3-yl | S-benzyl | H | 4-fluoro-3-methoxyphenyl | 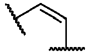 | |
| 35 | pyridin-3-yl | S-benzyl | H | 3-methoxyphenyl | 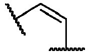 | |
| 36 | pyridin-3-yl | benzyl | H | 3-pentanoylaminophenyl | 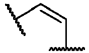 | |
| 37 | pyridin-3-yl | benzyl | H | 3-butanoylaminophenyl | 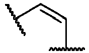 | |
| 38 | pyridin-3-yl | benzyl | H | 3-aminophenyl | 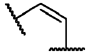 | |
| 39 | pyridin-3-yl | benzyl | H | 3-acetylaminophenyl | 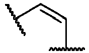 | |
| 40 | quinolin-3-yl | benzyl | H | 3-butyramidophenyl | 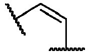 | |
| 41 | pyridin-3-yl | benzyl | H | 3-biotinylaminophenyl | 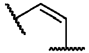 | |
| 42 | pyridin-3-yl | S-benzyl | H | 3-(methylbutanoylamino) -phenyl | 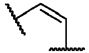 | |
| 43 | pyridin-3-yl | S-benzyl | H | 3-(2,2-dimethylpropanoylami no)-phenyl | 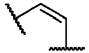 | |
| 44 | pyridin-3-yl | S-benzyl | H | 3-(cyclopropanylmethano ylamino)-phenyl | 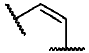 | |
| 45 | pyridin-3-yl | S-benzyl | H | 3-(cyclobutanylmethanoy lamino)-phenyl | 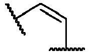 | |
| 46 | pyridin-3-yl | S-benzyl | H | 3-(cyclopentanylmethano ylamino)-phenyl | 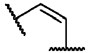 | |
| 47 | pyridin-3-yl | S-benzyl | H | 2-(butanoylamino)-phenyl | 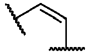 | |
| 48 | pyridin-3-yl | S-benzyl | H | 4-(butanoylamino)-phenyl | 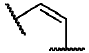 | |
| 49 | pyridin-3-yl | S-benzyl | H | quinolin-2-yl | 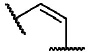 | |
| 50 | pyridin-3-yl | S-benzyl | H | pyrazin-2-yl | 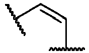 | |
| 51 | pyridin-3-yl | S-benzyl | H | pyridin-3-yl | 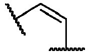 | |
| 52 | pyridin-3-yl | S-benzyl | H | furan-2-yl | 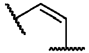 | |
| 53 | pyridin-3-yl | S-benzyl | H | 3-(2-aminoacetamido)-phenyl | 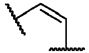 | |
| 54 | pyridin-3-yl | S-benzyl | H | 3-(3-aminopropanoylamino) -phenyl | 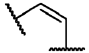 | |
| 55 | pyridin-3-yl | S-benzyl | H | 3-(4-aminobutanoylamino)-phenyl | 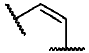 | |
| 56 | pyridin-3-yl | S-benzyl | H | pyrrole-2-yl | 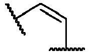 | |
| 57 | pyridin-3-yl | S-benzyl | H | (S)-3-(2-methylbutanoylamino)-phenyl | 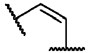 | |
| 58 | pyridin-3-yl | S-benzyl | H | (R)-3-(2-methylbutanoylamino)-phenyl | 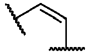 | |
| 59 | pyridin-3-yl | S-benzyl | H | 3-(N-methylbutanoylamino)-phenyl | 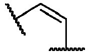 | |
| 60 | pyridin-3-yl | S-benzyl | H | 3-(butylamino)-phenyl | 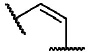 | |
| 61 | pyridin-3-yl | S-benzyl | H | N-butyl-1H-pyrrole-2-yl | 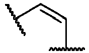 | |
| 63 | pyridin-3-yl | S-benzyl | H | 3-(butanoylamino)-phenyl | 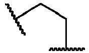 | |
| 64 | pyridin-3-yl | S-naphth-1-yl | H | 3-(butanoylamino)-phenyl | 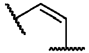 | |
| 65 | pyridin-3-yl | S-naphth-2-yl | H | 3-(butanoylamino)-phenyl | 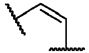 | |
| 66 | pyridin-3-yl | S-cyclohexy l | H | 3-(butanoylamino)-phenyl | 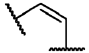 | |
| 67 | 2-chloro-quinolin-3-yl | benzyl | H | 3-(butanoylamino)-phenyl | 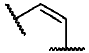 | |
| 68 | quinolin-3-yl | methyl | H | 3-(butanoylamino)-phenyl | 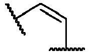 | |

Additional contemplated compounds of Formula (I) are provided in Table 2 below:

**Table 2**

| 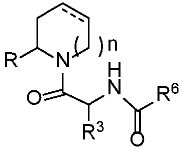 | | | | | |
|---|---|---|---|---|---|
| **Cpd No.** | **R** | **R³** | **R⁶ (R⁵ =COR⁶)** | 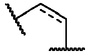 | **n** |
| 101 | pyridin-3-yl | benzyl | 3-butanoylaminophenyl | 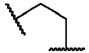 | 0 |
| 102 | pyridin-3-yl | benzyl | 3-butanoylaminophenyl | 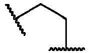 | 2 |
| 103 | pyridin-3-yl | 4-methoxybenzyl | 3-butanoylaminophenyl | 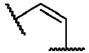 | 1 |
| 104 | pyridin-3-yl | 3,4-dimethoxybenzyl | 3-butanoylaminophenyl | 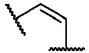 | 1 |
| 105 | pyridin-3-yl | benzyl | tetrahydroquinolin-7-yl | 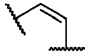 | 1 |
| 106 | pyridin-3-yl | benzyl | indol-6-yl | 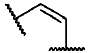 | 1 |
| 107 | pyridin-3-yl | benzyl | 3-propylsulfonylaminophenyl | 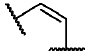 | 1 |
| 108 | pyridin-3-yl | benzyl | 3-isopropoxyphenyl | 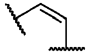 | 1 |
| 109 | pyridin-3-yl | benzyl | 3-cyclopentyloxyphenyl | 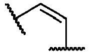 | 1 |
| 110 | pyridin-3-yl | benzyl | 3-(2-acetylaminoethyloxy)phenyl | 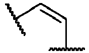 | 1 |
| 111 | pyridin-3-yl | benzyl | 3-(2-dimethylaminoethyloxy)phenyl | 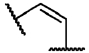 | 1 |
| 112 | pyridin-3-yl | benzyl | 3-(2-pyrrolidin-1-ylethyloxy)phenyl | 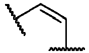 | 1 |
| 113 | pyridin-3-yl | benzyl | 3-(1-methylpiperidin-4-yloxy)phenyl | 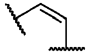 | 1 |
| 114 | pyridin-3-yl | benzyl | 3-[2-(1-methylpiperazin-4-yl)ethyloxy]phenyl | 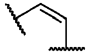 | 1 |
| 115 | pyridin-3-yl | benzyl | 3-(2-morpholin-4-ylethyloxy)phenyl | 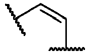 | 1 |
| 116 | pyridin-3-yl | benzyl | pyridin-3-yl | 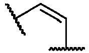 | 1 |
| 117 | pyridin-3-yl | benzyl | 2-aminothiadiazol-5-yl | 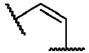 | 1 |
| 118 | pyridin-3-yl | benzyl | thien-2-yl | 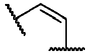 | 1 |
| 119 | pyridin-3-yl | benzyl | 5-chlorothien-5-yl | 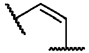 | 1 |
| 120 | pyridin-3-yl | benzyl | 3-(2-methylaminoethyloxy)phenyl | 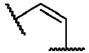 | 1 |

### General Synthetic Scheme

Compounds of this disclosure can be made by the methods depicted in the reaction schemes shown below.

The starting materials and reagents used in preparing these compounds are either available from commercial suppliers such as Aldrich Chemical Co., (Milwaukee, Wis.), Bachem (Torrance, Calif.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991), March's Advanced Organic Chemistry, (John Wiley and Sons, 4th Edition) and Larock's Comprehensive Organic Transformations (VCH Publishers Inc., 1989). These schemes are merely illustrative of some methods by which the compounds of this disclosure can be synthesized, and various modifications to these schemes can be made and will be suggested to one skilled in the art reading this disclosure. The starting materials and the intermediates, and the final products of the reaction may be isolated and purified if desired using conventional techniques, including but not limited to filtration, distillation, crystallization, chromatography and the like. Such materials may be characterized using conventional means, including physical constants and spectral data. Unless specified to the contrary, the reactions described herein take place at atmospheric pressure over a temperature range from about -78 °C to about 150 °C, such as from about 0 °C to about 125 °C and further such as at about room (or ambient) temperature, e.g., about 20°C.

Compounds of Formulae (IA) and (IB) where R¹, R², R³, Het are as defined in the Summary, R⁴ is hydrogen or alkyl and R⁵ is -COR⁶ where R⁶ is as defined in the Summary, can be prepared as described in Scheme 1 below.

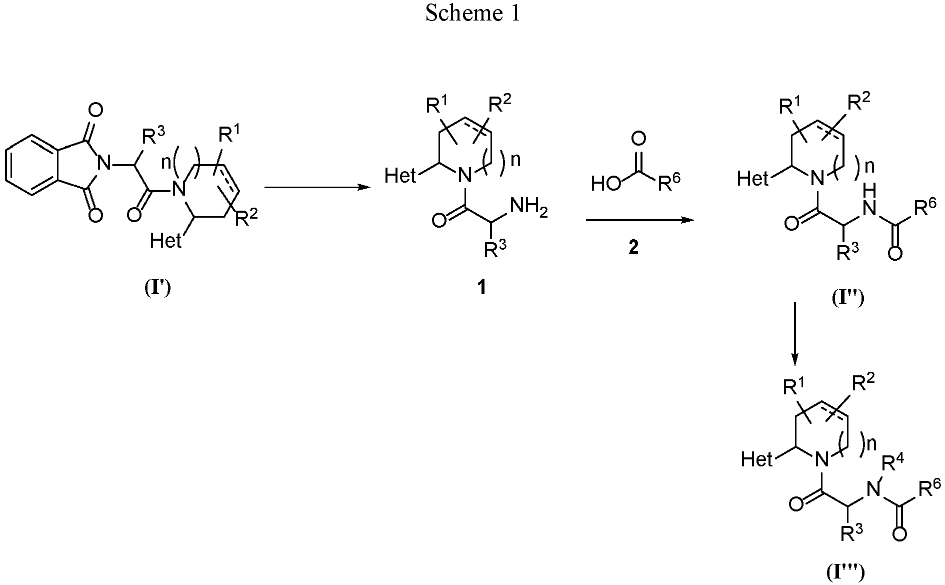

Treatment of compound (I') where R⁴ and R⁵ together with the nitrogen atom form phthalimido ring, prepared as described in Scheme 2 below, with aqueous hydrazine solution provides an amino compound of formula 1. The reaction can be carried out in alcoholic solvents, such as methanol, ethanol or isopropyl alcohol and the like.

Coupling of the compound of formula 1 with an acid of formula 2 where R⁶ is as defined in the Summary in the presence of a coupling agent such as HOBt, EDC, and the like, and a non-nucleophilic based such as triethylamine, diisopropylamine, i-Pr₂NEt and the like, in an organic solvent such as dichloromethane, chloroform, and the like, at room temperature provides a compound (I").

Alternatively, compounds of formula (I) can be prepared by reaction of a compound of formula 1 with an acetyl of formula R⁶CO(LG) where LG is halo, preferably chloro, under acylating reaction conditions.

Compounds of formula 2 are commercially available or they can be prepared by well-known methods in the art from readily available starting materials. For example, benzoic acid, o-, m-, and p-nitrobenzoic acid, phenylacetic acid, trifluorobenzoic acid, picolinic acid, nicotinic acid, methoxybenzoic acids, fluorobenzoic acids, chlorobenzoic acids, hydroxybenzoic acids, 3-fluoro-5-methoxybenzoic acid, 3-aminobenzoic acid, acetic acid, propionic acids, cyclopropanecarboxylic acid, cyclopentanecarboxylic acid are commercially available. Compounds of formula 2 can also be prepared from commercially available compounds. For example, compounds of formula 2 where R⁶ is aryl, aralkyl, heteroaryl, or heteroaralkyl substituted with hydroxy can be reacted with optionally substituted aralkyl halide, optionally substituted heterocycloalkyl halide, and optionally substituted heterocycloalkylalkyl halide under alkylating reaction conditions to give a corresponding compound of formula 2 where R⁶ is substituted with optionally substituted aralkyloxy, optionally substituted heterocycloalkyloxy, optionally substituted heterocycloalkylalkyloxy respectively. Similarly, compounds of formula 2 where R⁶ is substituted with an amino group can be reacted with acid halides or sulfonyl halides under acylating and sulfonylating reaction conditions to give corresponding compounds of formula 2 where R⁶ is substituted with acylamino or sulfonylamino groups respectively.

Alternatively, the above transformations can also be carried out on compounds of Formula (I") or (I‴) where R⁶ is aryl, aralkyl, heteroaryl, or heteroaralkyl substituted with a hydroxy or an amino group.

Compounds of Formulae (IA) and (IB) where dashed line, R¹, R², R³, Het are as defined in the Summary and R⁴ and R⁵ together with the nitrogen atom to which they are attached form a phthalimido ring can be prepared as described in Scheme 2 below.

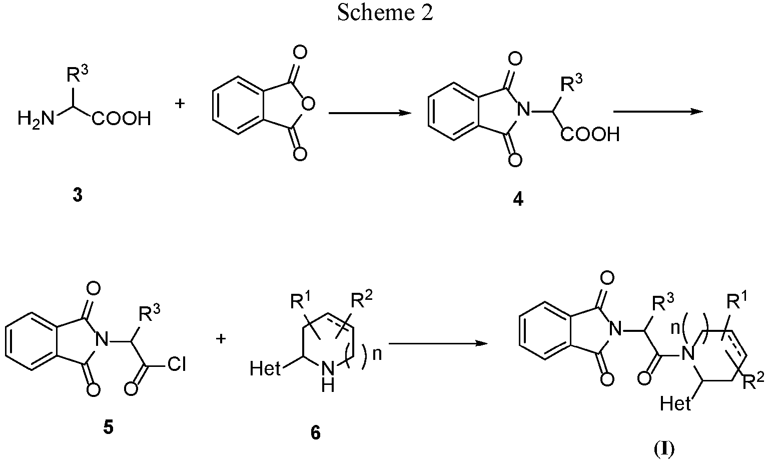

Compounds of formula 4 where R³ is as defined in the Summary, can be prepared by reacting a compound of formula 3 with phthalic anhydride under basic conditions (see J. C. Sheehan, D. W. Chapman, and R. W. Roth, J. Am. Chem. Soc., 74, 3822 (1952)) or acidic conditions (see R. Mahboub, int. J. Chem. Sci, 7(1), 2009, 28-36) at elevated temperatures. When basic reaction conditions are used, the reaction is carried out in the presence of organic bases such as Et₃N, iPr₂Net, and the like in an aromatic organic solvent such as benzene, toluene, and the like. Compounds of formula 3 are commercially available or readily prepared by methods known in the art.

Compound (I) can be prepared by adding a solution of a compound of formula 6 with an acid chloride of formula 5 under acylating reaction conditions. Suitable solvents include aromatic or halogenated organic solvents such as benzene, toluene or DCM in their anhydrous forms.

Compounds of formula 5 can be prepared by reacting a compound of formula 4 with a chlorinating agent such as SOCl₂, (COCl)₂, or PCl₅ under conditions well known in the art. Compounds of formula 6 such as (R)-2-(piperidin-2-yl)pyridine and S-2-(piperidin-2-yl)pyridine are commercially available or they can be prepared by methods well known in the art.

Compounds of Formulae (IA) and (IB) where dashed line, R¹, R², R³, Het are as defined in the Summary and R⁴ and R⁵ together with the nitrogen atom to which they are attached form pyrrolidinyl, piperidinyl, or homopiperidinyl can be prepared as described in Scheme 3 below.

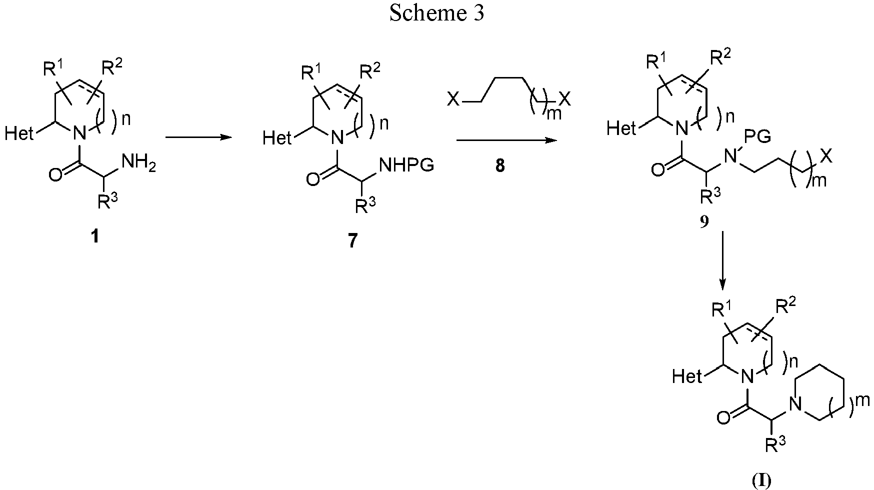

Protection of the amino group in the compound of formula 1 with a suitable amino protecting group such as Boc, benzyl, or Ns, followed by treatment of the resulting compound of formula 7 with a compound of formula 8, where each X is a leaving group such as halo (such as chloro or bromo), tosylate, or mesylate, and m is 0 to 2, provides a compound of formula 9. The reaction is carried out in the presence of a NaH in THF. Removal of the amino protecting group, followed by cyclization of the resulting amine in the presence of a base such as potassium hydroxide, sodium hydroxide, or the like in a suitable organic solvent such as dichloromethane provides a compound of Formula (I).

It will be recognized by a person skilled in the art that by proceeding as described above but substituting compound 8 with other starting materials, such as bis(2-chloroethyl)amine, oxybis(ethane-2,1-diyl) bis(4-methylbenzenesulfonate), (Z)-1,4-dichlorobut-2-ene, 2-(bromomethyl)benzoic acid, 2-(2-bromoethyl)benzoic acid, 2-(bromomethyl)-5-methoxybenzoic acid and 2-(bromomethyl)-4-fluorobenzoic acid, compounds of Formula (I) where NR⁴R⁵ form other heterocycloamino rings can be synthesized.

### Administration and Pharmaceutical Composition

In general, the compounds of this disclosure will be administered in a therapeutically effective amount by any of the accepted modes of administration for agents that serve similar utilities. Therapeutically effective amounts of compounds of this disclosure may range from about 0.01 to about 500 mg per kg patient body weight per day, which can be administered in single or multiple doses. A suitable dosage level may be from about 0.1 to about 250 mg/kg per day; or about 0.5 to about 100 mg/kg per day. A suitable dosage level may be about 0.01 to about 250 mg/kg per day, about 0.05 to about 100 mg/kg per day, or about 0.1 to about 50 mg/kg per day. Within this range the dosage can be about 0.05 to about 0.5, about 0.5 to about 5 or about 5 to about 50 mg/kg per day. For oral administration, the compositions can be provided in the form of tablets containing about 1.0 to about 1000 milligrams of the active ingredient, particularly about 1, 5, 10, 15, 20, 25, 50, 75, 100, 150, 200, 250, 300, 400, 500, 600, 750, 800, 900, and 1000 milligrams of the active ingredient. The actual amount of the compound of this disclosure, i.e., the active ingredient, will depend upon numerous factors such as the severity of the disease to be treated, the age and relative health of the patient, the potency of the compound being utilized, the route and form of administration, and other factors.

In general, compounds of this disclosure will be administered as pharmaceutical compositions by any one of the following routes: oral, systemic (e.g., transdermal, intranasal or by suppository), or parenteral (e.g., intramuscular, intravenous or subcutaneous) administration. The preferred manner of administration is oral using a convenient daily dosage regimen, which can be adjusted according to the degree of affliction. Compositions can take the form of tablets, pills, capsules, semisolids, powders, sustained release formulations, solutions, suspensions, elixirs, aerosols, or any other appropriate compositions.

The choice of formulation depends on various factors such as the mode of drug administration (e.g., for oral administration, formulations in the form of tablets, pills or capsules, including enteric coated or delayed release tablets, pills or capsules are preferred) and the bioavailability of the drug substance. Recently, pharmaceutical formulations have been developed especially for drugs that show poor bioavailability based upon the principle that bioavailability can be increased by increasing the surface area i.e., decreasing particle size. For example, U.S. Pat. No. 4,107,288 describes a pharmaceutical formulation having particles in the size range from 10 to 1,000 nm in which the active material is supported on a cross-linked matrix of macromolecules. U.S. Pat. No. 5,145,684 describes the production of a pharmaceutical formulation in which the drug substance is pulverized to nanoparticles (average particle size of 400 nm) in the presence of a surface modifier and then dispersed in a liquid medium to give a pharmaceutical formulation that exhibits remarkably high bioavailability.

The compositions are comprised of, in general, a compound of this disclosure in combination with at least one pharmaceutically acceptable excipient. Acceptable excipients are non-toxic, aid administration, and do not adversely affect the therapeutic benefit of the compound of this disclosure. Such excipients may be any solid, liquid, semi-solid or, in the case of an aerosol composition, gaseous excipient that is generally available to one of skill in the art.

Solid pharmaceutical excipients include starch, cellulose, talc, glucose, lactose, sucrose, gelatin, malt, rice, flour, chalk, silica gel, magnesium stearate, sodium stearate, glycerol monostearate, sodium chloride, dried skim milk and the like. Liquid and semisolid excipients may be selected from glycerol, propylene glycol, water, ethanol and various oils, including those of petroleum, animal, vegetable or synthetic origin, e.g., peanut oil, soybean oil, mineral oil, sesame oil, etc. Preferred liquid carriers, particularly for injectable solutions, include water, saline, aqueous dextrose, and glycols.

Compressed gases may be used to disperse a compound of this disclosure in aerosol form. Inert gases suitable for this purpose are nitrogen, carbon dioxide, etc.

Other suitable pharmaceutical excipients and their formulations are described in Remington's Pharmaceutical Sciences, edited by E. W. Martin (Mack Publishing Company, 20th ed., 2000).

The level of the compound in a formulation can vary within the full range employed by those skilled in the art. Typically, the formulation will contain, on a weight percent (wt. %) basis, from about 0.01-99.99 wt. % of a compound of this disclosure based on the total formulation, with the balance being one or more suitable pharmaceutical excipients. For example, the compound is present at a level of about 1-80 wt. %.

The compounds of this disclosure may be used in combination with one or more other drugs in the treatment of diseases or conditions for which compounds of this disclosure or the other drugs may have utility. Such other drug(s) may be administered by a route and in an amount commonly used therefor, contemporaneously or sequentially with a compound of the present disclosure. When a compound of this disclosure is used contemporaneously with one or more other drugs, a pharmaceutical composition in unit dosage form containing such other drugs and the compound of the present disclosure is preferred. However, the combination therapy may also include therapies in which the compound of this disclosure and one or more other drugs are administered on different overlapping schedules. It is also contemplated that when used in combination with one or more other active ingredients, the compounds of the present disclosure and the other active ingredients may be used in lower doses than when each is used singly.

### Examples

The following preparations of compounds of Formula (I) are given to enable those skilled in the art to more clearly understand and to practice the present disclosure. They should not be considered as limiting the scope of the disclosure, but merely as being illustrative and representative thereof.

### Synthetic Examples

### Example 1

### Synthesis of 2-((2S)-1-oxo-1-(6-(pyridin-3-yl)-5,6-dihydropyridin-1(2H)-yl)propan-2-yl)isoindoline-1,3-dione (Compound 1)

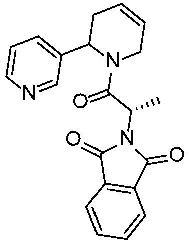

### Step 1

To a 1-necked round-bottom flask (1 L) equipped with a condenser and Dean-Stark apparatus was charged with L-alanine (17.9 g, 0.2 mole), phthalic anhydride (29.6 g, 0.2 mole), toluene (300 mL) and Et₃N (2.6 mL) in that order. The resulting mixture was refluxed for 16 hrs until no more H₂O was produced. H₂O (300 mL) was added to the cooled solution and layers were separated. Organic layer was further washed with brine, dried and concentrated. The crude was crystallized from EtOAc /hexanes (80 mL/200 mL) to give (S)-2-(1,3-dioxoisoindolin-2-yl)propanoic acid (34.5 g, 79%) as a white solid.

### Step 2

To a solution of (S)-2-(1,3-dioxoisoindolin-2-yl)propanoic acid (2.3 g, 10 mmol) in a mixture of hexane/benzene (20 mL/20 mL) was added oxalyl chloride (1.7 mL, 20 mmol) in a dropwise manner, followed by DMF (0.01 mL). After 4 hrs, all volatiles were completely removed via rotovap. The resulting (S)-2-(1,3-dioxoisoindolin-2-yl)propanoyl chloride as a crude was used in next step without further purification.

### Step 3

To a solution of (S)-2-(1,3-dioxoisoindolin-2-yl)propanoyl chloride (~ 10 mmol) in anhydrous DCM (60 mL) were added (+/-)-anatabine (1.4 g, 9 mmol) and Et₃N (1.7 mL, 12 mmol). The resulting solution was stirred at rt for 20 hrs and quenched with H₂O (60 mL). Layers were separated and aqueous layer was extracted with DCM (60 mL x 3). The combined organic layer was washed with 2N HCl, sat. NaHCO₃ and brine, dried and concentrated. Flash chromatography with normal phase column (hexane/EtOAc = 50/50) gave the title compound (1.0 g, 31%) as a white foam. MS (ESI, pos. ion) m/z: 362.1 (M+1).

### Example 2

### Synthesis of 2-((S)-1-oxo-1-((S)-6-(pyridin-3-yl)-5,6-dihydropyridin-1(2H)-yl)propan-2-yl)isoindoline-1,3-dione (Compound 2)

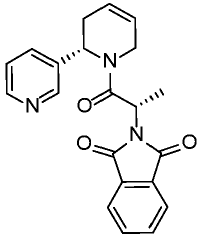

To a solution of (S)-2-(1,3-dioxoisoindolin-2-yl)propanoyl chloride (4.5 g, 20 mmol) in anhydrous benzene (120 mL) were added (+/-)-anatabine (3.0 g, 19 mmol) slowly. The resulting solution was stirred at rt for 20 hrs and quenched with sat. NaHCO₃ (100 mL). Layers were separated and the benzene layer was washed with brine, dried and concentrated. Crude 2-((2S)-1-oxo-1-(6-(pyridin-3-yl)-5,6-dihydropyridin-1(2H)-yl)propan-2-yl)isoindoline-1,3-dione (4.7 g, 68%) was obtained as a slightly yellow foam in mixture of two diastereomers: (A) 2-((S)-1-oxo-1-((S)-6-(pyridin-3-yl)-5,6-dihydropyridin-1(2H)-yl)propan-2-yl)isoindoline-1,3-dione; (B) 2-((S)-1-oxo-1-((R)-6-(pyridin-3-yl)-5,6-dihydropyridin-1(2H)-yl)propan-2-yl)isoindoline-1,3-dione, which were separated with CombiFlash^{®} using HP C18 Aq GOLD column with MeCN/buffered H₂O as an eluting mobile phase. The assignment is based on the hydrolysis experiments: A diastereomer gives S-anatabine and B diastereomer gives R-anatabine.

### Example 3

### Synthesis of 2-((S)-3-methyl-1-oxo-1-((S)-6-(pyridin-3-yl)-5,6-dihydropyridin-1(2H)-yl)butan-2-yl)isoindoline-1,3-dione (Compound 3) and 2-((S)-3-methyl-1-oxo-1-((R)-6-(pyridin-3-yl)-5,6-dihydropyridin-1(2H)-yl)butan-2-yl)isoindoline-1,3-dione (Compound 4)

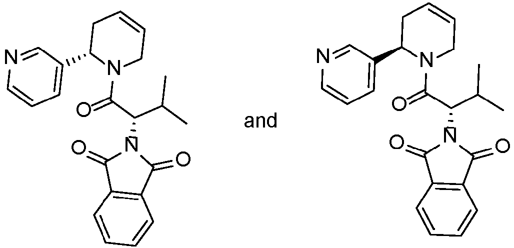

### Step 1

To a 1-necked round-bottom flask (1 L) equipped with a condenser and Dean-Stark apparatus was charged with L-valine (23.4 g, 0.2 mole), phthalic anhydride (29.6 g, 0.2 mole), toluene (300 mL) and Et₃N (2.6 mL) in that order. The resulting mixture was refluxed for 16 hrs until no more H₂O was produced. H₂O (300 mL) was added to the cooled solution and layers were separated. Organic layer was further washed with brine, dried and concentrated. The crude was crystallized from EtOAc /hexanes (80 mL/200 mL) to give (S)-2-(1,3-dioxoisoindolin-2-yl)-3-methylbutanoic acid (39.0 g, 80%) as a white solid.

### Step 2

To a solution of (S)-2-(1,3-dioxoisoindolin-2-yl)-3-methylbutanoic acid (5.0 g, 20 mmol) in a mixture of hexane/benzene (40 mL/40 mL) was added oxalyl chloride (3.5 mL, 40 mmol) in a dropwise manner, followed by DMF (0.02 mL). After 4 hrs, all volatiles were completely removed via rotovap. The resulting (S)-2-(1,3-dioxoisoindolin-2-yl)-3-methylbutanoyl chloride as a crude was used in next step without further purification.

### Step 3

To a solution of ((S)-2-(1,3-dioxoisoindolin-2-yl)-3-methylbutanoyl chloride (20 mmol) in anhydrous benzene (120 mL) were added (+/-)-anatabine (3.0 g, 19 mmol) slowly. The resulting solution was stirred at rt for 20 hrs and quenched with sat. NaHCO₃ (100 mL). Layers were separated and the benzene layer was washed with brine, dried and concentrated. The crude was purified with CombiFlash^{®} using HP C18 Aq GOLD column with MeCN/buffered H₂O as an eluting mobile phase. (A): First peak, 2-((S)-3-methyl-1-oxo-1-((S)-6-(pyridin-3-yl)-5,6-dihydropyridin-1(2H)-yl)butan-2-yl)isoindoline-1,3-dione (1.2 g, 15%) as a white foam. MS (ESI, pos. ion) m/z: 390.1 (M+1); (B): Second peak, 2-((S)-3-methyl-1-oxo-1-((R)-6-(pyridin-3-yl)-5,6-dihydropyridin-1(2H)-yl)butan-2-yl)isoindoline-1,3-dione (1.0 g, 12%) as a white foam. MS (ESI, pos. ion) m/z: 390.1 (M+1).

### Example 4

### Synthesis of a mixture of 2-((S)-1-oxo-1-((S)-6-(pyridin-3-yl)-5,6-dihydropyridin-1(2H)-yl)butan-2-yl)isoindoline-1,3-dione and 2-((R)-1-oxo-1-((R)-6-(pyridin-3-yl)-5,6-dihydropyridin-1(2H)-yl)butan-2-yl)isoindoline-1,3-dione (Compound 5) and a mixture of 2-((S)-1-oxo-1-((R)-6-(pyridin-3-yl)-5,6-dihydropyridin-1(2H)-yl)butan-2-yl)isoindoline-1,3-dione and 2-((R)-1-oxo-1-((S)-6-(pyridin-3-yl)-5,6-dihydropyridin-1(2H)-yl)butan-2-yl)isoindoline-1,3-dione (Compound 6)

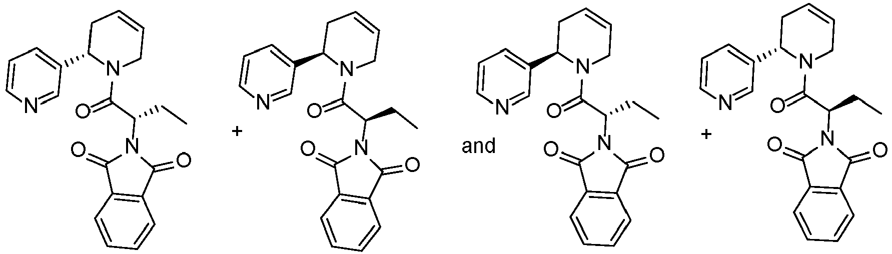

### Step 1

To a 1-necked round-bottom flask (200 mL) equipped with a condenser was charged with DL-2-aminobutanoic acid (3.5 g, 34 mmol), phthalic anhydride (5.0 g, 33.8 mmol), and glacial acetic acid (75 mL) in that order. The resulting mixture was refluxed for 6 hrs. The solvent was completely removed in vacuo to give 2-(1,3-dioxoisoindolin-2-yl)butanoic acid (7.3 g, 92%) as a clear oil.

### Step 2

To a solution of 2-(1,3-dioxoisoindolin-2-yl)butanoic acid (7.0 g, 29.3 mmol) in a mixture of hexane/benzene (100 mL/100 mL) was added oxalyl chloride (5.1 mL, 58.6 mmol) in a dropwise manner, followed by DMF (0.02 mL). After 4 hrs, all volatiles were completely removed via rotovap. The resulting 2-(1,3-dioxoisoindolin-2-yl)butanoyl chloride as a crude was used in next step without further purification.

### Step 3

To a solution of 2-(1,3-dioxoisoindolin-2-yl)butanoyl chloride (29 mmol) in anhydrous benzene (120 mL) were added (+/-)-anatabine (4.2 g, 27 mmol) slowly. The resulting solution was stirred at rt for 20 hrs and quenched with sat. NaHCO₃ (100 mL). Layers were separated and the benzene layer was washed with brine, dried and concentrated to give 2-(1-oxo-1-(6-(pyridin-3-yl)-5,6-dihydropyridin-1(2H)-yl)butan-2-yl)isoindoline-1,3-dione as a crude (5.5 g, 54%). Racemic diastereomers were separated with CombiFlash^{®} using HP C18 Aq GOLD column with MeCN/buffered H₂O as an eluting mobile phase. (A): First peak, 2-((S)-1-oxo-1-((S)-6-(pyridin-3-yl)-5,6-dihydropyridin-1(2H)-yl)butan-2-yl)isoindoline-1,3-dione and 2-((R)-1-oxo-1-((R)-6-(pyridin-3-yl)-5,6-dihydropyridin-1(2H)-yl)butan-2-yl)isoindoline-1,3-dione as a white foam. MS (ESI, pos. ion) m/z: 376.1 (M+1); (B): Second peak, 2-((S)-1-oxo-1-((R)-6-(pyridin-3-yl)-5,6-dihydropyridin-1(2H)-yl)butan-2-yl)isoindoline-1,3-dione and 2-((R)-1-oxo-1-((S)-6-(pyridin-3-yl)-5,6-dihydropyridin-1(2H)-yl)butan-2-yl)isoindoline-1,3-dione as a white foam. MS (ESI, pos. ion) m/z: 376.1 (M+1).

### Example 5

### Synthesis of 2-(1-oxo-3-phenyl-1-(6-(pyridin-3-yl)-5,6-dihydropyridin-1(2H)-yl)propan-2-yl)isoindoline-1,3-dione (Compound 7)

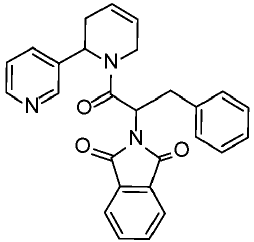

### Step 1

To a 1-necked round-bottom flask (200 mL) equipped with a condenser was charged with DL-phenylalanine (5.6 g, 34 mmol), phthalic anhydride (5.0 g, 33.8 mmol), and glacial acetic acid (100 mL) in that order. The resulting mixture was refluxed for 6 hrs. The solvent was completely removed in vacuo, and the residue was precipitated from a mixture of ethyl acetate and hexanes to give 2-(1,3-dioxoisoindolin-2-yl)-3-phenylpropanoic acid (7.0 g, 70%) as a white solid.

### Step 2

To a solution 2-(1,3-dioxoisoindolin-2-yl)-3-phenylpropanoic acid (5.0 g, 16.9 mmol) in a mixture of hexane/benzene (100 mL/100 mL) was added oxalyl chloride (3.5 mL, 40.2 mmol) in a dropwise manner, followed by DMF (0.02 mL). After 4 hrs, all volatiles were completely removed via rotovap. The resulting 2-(1,3-dioxoisoindolin-2-yl)-3-phenylpropanoyl chloride as a crude was used in next step without further purification.

### Step 3

To a solution of 2-(1,3-dioxoisoindolin-2-yl)-3-phenylpropanoyl chloride (16.9 mmol) in anhydrous benzene (100 mL) were added (+/-)-anatabine (2.4 g, 15.7 mmol) slowly. The resulting solution was stirred at rt for 20 hrs and quenched with sat. NaHCO₃ (100 mL). Layers were separated and the benzene layer was washed with brine, dried and concentrated to give 2-(1-oxo-3-phenyl-1-(6-(pyridin-3-yl)-5,6-dihydropyridin-1(2H)-yl)propan-2-yl)isoindoline-1,3-dione (4.5 g, 65%) in a white foam as a mixture of 2 diastereomers after purification with CombiFlash^{®} using HP C18 Aq GOLD column with MeCN/buffered H₂O as an eluting mobile phase. MS (ESI, pos. ion) m/z: 438.1 (M+1).

### Example 6

### Synthesis of 2-(2-oxo-2-(6-(pyridin-3-yl)-5,6-dihydropyridin-1(2H)-yl)ethyl)isoindoline-1,3-dione (Compound 8)

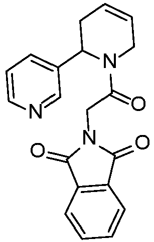

### Step 1

To a 1-necked round-bottom flask (200 mL) equipped with a condenser was charged with glycine (5.1 g, 68 mmol), phthalic anhydride (10.0 g, 67.5 mmol), and glacial acetic acid (120 mL) in that order. The resulting mixture was refluxed for 6 hrs. The solvent was completely removed in vacuo, and the residue was precipitated from a mixture of ethyl acetate and hexanes to give 2-(1,3-dioxoisoindolin-2-yl)acetic acid (10.8 g, 80%) as a white solid.

### Step 2

To a solution of 2-(1,3-dioxoisoindolin-2-yl)acetic acid (4.1 g, 20 mmol) in a mixture of hexane/benzene (40 mL/40 mL) was added oxalyl chloride (3.5 mL, 40 mmol) in a dropwise manner, followed by DMF (0.02 mL). After 4 hrs, all volatiles were completely removed via rotovap. The resulting 2-(1,3-dioxoisoindolin-2-yl)acetyl chloride as a crude was used in next step without further purification.

### .Step 3

To a solution of 2-(1,3-dioxoisoindolin-2-yl)acetyl chloride (20 mmol) in anhydrous benzene (120 mL) were added (+/-)-anatabine (2.9 g, 19.0 mmol) slowly. The resulting solution was stirred at rt for 20 hrs and quenched with sat. NaHCO₃ (100 mL). Layers were separated and the benzene layer was washed with brine, dried and concentrated to give 2-(2-oxo-2-(6-(pyridin-3-yl)-5,6-dihydropyridin-1(2H)-yl)ethyl)isoindoline-1,3-dione (3.8 g, 58%) as a white foam after purification with CombiFlash^{®} using HP C18 Aq GOLD column with MeCN/buffered H₂O as an eluting mobile phase. MS (ESI, pos. ion) m/z: 348.1 (M+1).

### Example 7

### Synthesis of 2-((2R)-1-oxo-1-(6-(pyridin-3-yl)-5,6-dihydropyridin-1(2H)-yl)propan-2-yl)isoindoline-1,3-dione (Compound 17)

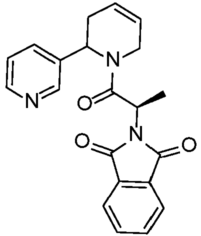

### Step 1

To a 1-necked round-bottom flask (200 mL) equipped with a condenser was charged with D-alanine (5.0 g, 56.2 mmol), phthalic anhydride (18.2 g, 56.2 mmol), and glacial acetic acid (100 mL) in that order. The resulting mixture was refluxed for 3 hrs. The solvent was completely removed in vacuo, and the residue was precipitated from a mixture of ethyl acetate and hexanes to give (R)-2-(1,3-dioxoisoindolin-2-yl)propanoic acid (8.0 g, 64%) as a white solid.

### Step 2

To a solution of (R)-2-(1,3-dioxoisoindolin-2-yl)propanoic acid (7.5 g, 33.3 mmol) in anhydrous DCM (120 mL) was added PCl₅ (7.6 g, 36.5 mmol) in portions. The resulting reaction mixture was stirred for 3 hrs at rt. All volatiles were completely removed via rotovap. The residue (R)-2-(1,3-dioxoisoindolin-2-yl)propanoyl chloride as a clear oil was used in next step without further purification.

### Step 3

To a solution of (R)-2-(1,3-dioxoisoindolin-2-yl)propanoyl chloride (33 mmol) in anhydrous benzene (120 mL) were added (+/-)-anatabine (5.3 g, 34.7 mmol) slowly. The resulting solution was stirred at rt for 20 hrs and quenched with sat. NaHCO₃ (100 mL). Layers were separated and the benzene layer was washed with brine, dried and concentrated to give 2-((2R)-1-oxo-1-(6-(pyridin-3-yl)-5,6-dihydropyridin-1(2H)-yl)propan-2-yl)isoindoline-1,3-dione (9.7 g, 81%) as a foam in a mixture of two diastereomers. MS (ESI, pos. ion) m/z: 362.1 (M+1).

### Example 8

### Synthesis of 3-nitro-N-((2S)-(1-oxo-1-(6-(pyridin-3-yl)-5,6-dihydropyridin-1(2H)-yl)propan-2-yl)benzamide (Compound 9)

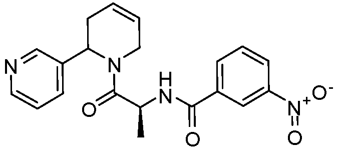

### Step 1

To a solution of (S)-2-(1,3-dioxoisoindolin-2-yl)propanoic acid (11.2 g, 50.0 mmol) in anhydrous DCM (150 mL) was added PCl₅ (11.4 g, 55 mmol) in portions. The resulting reaction mixture was stirred for 5 hrs at rt. All volatiles were completely removed via rotovap. The residue (S)-2-(1,3-dioxoisoindolin-2-yl)propanoyl chloride as a clear oil was used in next step without further purification.

### Step 2

To a solution of (S)-2-(1,3-dioxoisoindolin-2-yl)propanoyl chloride (~50 mmol) in anhydrous benzene (120 mL) were added (+/-)-anatabine (7.6 g, 50 mmol) slowly. The resulting solution was stirred at rt for 20 hrs and quenched with sat. NaHCO₃ (100 mL). Layers were separated and the benzene layer was washed with brine, dried and concentrated to give (S)-2-(1-oxo-1-(6-(pyridin-3-yl)-5,6-dihydropyridin-1(2H)-yl)propan-2-yl)isoindoline-1,3-dione (13.5 g, 74%) as a crude in a mixture of two diastereomers. MS (ESI, pos. ion) m/z: 362.4 (M+1).

### Step 3

To a solution of (S)-2-(1-oxo-1-(6-(pyridin-3-yl)-5,6-dihydropyridin-1(2H)-yl)propan-2-yl)isoindoline-1,3-dione (13.5 g, 37 mmol) in EtOH (200 mL) were added hydrazine monohydrate (3.6 mL). The reaction solution was refluxed for 2 hrs and the resulting suspension was filtered at rt. The filtrate was concentrated to give (S)-2-amino-1-(6-(pyridin-3-yl)-5,6-dihydropyridin-1(2H)-yl)propan-1-one (9.0 g, 77% from step 2) as a crude in a mixture of two diastereomers.

### Step 4

To a solution of 3-nitrobenzoic acid (0.50 g, 3.0 mmol) and (S)-2-amino-1-(6-(pyridin-3-yl)-5,6-dihydropyridin-1(2H)-yl)propan-1-one (0.7 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-nitro-N-(1-oxo-1-(6-(pyridin-3-yl)-5,6-dihydropyridin-1(2H)-yl)propan-2-yl)benzamide (0.2 g, 17%) as a film in the flask, existing as a mixture of two diastereomers. MS (ESI, pos. ion) m/z: 381.3 (M+1).

The compounds in Examples 9-13 were prepared via a similar procedure as Example 8 starting with corresponding benzoic acids.

### Example 9: 3-methoxy-N-((2S)-1-oxo-1-(6-(pyridin-3-yl)-5,6-dihydropyridin-1(2H)-yl)propan-2-yl)benzamide (0.5 g, 46%). (Compound 10)

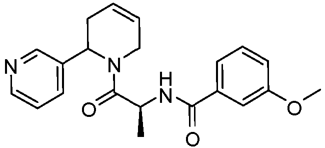

MS (ESI, pos. ion) m/z: 366.1 (M+1).

### Example 10: 3,4-diethoxy-N-((2S)-1-oxo-1-(6-(pyridin-3-yl)-5,6-dihydropyridin-1(2H)-yl)propan-2-yl)benzamide (0.6 g, 47%). (Compound 11)

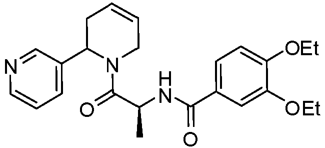

MS (ESI, pos. ion) m/z: 424.2 (M+1).

### Example 11: 3,4-dimethoxy-N-((2S)-1-oxo-1-(6-(pyridin-3-yl)-5,6-dihydropyridin-1(2H)-yl)propan-2-yl)benzamide (0.7 g, 59%). (Compound 12)

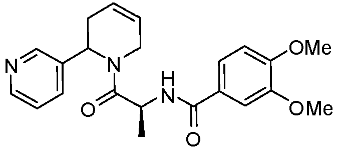

MS (ESI, pos. ion) m/z: 396.1 (M+1).

### Example 12: 4-nitro-N-((2S)-1-oxo-1-(6-(pyridin-3-yl)-5,6-dihydropyridin-1(2H)-yl)propan-2-yl)benzamide (0.1 g, 8%), which was crystallized from MeCN. (Compound 13)

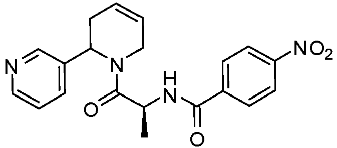

MS (ESI, pos. ion) m/z: 381.1 (M+1).

### Example 13. 4-chloro-N-((2S)-1-oxo-1-(6-(pyridin-3-yl)-5,6-dihydropyridin-1(2H)-yl)propan-2-yl)benzamide (150 mg, 13%), which was crystallized from MeCN. (Compound 14)

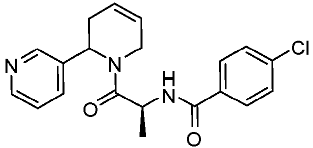

MS (ESI, pos. ion) m/z: 370.1 (M+1).

### Example 14

### Synthesis of 3-nitro-N-(1-oxo-3-phenyl-1-(6-(pyridin-3-yl)-5,6-dihydropyridin-1(2H)-yl)propan-2-yl)benzamide (Compound 15)

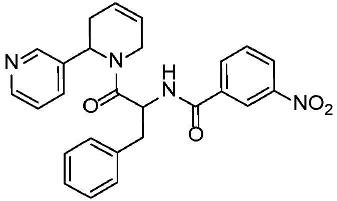

### Step 1

To a 1-necked round-bottom flask (200 mL) equipped with a condenser was charged with DL-phenylalanine (11.2 g, 68 mmol), phthalic anhydride (10.0 g, 67.6 mmol), and glacial acetic acid (200 mL) in that order. The resulting mixture was refluxed for 2 hrs. The solvent was completely removed in vacuo, and the residue was precipitated from a mixture of ethyl acetate and hexanes to give 2-(1,3-dioxoisoindolin-2-yl)-3-phenylpropanoic acid (18.1 g, 90%) as a white solid.

### Step 2

To a solution 2-(1,3-dioxoisoindolin-2-yl)-3-phenylpropanoic acid (19.8 g, 67.1 mmol) in anhydrous DCM (300 mL) was added PCl₅ (15,3 g, 73.8 mmol) in portions. The reaction mixture was stirred for 3 hrs at rt. All volatiles were completely removed via rotovap to give 2-(1,3-dioxoisoindolin-2-yl)-3-phenylpropanoyl chloride as a crude was used in next step without further purification.

### Step 3

To a solution of 2-(1,3-dioxoisoindolin-2-yl)-3-phenylpropanoyl chloride (67 mmol) in anhydrous benzene (300 mL) were added (+/-)-anatabine (12.0 g, 75.0 mmol) slowly. The resulting solution was stirred at rt overnight and quenched with sat. NaHCO₃ (300 mL). Layers were separated and the benzene layer was washed with brine, dried and concentrated to give 2-(1-oxo-3-phenyl-1-(6-(pyridin-3-yl)-5,6-dihydropyridin-1(2H)-yl)propan-2-yl)isoindoline-1,3-dione (25.0 g, 86%) in a foam as a mixture of 2 diastereomers without further purification.

### Step 4

To a solution of 2-(1-oxo-3-phenyl-1-(6-(pyridin-3-yl)-5,6-dihydropyridin-1(2H)-yl)propan-2-yl)isoindoline-1,3-dione (25.0 g) in EtOH (300 mL) were added hydrazine monohydrate (5.0 mL). The reaction solution was refluxed for 3 hrs and the resulting suspension was filtered at rt. The filtrate was dissolved in a mixture of EtOAc and hexanes and was left overnight. The resulting solid was filtered off and the filtrate was concentrated to give 2-amino-3-phenyl-1-(6-(pyridin-3-yl)-5,6-dihydropyridin-1(2H)-yl)propan-1-one (16.7 g, 54%) as a crude in a mixture of two diastereomers.

### Step 5

To a solution of 3-nitrobenzoic acid (0.5 g, 3.0 mmol) and 2-amino-3-phenyl-1-(6-(pyridin-3-yl)-5,6-dihydropyridin-1(2H)-yl)propan-1-one (0.9 g, 3.0 mmol) was added EDC hydrochloride (0.7 g, 3.8 mmol) and *i*-Pr₂NEt (0.7 mL, 3.9 mmol) in DCM (10 mL). The reaction mixture was stirred at rt overnight and quenched with H₂O (10 mL). Layers were separated and the organic layer was washed with brine, dried and concentrated to give 3-nitro-N-(1-oxo-3-phenyl-1-(6-(pyridin-3-yl)-5,6-dihydropyridin-1(2H)-yl)propan-2-yl)benzamide (0.6 g, 43%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 457.1 (M+1).

### Alternative Step 5

To a solution of 3-nitrobenzoic acid (0.8 g, 5 mmol) in DCM (20 mL) was added PCl₅ (1.2 g). The reaction mixture was stirred at rt for 2 hrs, concentrated and diluted with anhydrous DCM, which was transferred to a solution of 2-amino-3-phenyl-1-(6-(pyridin-3-yl)-5,6-dihydropyridin-1(2H)-yl)propan-1-one (1.5 g, 5 mmol) and Et₃N (7.5 mmol). Workups and purification provide the titled product as described in above Step 5.

### Example 15

### Synthesis of 3-methoxy-N-(1-oxo-3-phenyl-1-(6-(pyridin-3-yl)-5,6-dihydropyridin-1(2H)-yl)propan-2-yl)benzamide (Compound 16)

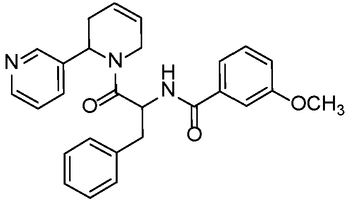

To a solution of 3-methoxybenzoic acid (0.46 g, 3.0 mmol) and 2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-methoxy-N-(1-oxo-3-phenyl-1-(6-(pyridin-3-yl)-5,6-dihydropyridin-1(2H)-yl)propan-2-yl)benzamide (0.60 g, 45%) as a film in the flask, existing as a mixture of two diastereomers after purification with CombiFlash^{®} using normal phase silica gel column with EtOAc and hexanes as an eluting mobile phase. MS (ESI, pos. ion) m/z: 442.2 (M+1).

### Example 16

### Synthesis of 3-chloro-N-(1-oxo-3-phenyl-1-(6-(pyridin-3-yl)-5,6-dihydropyridin-1(2H)-yl)propan-2-yl)benzamide (Compound 18)

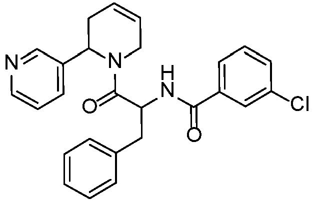

### Step 1

To a purple solution of KMnO₄ (4.0 g, 25.0 mmol) and Na₂HPO₄ (6.7 g, 25 mmol) in H₂O (100 mL) was added a solution of 3-chlorobenzaldehyde (3.5 g, 25 mmol) in MeOH (100 mL) in a dropwise manner at rt. The reaction mixture was stirred for about 30 min until a brown suspension formed. The resulting suspension was filtered through a pad of Celite^{®}, and the filtrate was concentrated via rotovap and was diluted with H₂O (100 mL). The diluted filtrate was acidified with 1N HCl until pH reached 3-4. The resulting precipitate was filtered and dried in *vacuo* at 76 °C overnight to give 3-chlorobenzoic acid (3.5 g, 90%) as a white solid.

### Step 2

To a solution of 3-chlorobenzoic acid (0.47 g, 3.0 mmol) and 2-amino-3-phenyl-1-(6-(pyridin-3-yl)-5,6-dihydropyridin-1(2H)-yl)propan-1-one (0.92 g, 3.0 mmol) was added EDC hydrate (0.75 g, 3.8 mmol) and *i*-Pr₂NEt (0.72 mL, 3.9 mmol) in DCM (10 mL). The reaction mixture was stirred at rt overnight and quenched with H₂O (10 mL). Layers were separated and the organic layer was washed with brine, dried and concentrated to give 3-chloro-N-(1-oxo-3-phenyl-1-(6-(pyridin-3-yl)-5,6-dihydropyridin-1(2H)-yl)propan-2-yl)benzamide (0.30 g, 20%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 446.1 (M+1).

### Example 17

### Synthesis of 3-fluoro-N-(1-oxo-3-phenyl-1-(6-(pyridin-3-yl)-5,6-dihydropyridin-1(2H)-yl)propan-2-yl)benzamide (Compound 19)

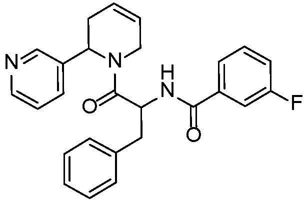

### Step 1

To a purple solution of KMnO₄ (4.0 g, 25.0 mmol) and Na₂HPO₄ (6.7 g, 25 mmol) in H₂O (100 mL) was added a solution of 3-fluorobenzaldehyde (3.1 g, 25 mmol) in MeOH (100 mL) in a dropwise manner at rt. The reaction mixture was stirred for about 30 min until a brown suspension formed. The resulting suspension was filtered through a pad of Celite^{®}, and the filtrate was concentrated via rotovap and was diluted with H₂O (100 mL). The diluted filtrate was acidified with 1N HCl until pH reached 3-4. The resulting precipitate was filtered and dried in *vacuo* at 76 °C overnight to give 3-fluorobenzoic acid (2.0 g, 57%) as a white solid.

### Step 2

To a solution of 3-fluorobenzoic acid (0.46 g, 3.0 mmol) and 2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-fluoro-N-(1-oxo-3-phenyl-1-(6-(pyridin-3-yl)-5,6-dihydropyridin-1(2H)-yl)propan-2-yl)benzamide (0.50 g, 39%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 430.1 (M+1).

### Example 18

### Synthesis of 3-ethoxy-N-(1-oxo-3-phenyl-1-(6-(pyridin-3-yl)-5,6-dihydropyridin-1(2H)-yl)propan-2-yl)benzamide (Compound 20)

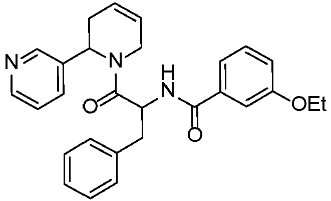

### Step 1

To a solution of 3-hydroxybenzaldehyde (6.1 g, 50 mmol) in DMF (200 mL) was added ethyl iodide (9.4 g, 60 mmol) and K₂CO₃ (10.3 g, 75 mmol) in that order. The resulting suspension was heated to 120 °C for 4 hrs, and cooled to rt. The reaction was quenched by H₂O (200 mL) and extracted with ether (200 mL). The ether layers were washed with H₂O (200 mL x3), dried and concentrated to give 3-ethoxybenzaldehyde (6.2 g, 83%) as a clear oil.

### Step 2

To a purple solution of KMnO₄ (6.2 g, 39.0 mmol) and Na₂HPO₄ (10.5 g, 39 mmol) in H₂O (150 mL) was added a solution of 3-ethoxybenzaldehyde (5.8 g) in MeOH (150 mL) in a dropwise manner at rt. The reaction mixture was stirred for about 30 min until a brown suspension formed. The resulting suspension was filtered through a pad of Celite^{®}, and the filtrate was concentrated via rotovap and was diluted with H₂O (100 mL). The diluted filtrate was acidified with 1N HCl until pH reached 3-4. The resulting precipitate was filtered and dried in *vacuo* at 76 °C overnight to give 3-ethoxybenzoic acid (2.9 g, 50%) as a white solid.

### Step 3

To a solution of 3-ethoxybenzoic acid (0.55 g, 3.0 mmol) and 2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-ethoxy-N-(1-oxo-3-phenyl-1-(6-(pyridin-3-yl)-5,6-dihydropyridin-1(2H)-yl)propan-2-yl)benzamide (0.66 g, 48%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 456.2 (M+1).

Proceeding analogously as in Example 18 above, the compounds in Examples 19-22 were synthesized using corresponding prepared or commercial benzoic acids.

### Example 19: 3,4-dimethoxy-N-(1-oxo-3-phenyl-1-(6-(pyridin-3-yl)-5,6-dihydropyridin-1(2H)-yl)propan-2-yl)benzamide (0.35 g, 25%). (Compound 21)

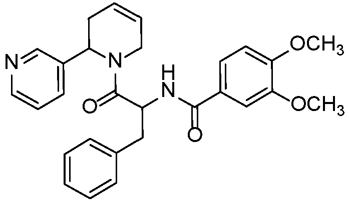

MS (ESI, pos. ion) m/z: 472.2 (M+1).

### Example 21: N-(1-oxo-3-phenyl-1-(6-(pyridin-3-yl)-5,6-dihydropyridin-1(2H)-yl)propan-2-yl)-3-(trifluoromethyl)benzamide (0.55 g, 38%). (Compound 23)

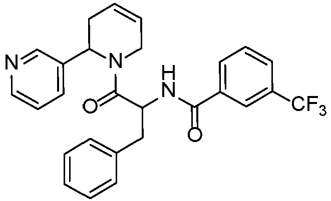

MS (ESI, pos. ion) m/z: 480.1 (M+1).

### Example 22: 3-fluoro-5-methoxy-N-(1-oxo-3-phenyl-1-(6-(pyridin-3-yl)-5,6-dihydropyridin-1(2H)-yl)propan-2-yl)benzamide (0.57 g, 41%). (Compound 24)

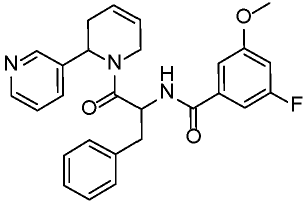

MS (ESI, pos. ion) m/z: 460.2 (M+1).

### Example 23

### Synthesis of 3-methoxy-N-((2R)-1-oxo-3-phenyl-1-(6-(pyridin-3-yl)-5,6-dihydropyridin-1(2H)-yl)propan-2-yl)benzamide (Compound 25)

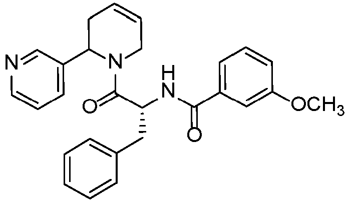

### Step 1

To a 1-necked round-bottom flask (500 mL) equipped with a condenser was charged with D-phenylalanine (25.0 g, 151.3 mmol), phthalic anhydride (22.2 g, 151.3 mmol), and glacial acetic acid (300 mL) in that order. The resulting mixture was refluxed for 4 hrs. The solvent was completely removed in vacuo, and the residue was precipitated from a mixture of ethyl acetate and hexanes to give (R)-2-(1,3-dioxoisoindolin-2-yl)-3-phenylpropanoic acid (38.4 g, 86%) as a white solid.

### Step 2

To a solution (R)-2-(1,3-dioxoisoindolin-2-yl)-3-phenylpropanoic acid (37.9 g, 128.0 mmol) in anhydrous DCM (500 mL) was added PCl₅ (29.4 g, 141.0 mmol) in portions. The reaction mixture was stirred for 3 hrs at rt. All volatiles were completely removed via rotovap to give (R)-2-(1,3-dioxoisoindolin-2-yl)-3-phenylpropanoyl chloride as a crude was used in next step without further purification.

### Step 3

To a solution of (R)-2-(1,3-dioxoisoindolin-2-yl)-3-phenylpropanoyl chloride (128 mmol) in anhydrous benzene (500 mL) were added (+/-)-anatabine (20.6 g, 128.7 mmol) slowly. The resulting solution was stirred at rt overnight and quenched with sat. NaHCO₃ (500 mL). Layers were separated and the benzene layer was washed with brine, dried and concentrated to give 2-((2R)-1-oxo-3-phenyl-1-(6-(pyridin-3-yl)-5,6-dihydropyridin-1(2H)-yl)propan-2-yl)isoindoline-1,3-dione (43.3 g, 79%) in a foam as a mixture of 2 diastereomers without further purification.

### Step 4

To a solution of 2-((2R)-1-oxo-3-phenyl-1-(6-(pyridin-3-yl)-5,6-dihydropyridin-1(2H)-yl)propan-2-yl)isoindoline-1,3-dione (43.3 g) in EtOH (500 mL) were added hydrazine monohydrate (8.7 mL). The reaction solution was refluxed for 3 hrs and the resulting suspension was filtered at rt. The filtrate was dissolved in a mixture of EtOAc and hexanes and was left overnight. The resulting solid was filtered off and the filtrate was concentrated to give (2R)-2-amino-3-phenyl-1-(6-(pyridin-3-yl)-5,6-dihydropyridin-1(2H)-yl)propan-1-one (32.3 g, 82%) as a crude in a mixture of two diastereomers.

### Step 5

To a solution of 3-methoxybenzoic acid (0.46 g, 3.0 mmol) and (2R)-2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-methoxy-N-((2R)-1-oxo-3-phenyl-1-(6-(pyridin-3-yl)-5,6-dihydropyridin-1(2H)-yl)propan-2-yl)benzamide (0.73 g, 55%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 442.2 (M+1).

### Example 24

### Synthesis of 3-ethoxy-4-methoxy-N-((2R)-1-oxo-3-phenyl-1-(6-(pyridin-3-yl)-5,6-dihydropyridin-1(2H)-yl)propan-2-yl)benzamide (Compound 26)

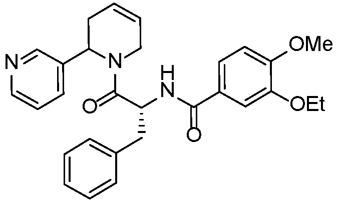

### Step1

To a solution of 3-ethoxy-4-hydroxybenzaldehyde (8.6 g, 50 mmol) in DMF (200 mL) was added NaH (3.0 g, 75 mmol, 60 wt. %) in portions at 0 °C, followed by MeI (3.35 mL, 60 mmol). The reaction mixture was stirred at rt overnight and was quenched with water. Extraction was conducted with ether (100 mL x3). The ether layer was washed, dried and concentrated to give 3-ethoxy-4-methoxybenzaldehyde (5.9 g, 66%) as a crude.

### Step 2

To a purple solution of KMnO₄ (5.1 g, 32.0 mmol) and Na₂HPO₄ (8.4 g, 32 mmol) in H₂O (120 mL) was added a solution of 3-ethoxy-4-methoxybenzaldehyde (5.6 g) in MeOH (120 mL) in a dropwise manner at rt. The reaction mixture was stirred for about 30 min until a brown suspension formed. The resulting suspension was filtered through a pad of Celite^{®}, and the filtrate was concentrated via rotovap and was diluted with H₂O (100 mL). The diluted filtrate was acidified with 1N HCl until pH reached 3-4. The resulting precipitate was filtered and dried in *vacuo* at 76 °C overnight to give 3-ethoxy-4-methoxybenzoic acid (4.0 g, 66%) as a white solid.

### Step 3

To a solution of 3-ethoxy-4-methoxybenzoic acid (0.59 g, 3.0 mmol) and (2R)-2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give 3-ethoxy-4-methoxy-N-((2R)-1-oxo-3-phenyl-1-(6-(pyridin-3-yl)-5,6-dihydropyridin-1(2H)-yl)propan-2-yl)benzamide as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 486.2 (M+1).

Proceeding analogously as described in Example 24 above, the compounds in Examples 25-30 were prepared by substituting either synthesized or commercially available benzoic acids.

### Example 25: 2-chloro-5-methoxy-N-((2R)-1-oxo-3-phenyl-1-(6-(pyridin-3-yl)-5,6-dihydropyridin-1(2H)-yl)propan-2-yl)benzamide (0.76 g, 53%). (Compound 27)

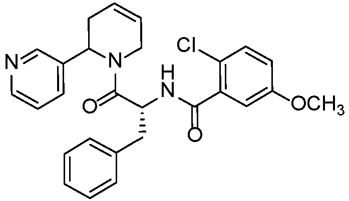

MS (ESI, pos. ion) m/z: 476.1 (M+1).

### Example 26: 3,5-dimethoxy-N-((2R)-1-oxo-3-phenyl-1-(6-(pyridin-3-yl)-5,6-dihydropyridin-1(2H)-yl)propan-2-yl)benzamide (0.86 g, 61%). (Compound 28)

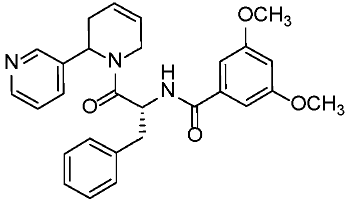

MS (ESI, pos. ion) m/z: 472.2 (M+1).

### Example 27: 5-methoxy-2-methyl-N-((2R)-1-oxo-3-phenyl-1-(6-(pyridin-3-yl)-5,6-dihydropyridin-1(2H)-yl)propan-2-yl)benzamide (0.76 g, 56%). (Compound 29)

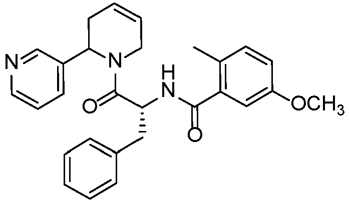

MS (ESI, pos. ion) m/z: 456.2 (M+1).

### Example 28: 4-chloro-2-methoxy-N-((2R)-1-oxo-3-phenyl-1-(6-(pyridin-3-yl)-5,6-dihydropyridin-1(2H)-yl)propan-2-yl)benzamide (0.56 g, 41%). (Compound 30)

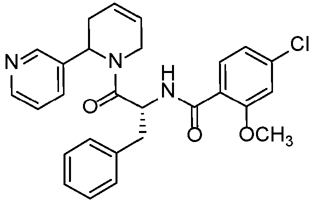

MS (ESI, pos. ion) m/z: 476.1 (M+1).

### Example 29: 4-fluoro-3-methoxy-N-((2R)-1-oxo-3-phenyl-1-(6-(pyridin-3-yl)-5,6-dihydropyridin-1(2H)-yl)propan-2-yl)benzamide (0.65 g, 47%). (Compound 31)

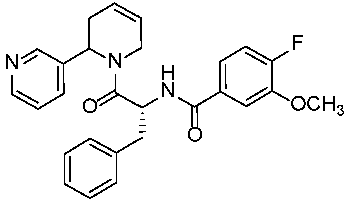

MS (ESI, pos. ion) m/z: 460.2 (M+1).

### Example 30: 2-methoxy-N-((2R)-1-oxo-3-phenyl-1-(6-(pyridin-3-yl)-5,6-dihydropyridin-1(2H)-yl)propan-2-yl)benzamide. (Compound 32)

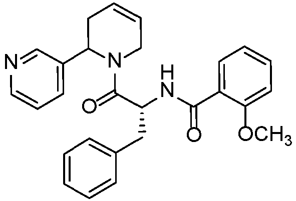

MS (ESI, pos. ion) m/z: 442.3 (M+1).

### Example 31

### Synthesis of 3-ethoxy-N-((2S)-1-oxo-3-phenyl-1-(6-(pyridin-3-yl)-5,6-dihydropyridin-1(2H)-yl)propan-2-yl)benzamide (Compound 33)

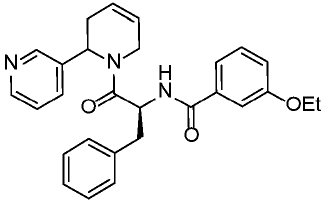

### Step 1

To a 1-necked round-bottom flask (500 mL) equipped with a condenser was charged with L-phenylalanine (25.0 g, 151.3 mmol), phthalic anhydride (22.2 g, 151.3 mmol), and glacial acetic acid (300 mL) in that order. The resulting mixture was refluxed for 4 hrs. The solvent was completely removed in vacuo, and the residue was precipitated from a mixture of ethyl acetate and hexanes to give (S)-2-(1,3-dioxoisoindolin-2-yl)-3-phenylpropanoic acid (42.8 g, 96%) as a white solid.

### Step 2

To a solution (S)-2-(1,3-dioxoisoindolin-2-yl)-3-phenylpropanoic acid (42.3 g, 142.0 mmol) in anhydrous DCM (550 mL) was added PCl₅ (32.8 g, 157.0 mmol) in portions. The reaction mixture was stirred for 3 hrs at rt. All volatiles were completely removed via rotovap to give (S)-2-(1,3-dioxoisoindolin-2-yl)-3-phenylpropanoyl chloride as a crude was used in next step without further purification.

### Step 3

To a solution of (S)-2-(1,3-dioxoisoindolin-2-yl)-3-phenylpropanoyl chloride (142 mmol) in anhydrous benzene (550 mL) were added (+/-)-anatabine (23.0 g, 142.9 mmol) slowly. The resulting solution was stirred at rt overnight and quenched with sat. NaHCO₃ (550 mL). Layers were separated and the benzene layer was washed with brine, dried and concentrated to give 2-((2S)-1-oxo-3-phenyl-1-(6-(pyridin-3-yl)-5,6-dihydropyridin-1(2H)-yl)propan-2-yl)isoindoline-1,3-dione (57.7 g, 100%) in a foam as a mixture of 2 diastereomers without further purification.

### Step 4

To a solution of 2-((2S)-1-oxo-3-phenyl-1-(6-(pyridin-3-yl)-5,6-dihydropyridin-1(2H)-yl)propan-2-yl)isoindoline-1,3-dione (57.7 g) in EtOH (500 mL) were added hydrazine monohydrate (11.6 mL). The reaction solution was refluxed overnight and the resulting suspension was filtered at rt. The filtrate was dissolved in a mixture of EtOAc and hexanes and was left overnight. The resulting solid was filtered off and the filtrate was concentrated to give (2S)-2-amino-3-phenyl-1-(6-(pyridin-3-yl)-5,6-dihydropyridin-1(2H)-yl)propan-1-one (39.0 g, 99%) as a crude in a mixture of two diastereomers.

### Step 5

To a solution of 3-ethoxybenzoic acid (0.83 g, 5.0 mmol) and (2S)-2-amino-3-phenyl-1-(6-(pyridin-3-yl)-5,6-dihydropyridin-1(2H)-yl)propan-1-one (1.5 g, 5.0 mmol) was added EDC hydrochloride (1.2 g, 6.3 mmol) and *i*-Pr₂NEt (1.1 mL, 6.5 mmol) in DCM (30 mL). The reaction mixture was stirred at rt overnight and quenched with H₂O (30 mL). Layers were separated and the organic layer was washed with brine, dried and concentrated to give 3-ethoxy-N-((2S)-1-oxo-3-phenyl-1-(6-(pyridin-3-yl)-5,6-dihydropyridin-1(2H)-yl)propan-2-yl)benzamide (1.3 g, 63%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 456.2 (M+1).

Proceeding analogously as described in Example 31 above, the compounds in Example 32-33 were prepared by substituting either synthesized or commercially available benzoic acids.

### Example 32: 4-fluoro-3-methoxy-N-((2S)-1-oxo-3-phenyl-1-(6-(pyridin-3-yl)-5,6-dihydropyridin-1(2H)-yl)propan-2-yl)benzamide (0.4 g, 29%). (Compound 34)

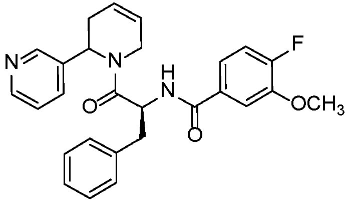

MS (ESI, pos. ion) m/z: 460.2 (M+1).

### Example 33: 3-methoxy-N-((2S)-1-oxo-3-phenyl-1-(6-(pyridin-3-yl)-5,6-dihydropyridin-1(2H)-yl)propan-2-yl)benzamide (0.8 g, 36%). (Compound 35)

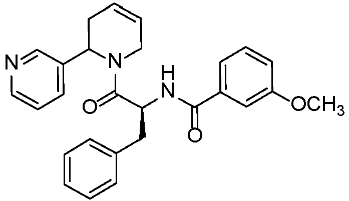

MS (ESI, pos. ion) m/z: 442.2 (M+1).

Proceeding analogously as described in Example 31 above, the compounds in Examples 40-56 were prepared by substituting either commercially available carboxylic acids or carboxylic acids which can be prepared readily from amino benzoic acids and corresponding acid chlorides or acids using coupling reactions.

### Example 40: 3-(3-methylbutanamido)-N-((2S)-1-oxo-3-phenyl-1-(6-(pyridin-3-yl)-5,6-dihydropyridin-1(2H)-yl)propan-2-yl)benzamide as a tan solid (0.90 g, 60%). (Compound 42)

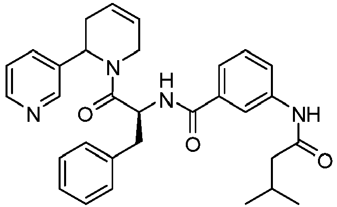

MS (ESI, pos, ion) m/z: 511.2 (M+1).

### Example 41: N-((2S)-1-oxo-3-phenyl-1-(6-(pyridin-3-yl)-5,6-dihydropyridin-1(2H)-yl)propan-2-yl)-3-pivalamidobenzamide as a gray solid (0.93 g, 62%). (Compound 43)

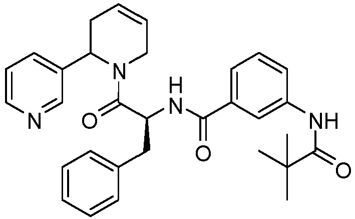

MS (ESI, pos, ion) m/z: 511.2 (M+1).

### Example 42: 3-(cyclopropanecarboxamido)-N-((2S)-1-oxo-3-phenyl-1-(6-(pyridin-3-yl)-5,6-dihydropyridin-1(2H)-yl)propan-2-yl)benzamide as a white solid (1.01 g, 69%). (Compound 44)

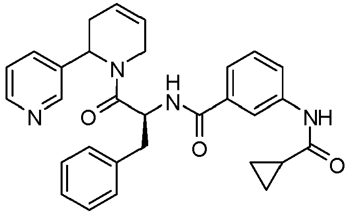

MS (ESI, pos, ion) m/z: 495.2 (M+1).

### Example 43: 3-(cyclobutanecarboxamido)-N-((2S)-1-oxo-3-phenyl-1-(6-(pyridin-3-yl)-5,6-dihydropyridin-1(2H)-yl)propan-2-yl)benzamide as an orange solid (1.05 g, 69%). (Compound 45)

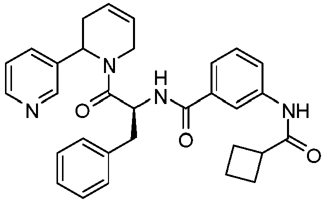

MS (ESI, pos, ion) m/z: 509.2 (M+1).

### Example 44: 3-(cyclopentanecarboxamido)-N-((2S)-1-oxo-3-phenyl-1-(6-(pyridin-3-yl)-5,6-dihydropyridin-1(2H)-yl)propan-2-yl)benzamide as a white solid (0.69 g, 64%). (Compound 46)

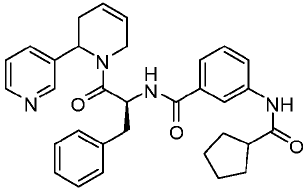

MS (ESI, pos, ion) m/z: 523.2 (M+1).

### Example 45: 2-butyramido-N-((2S)-1-oxo-3-phenyl-1-(6-(pyridin-3-yl)-5,6-dihydropyridin-1(2H)-yl)propan-2-yl)benzamide as a white solid (0.12 g, 12%). (Compound 47)

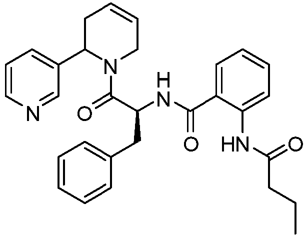

MS (ESI, pos, ion) m/z: 497.2 (M+1).

### Example 46: 4-butyramido-N-((2S)-1-oxo-3-phenyl-1-(6-(pyridin-3-yl)-5,6-dihydropyridin-1(2H)-yl)propan-2-yl)benzamide as a white solid (0.45 g, 45%). (Compound 48)

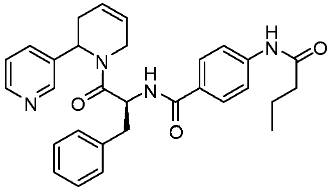

MS (ESI, pos, ion) m/z: 497.2 (M+1).

### Example 47: N-((2S)-1-oxo-3-phenyl-1-(6-(pyridin-3-yl)-5,6-dihydropyridin-1(2H)-yl)propan-2-yl)quinoline-2-carboxamide as a white solid (0.50 g, 53%). (Compound 49)

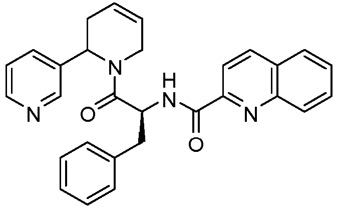

MS (ESI, pos, ion) m/z: 463.2 (M+1).

### Example 48: N-((2S)-1-oxo-3-phenyl-1-(6-(pyridin-3-yl)-5,6-dihydropyridin-1(2H)-yl)propan-2-yl)pyrazine-2-carboxamide as a yellow solid (0.50 g, 61%). (Compound 50)

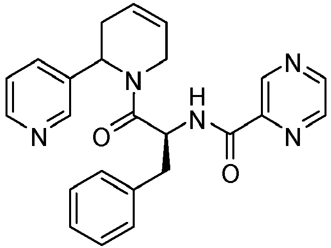

MS (ESI, pos, ion) m/z: 414.1 (M+1).

### Example 49: N-((2S)-1-oxo-3-phenyl-1-(6-(pyridin-3-yl)-5,6-dihydropyridin-1(2H)-yl)propan-2-yl)nicotinamide as a white solid (0.41 g, 50%). (Compound51)

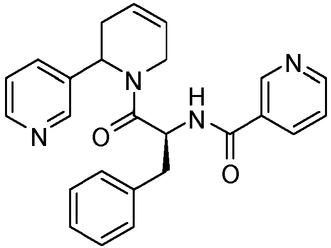

MS (ESI, pos, ion) m/z: 413.1 (M+1).

### Example 50: N-((2S)-1-oxo-3-phenyl-1-(6-(pyridin-3-yl)-5,6-dihydropyridin-1(2H)-yl)propan-2-yl)furan-2-carboxamide as a white solid (0.50 g, 63%). (Compound 52)

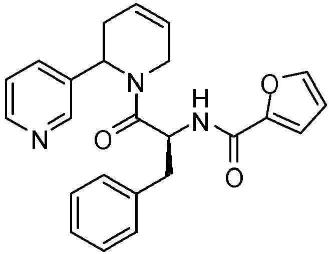

MS (ESI, pos, ion) m/z: 402.1 (M+1).

### Example 51: N-((2S)-1-oxo-3-phenyl-1-(6-(pyridin-3-yl)-5,6-dihydropyridin-1(2H)-yl)propan-2-yl)-1H-pyrrole-2-carboxamide as a light yellow solid (0.20 g, 25%). (Compound 56)

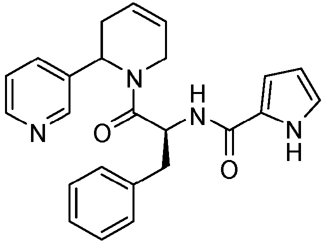

MS (ESI, pos, ion) m/z: 401.1 (M+1).

### Example 52: 3-((S)-2-methylbutanamido)-N-((2S)-1-oxo-3-phenyl-1-(6-(pyridin-3-yl)-5,6-dihydropyridin-1(2H)-yl)propan-2-yl)benzamide as a light yellow solid (0.60 g, 56%).

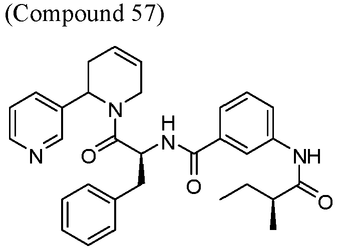

MS (ESI, pos, ion) m/z: 511.2 (M+1).

### Example 53: 3-((R)-2-methylbutanamido)-N-((2S)-1-oxo-3-phenyl-1-(6-(pyridin-3-yl)-5,6-dihydropyridin-1(2H)-yl)propan-2-yl)benzamide as a light yellow solid (0.67 g, 65%).

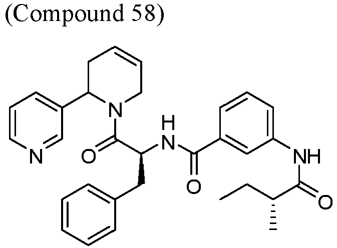

MS (ESI, pos, ion) m/z: 511.2 (M+1).

### Example 54: 3-(N-methylbutyramido)-N-((2S)-1-oxo-3-phenyl-1-(6-(pyridin-3-yl)-5,6-dihydropyridin-1(2H)-yl)propan-2-yl)benzamide as a light yellow solid (0.80 g, 78%).

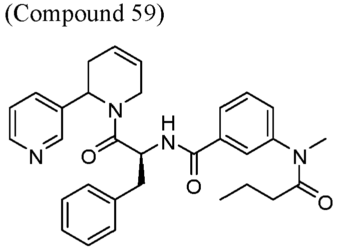

MS (ESI, pos, ion) m/z: 511.2 (M+1).

### Example 55: 3-(butylamino)-N-((2S)-1-oxo-3-phenyl-1-(6-(pyridin-3-yl)-5,6-dihydropyridin-1(2H)-yl)propan-2-yl)benzamide as a light yellow solid (0.82 g, 85%). (Compound 60)

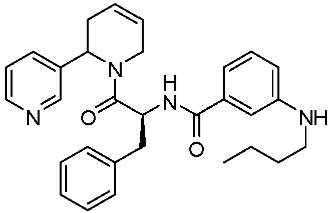

MS (ESI, pos, ion) m/z: 483.2 (M+1).

### Example 56: 1-butyl-N-((2S)-1-oxo-3-phenyl-1-(6-(pyridin-3-yl)-5,6-dihydropyridin-1(2H)-yl)propan-2-yl)-1H-pyrrole-2-carboxamide as a yellow oil (0.21 g, 23%). (Compound 61)

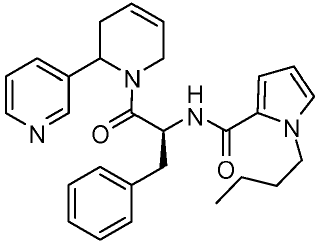

MS (ESI, pos, ion) m/z: 457.2 (M+1).

### Example 34

### Synthesis of N-(1-oxo-3-phenyl-1-(6-(pyridin-3-yl)-5,6-dihydropyridin-1(2H)-yl)propan-2-yl)-3-pentanamidobenzamide (Compound 36)

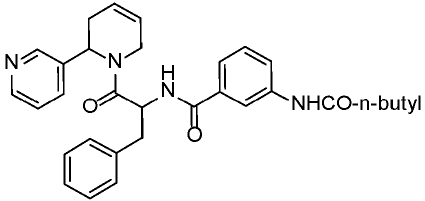

### Step 1

To a suspension of 3-aminobenzoic acid (5.0 g, 36.5 mmol) in DCM (120 mL) was added pyridine (6.5 mL) and valeroyl chloride (5.5 mL, 45.6 mmol) in that order at 0 °C. The resulting solution was stirred at rt overnight and quenched by water. The layers were separated and the organic layer was washed, dried and concentrated to give 3-pentanamidobenzoic acid (7.2 g, 90%) as white solid.

### Step 2

To a solution of 3-pentanamidobenzoic acid (0.66 g, 3.0 mmol) and 2-amino-3-phenyl-1-(6-(pyridin-3-yl)-5,6-dihydropyridin-1(2H)-yl)propan-1-one (0.92 g, 3.0 mmol) was added EDC hydrochloride (0.72 g, 3.8 mmol) and *i*-Pr₂NEt (0.70 mL, 3.9 mmol) in DCM (10 mL). The reaction mixture was stirred at rt overnight and quenched with H₂O (10 mL). Layers were separated and the organic layer was washed with brine, dried and concentrated to give N-(1-oxo-3-phenyl-1-(6-(pyridin-3-yl)-5,6-dihydropyridin-1(2H)-yl)propan-2-yl)-3-pentanamidobenzamide (0.81 g, 59%) as a film in the flask, existing as a mixture of two diastereomers after purification with CombiFlash^{®} using HP C18 Aq GOLD column with MeCN/buffered H₂O as an eluting mobile phase. MS (ESI, pos. ion) m/z: 511.2 (M+1).

Proceeding analogously as described in Example 34 above, the compounds in Examples 35-37 were prepared by substituting either synthesized or commercially available benzoic acids.

### Example 35: 3-butyramido-N-(1-oxo-3-phenyl-1-(6-(pyridin-3-yl)-5,6-dihydropyridin-1(2H)-yl)propan-2-yl)benzamide. (Compound 37)

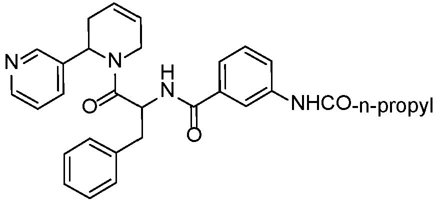

MS (ESI, pos. ion) m/z: 497.2 (M+1).

### Example 36: 3-amino-N-(1-oxo-3-phenyl-1-(6-(pyridin-3-yl)-5,6-dihydropyridin-1(2H)-yl)propan-2-yl)benzamide. (Compound 38)

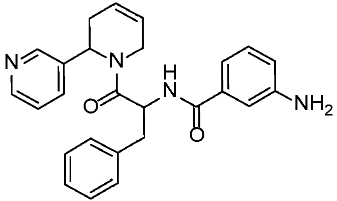

MS (ESI, pos. ion) m/z: 427.2 (M+1).

### Example 37: 3-acetamido-N-(1-oxo-3-phenyl-1-(6-(pyridin-3-yl)-5,6-dihydropyridin-1(2H)-yl)propan-2-yl)benzamide (169 mg, 12%). (Compound 39)

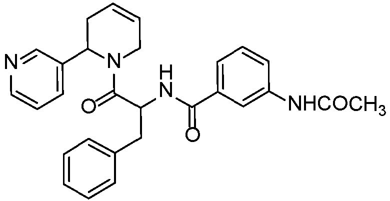

MS (ESI, pos. ion) m/z: 469.2 (M+1).

### Example 38

### Synthesis of 3-butyramido-N-(1-oxo-3-phenyl-1-(2-(quinolin-3-yl)-3,6-dihydropyridin-1(2H)-yl)propan-2-yl)benzamide (Compound 40)

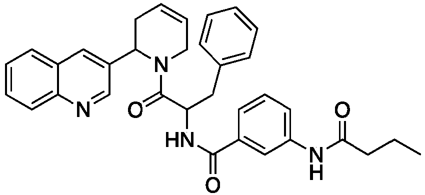

### Synthetic Scheme for 3-butyramido-N-(1-oxo-3-phenyl-1-(2-(quinolin-3-yl)-3,6-dihydropyridin-1(2H)-yl)propan-2-yl)benzamide (1,2-combo compound):

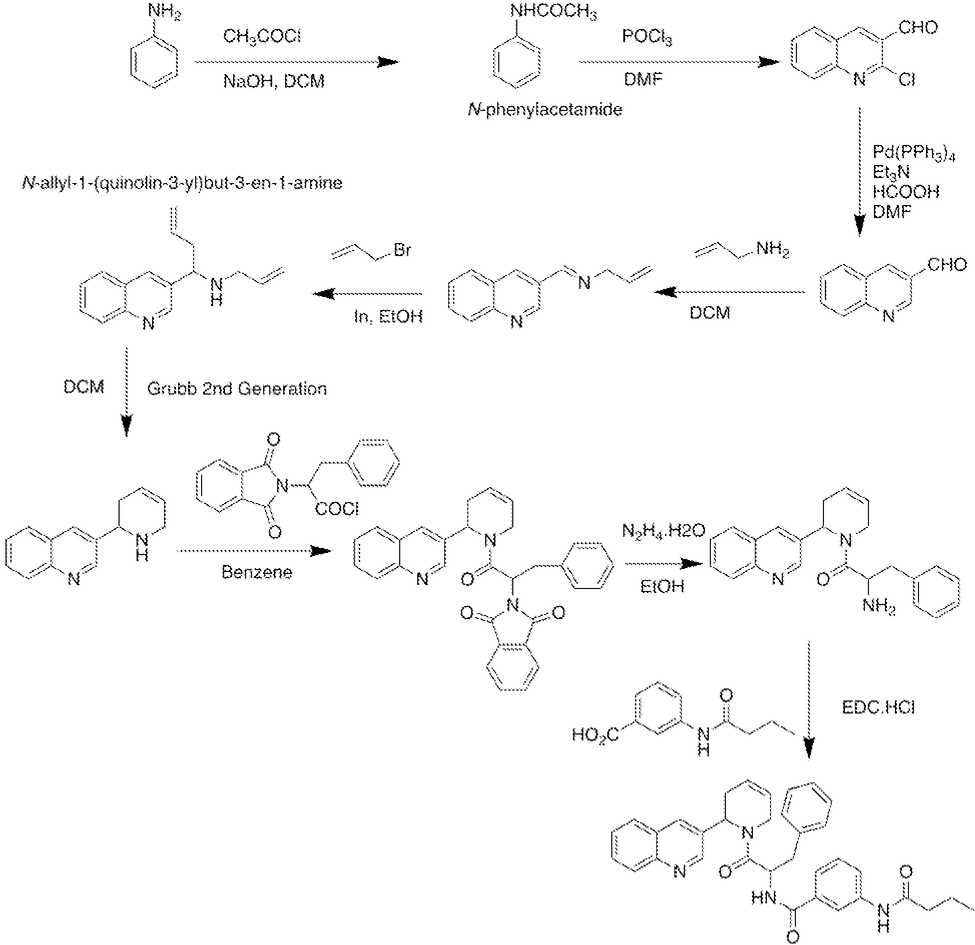

### Step 1

### Synthesis ofN-phenylacetamide

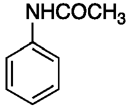

To a suspension of aniline (5g, 53.7 mmol) in 2N NaOH (40.3 mL) was added acetyl chloride (4.63 g, 59.07 mmol, 1.1 eq.) in 40.3 mL of dichloromethane dropwise in 1 h at ice-bath. The resulting mixture was stirred an additional hour at room temperature. Organic layer was washed with dilute ice, NaHCO₃ and brine. The organic layer was concentrated under reduced pressure to get the desired product as a crude acetanilide. The crude acetanilide was recrystallized on dissolving with distilled water to get pure and dry acetanilide as a white crystalline solid (6.3 g, 87% isolated yield).

### Step 2

### Synthesis of 2-chloroquinoline-3-carbaldehyde

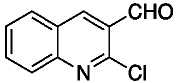

To DMF (10.82 g, 148 mmol, 2.5 eq.) was added phosphorus oxychloride (63.5 g, 414.13 mmol, 7 eq.) dropwise cooled by ice-bath (30 min). The mixture was stirred another 30 min. at 0 °C then acetanilide (8 g, 59.18 mmol, 1 eq.) was added in one portion. The white suspension turned into greenish solution which was heated overnight. Mixture was poured into ice. Yellow precipitate was filtered and dried to get the yellow orange solid (6.8 g, 60% isolated yield)

### Step 3

### Synthesis of quinoline-3-carbaldehyde

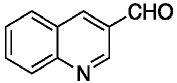

To 2-chloroquinoline-3-carbaldehyde (3g, 15.69 mmol, 1 eq.) in DMF (18 mL) at 23 °C and under an atmosphere of nitrogen was added triethylamine 19.05 g, 188.28 mmol, 12 eq.), tetrakis(triphenylphosphine)palladium (0) (0.9 g, 0.78 mmol, 5 mol%) and formic acid (3.9 g, 84.72 mmol, 5.4 eq.). After stirring for 3 h at 110°C, the reaction mixture was cooled to 23 °C and water (90 mL) and ethyl acetate (3x60 mL). The combined organic phase was washed with brine (50 mL) dried with anhydrous Na₂SO₄ and filtered. The filtrate was concentrated in vacuum to obtained crude product which was purified on flash chromatography using 50% EtOAc/hexane to get desired product as a reddish brown solid (1.5 g, 60.8% isolated yield).

### Step 4

### Synthesis of (E)-N-allyl-1-(quinolin-3-yl)methanimine

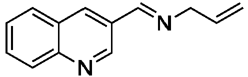

In a 250 mL round bottomed flask allyl amine (1.3 g, 22.92 mmol, 1.2 eq.) was added to a stirred solution of quinoline-3-carbaldehyde (3 g, 19.1 mmol, 1 eq.) in anhydrous dichloromethane (96 mL) containing activated 4 °A MS (5 g). Then the resulting mixture was stirred at room temperature for 24 h. Successively, the molecular sieves were filtered off, washed with DCM and after the solvent evaporation, we obtained the imine product as brown solid (4.3 g, 95% isolated yield).

### Step 5

### Synthesis of (N-allyl-1-(quinolin-3-yl)but-3-en-1-amine

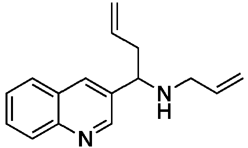

In a 250 mL 2-neck flask, allyl bromide (3.95 g, 32.63 mmol, 2 eq.) was dropwise added to a cold (0 °C) anhydrous ethanolic (83 mL) solution of (*E*)-*N*-allyl-1-(quinolin-3-yl)methanimine (3.2 g, 16.31 mmol, 1 eq.) and indium powder (2.79 g, 24.46 mmol, 1.5 eq.). The resulting mixture was stirred at same temperature for 30 minutes. Afterwards, the temperature was left to reach room temperature and the system was stirred for an additional 3.5 h. Successively, the solvent was evaporated under vacuum, the residue was dissolved in EtOAc (60 mL) and treated with sat. aq. NaHCO₃ (40 mL). The two layers were separated, and the aq. Layer was extracted with EtOAc (3x20 mL). The combined organic layers were dried using anhydrous Na₂SO₄, filtered and the solvent evaporated under reduced pressure. The resulting crude product was purified on column chromatography using 50% EtOAc /hexane as eluent to get pure product as yellowish-brown oil (3.4 g, 88% isolated yield).

### Step 6

### Synthesis of 3-(1,2,3,6-tetrahydropyridin-2-yl)quinoline

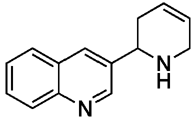

In a 100 mL 2-neck pear shaped flask equipped with magnetic stirrer, *N*-allyl-1-(quinolin-3-yl)but-3-en-1-amine (1.56 g, 6.55 mmol, 1 eq.) was dissolved in anhydrous dichloromethane ( 64 mL). p-toulenesulfonic acid monohydrate (2.74 g, 14.41 mmol, 2.2 eq.) was added and the mixture was stirred for 10 min. Grubb's added till the solution pH was found to be 9-10. The organic layer was separated and dried using anhydrous Na₂SO₄, filtered and the solvent evaporated under reduced pressure. The resulting crude product was purified second generation catalyst (0.555 g, 0.65 mmol, 10 mol%) was added and the resulting mixture was stirred at room temperature for 18 h. The reaction mixture was neutralized using 50 mL of 2N NaOH and was stirred for 20 minutes. The organic layer was collected and washed with water (3x50 mL). The organic layer was dried using anhydrous Na₂SO₄ and concentrated under reduced pressure. The residue was dissolved in 100 mL of EtOAc and then added 30 mL of 6N HCl, 70 ml of deionized water, 20 mL of brine solution and the mixture was stirred for 20 minutes. The aq. Layer was collected, and aq. Layer was further extracted with (3x 50 mL) of EtOAc. The aq. Layer was transferred to 500 mL round bottomed flask and was cooled at 0 °C. 120 mL of methyl tert-butyl ether was added to the aq. layer and the anhydrous potassium carbonate was on column chromatography packed with mixture of hexane and ammonium hydroxide in the ration of 20:1 and using 4% Methanol /Chloroform as eluent to get pure product as reddish-brown oil (0.325 g, 28% isolated yield).

### Step 7

### Synthesis of 2-(1,3-dioxoisoindolin-2-yl)-3-phenylpropanoyl chloride

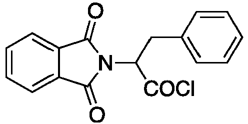

### Step 7(i)

To an oven dried 1-necked round-bottom flask (500 mL) equipped with a condenser and Dean-Stark apparatus was charged with DL-phenylalanine (20 g, 121 mmole, 1 eq.), phthalic anhydride (17.9 g, 121 mmole, 1 eq.), toluene (180 mL) and Et₃N (1.6 mL) in that order. The resulting mixture was refluxed for 16 hrs until no more water was produced. H₂O (180 mL) was added to the cooled solution and the product was precipitated. The product was filtered and dried on high vacuum for overnight. The dried product 2-(1,3-dioxoisoindolin-2-yl)-3-phenylpropanoic acid was collected as a white solid (29.5 g, 82.6% isolated yield). ¹H NMR (CDCl₃): 7.80-7.78 (m, 2H), 7.70 -7.69(m, 2H), 7.27-7.13 (m, 5H), 5.23 (t, J = 8.2 Hz, 1H), 3.61 (d, J = 7.5 Hz, 2H); ¹³C NMR (CDCl₃): 174.1, 167.3, 136.4, 134.1, 131.4, 128.8, 128.5, 126.9, 123.5, 53.0, 34.4.

### Step 7(ii)

To a solution of 2-(1,3-dioxoisoindolin-2-yl)-3-phenylpropanoic acid (2.5 g, 8.47 mmol, 1 eq.) in anhydrous DCM (32 mL) was added PCl₅ (1.93 g, 9.28 mmol, 1.09 eq.) in portions. The resulting reaction mixture was stirred for 3 hrs at rt. All volatiles were completely removed under reduced pressure. The residue 2-(1,3-dioxoisoindolin-2-yl)-3-phenylpropanoyl chloride (2.12 g) as a white solid was used in next step without further purification.

### Step 8

### Synthesis of 2-(1-oxo-3-phenyl-1-(2-(quinolin-3-yl)-3,6-dihydropyridin-1(2H-yl)propan-2-yl)isoindoline-1,3-dione

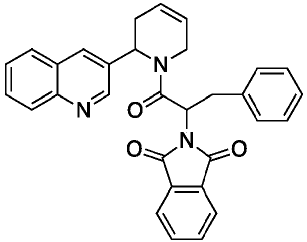

To a solution of 2-(1,3-dioxoisoindolin-2-yl)-3-phenylpropanoyl chloride (6.78 mmol) in anhydrous benzene (25 mL) were added 3-(1,2,3,6-tetrahydropyridin-2-yl)quinoline (1.5 g, 7.13 mmo, 1.05 eq.) slowly. The resulting solution was stirred at room temperature for 20 hrs and quenched with sat. NaHCO₃ (20 mL). Layers were separated and the benzene layer was washed with brine, dried and concentrated to give 3-(1,2,3,6-tetrahydropyridin-2-yl)quinoline as a crude product. The crude product was purified on column chromatography using 50% EtOAc/ Hexane mixture as an eluent to give the desired product as a white solid (1.4 g, 42.3%) in a mixture of diastereomers. MS (ESI, pos. ion) m/z: 488.2 (M+1).

### Step 9

### Synthesis of 2-amino-3-phenyl-1-(2-(quinolin-3-yl)-3,6-dihydropyridin-1(2H)-yl)propan-1-one

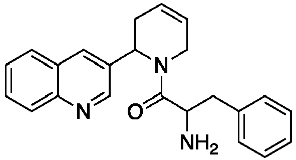

To a solution of 2-(1-oxo-3-phenyl-1-(2-(quinolin-3-yl)-3,6-dihydropyridin-1(2*H*)-yl)propan-2-yl)isoindoline-1,3-dione (1.4. g, 2.9 mmol) in EtOH (20 mL) were added hydrazine monohydrate (0.45 mL). The reaction solution was refluxed for overnight at 85 °C and the resulting suspension was filtered at room temperature. The filtrate was concentrated and further dissolved with 20 mL 1: 1 mixture of EtOAc/hexane and kept at 0 °C for overnight. The mixture was filtered and the filtrate was concentrated to give 2-amino-3-phenyl-1-(2-(quinolin-3-yl)-3,6-dihydropyridin-1(2*H*)-yl)propan-1-one (0.8 g, 77%) as a brown oil in a mixture of diastereomers. MS (ESI, pos. ion) m/z: 358.19 (M+1).

### Step 10

### Synthesis of 2-amino-3-phenyl-1-(2-(quinolin-3-yl)-3,6-dihydropyridin-1(2H)-yl)propan-1-one

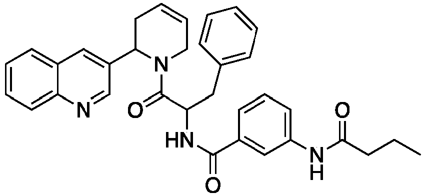

To a solution of 3-butyramidobenzoic acid (0.47 g, 2.15 mmol, 1 eq.) and 2-amino-3-phenyl-1-(2-(quinolin-3-yl)-3,6-dihydropyridin-1(2*H*)-yl)propan-1-one (0.47 g, 2.15 mmol, 1 eq.) was added EDC hydrochloride (0.56 g, 2.8 mmol, 1.3 eq.) and *i*-Pr₂NEt (0.5 mL, 2.80 mmol, 1.3 eq.) in DCM (10 mL). The reaction mixture was stirred at room temperature for overnight and quenched with H₂O (10 mL). Layers were separated, and the organic layer was washed with brine, dried and concentrated to give crude product and the crude product was crystallized using acetonitrile as a solvent. The crystals were filtered and collected as a white collect (0.45 g, 38.3%) existing as a mixture of diastereomers. MS (ESI, pos. ion) m/z: 547.5 (M+1).

Proceeding analogously as described in Example 38 above, the compounds in Examples 65-66 were prepared starting with corresponding amino acids.

### Example 65: 3-butyramido-N-(1-(2-(2-chloroquinolin-3-yl)-3,6-dihydropyridin-1(2H)-yl)-1-oxo-3-phenylpropan-2-yl)benzamide as a pale yellow solid (0.05 g, 17.2%). (Compound 67)

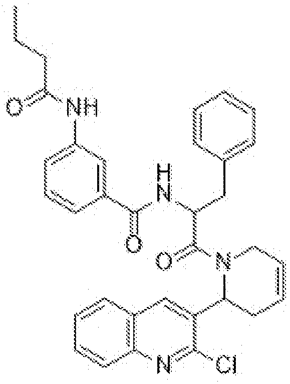

MS (ESI, pos, ion) m/z: 581.3(M+1).

### Example 66: 3-butyramido-N-(1-oxo-1-(2-(quinolin-3-yl)-3,6-dihydropyridin-1(2H)-yl)propan-2-yl)benzamide as a creamy-white solid (0.13 g, 38.6%). (Compound 68)

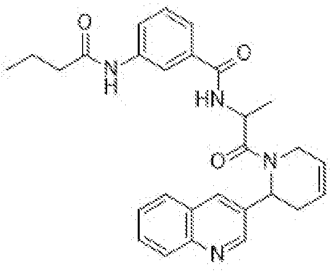

MS (ESI, pos, ion) m/z: 471.4 (M+1).

### Example 39

### Synthesis of 3-{5-[(3aS,4S,6aR)-2-oxo-hexahydro-1H-thieno[3,4-d]imidazol-4-yl]pentanamido}-N-[(2S)-1-oxo-3-phenyl-1-{1,2,3,6-tetrahydro-[2,3'-bipyridin]-1-yl}propan-2-yl]benzamide

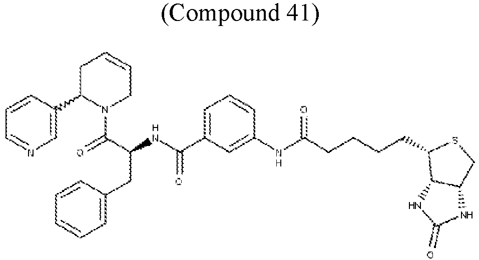

### Step 1

To a solution of 3-nitrobenzoic acid (8.4 g, 50 mmol) in MeOH (150 mL) was added SOCl2 (7.1 mL, 100 mmol) slowly at rt. The reaction mixture was refluxed for 2 hrs. Removal of all solvents gave methyl 3-nitrobenzoate (9.1 g, 100%) as a crude.

### Step 2

To a solution of methyl 3-nitrobenzoate (9.1 g, 50 mmol) in EtOH (150 mL) was added SnCl2·2H2O (22.5 g, 100 mmol) in one portion. The reaction mixture was refluxed for 2 hours and solvent was then removed. The residue was extracted with EtOAc. The organic layer was washed, dried, and concentrated to give methyl 3-aminobenzoate (8.4 g, 100%) as a brown oil.

### Step 3

To a solution of methyl 3-aminobenzoate (0.87 g, 5.7 mmol) and biotin (1.4 g, 5.7 mmol) in DCM (15 mL) was added EDC (1.3 g, 7.0 mmol) and i-Pr2NEt (1.2 mL, 7.5 mmol). The reaction mixture was stirred at rt overnight and quenched with H2O (10 mL). Layers were separated and the organic layer was washed with brine, dried and concentrated to give methyl 3-(5-(2-oxohexahydro-1H-thieno[3,4-d]imidazol-4-yl)pentanamido)benzoate (1.13 g, 52%).

### Step 4

To a solution of methyl 3-(5-(2-oxohexahydro-1H-thieno[3,4-d]imidazol-4-yl)pentanamido)benzoate (1.13 g, 2.9 mmol) in a mixture of THF (15 mL) and H2O (15 mL) was added LiOH (0.36 g, 14 mmol) in one portion and stirred at rt overnight. The reaction was further diluted with 1N NaOH, and the aqueous layer was washed with EtOAc, neutralized with 1N HCl. The resulting solid was collected to give 3-(5-(2-oxohexahydro-1H-thieno[3,4-d]imidazol-4-yl)pentanamido)benzoic acid (0.7 g, 83%) as a crude.

### Step 5

To a solution of 3-(5-(2-oxohexahydro-1H-thieno[3,4-d]imidazol-4-yl)pentanamido)benzoic acid (0.7 g, 1.9 mmol) and (2S)-2-amino-3-phenyl-1-(6-(pyridin-3-yl)-5,6-dihydropyridin-1(2H)-yl)propan-1-one (0.6 g, 1.9 mmol) was added EDC hydrochloride (0.5 g, 2.4 mmol) and i-Pr2NEt (0.4 mL, 2.5 mmol) in DCM (20 mL). The reaction mixture was stirred at rt overnight and quenched with H2O (30 mL). Layers were separated and the organic layer was washed with brine, dried and concentrated to give 3-{5-[(3aS,4S,6aR)-2-oxo-hexahydro-1H-thieno[3,4-d]imidazol-4-yl]pentanamido}-N-[(2S)-1-oxo-3-phenyl-1-{1,2,3,6-tetrahydro-[2,3'-bipyridin]-1-yl}propan-2-yl]benzamide (80 mg, 7%) as a powder after purification with CombiFlash^{®} using HP C18 Aq GOLD column with MeCN/buffered H2O as an eluting mobile phase. MS (ESI, pos. ion) m/z: 653.5 (M+1).

### Example 57

### Synthesis of 3-(3-aminopropanamido)-N-((2S)-1-oxo-3-phenyl-1-(6-(pyridin-3-yl)-5,6-dihydropyridin-1(2H)-yl)propan-2-yl)benzamide hydrochloride (Compound 54)

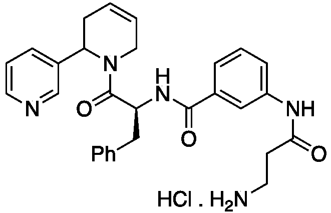

### Synthetic Scheme for 3-(3-aminopropanamido)-N-((2S)-1-oxo-3-phenyl-1-(6-(pyridin-3-yl)-5,6-dihydropyridin-1(2H)-yl)propan-2-yl)benzamide hydrochloride:

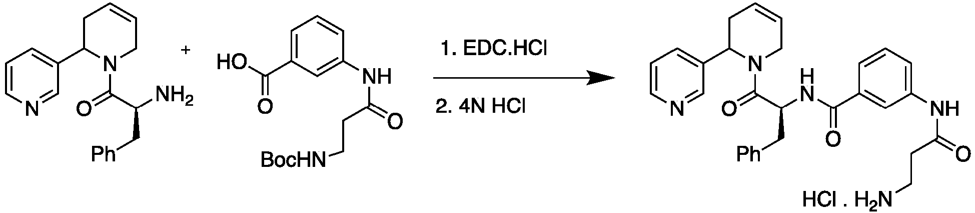

To a solution of 3-(3-((*tert*-butoxycarbonyl)amino)propanamido)benzoic acid (0.62 g, 2.0 mmol) and (2S)-2-amino-3-phenyl-1-(6-(pyridin-3-yl)-5,6-dihydropyridin-1(2H)-yl)propan-1-one (0.62 g, 2.0 mmol) was added EDC hydrochloride (0.57 g, 2.9 mmol) and *i*-Pr₂NEt (0.47 mL, 3.9 mmol) in DCM (10 mL). The reaction mixture was stirred at rt overnight and quenched with H₂O (10 mL). The layers were separated and the organic layer was washed with brine, dried and concentrated to give 3-(3-aminopropanamido)-N-((2S)-1-oxo-3-phenyl-1-(6-(pyridin-3-yl)-5,6-dihydropyridin-1(2H)-yl)propan-2-yl)benzamide as a film in the flask, existing as a mixture of two diastereomers after purification with CombiFlash^{®} using HP C18 Aq GOLD column with MeCN/buffered H₂O as an eluting mobile phase. The benzamide was dissolved in 5 ml of 1,4-dioxane and was converted to benzamide hydrochloride by stirring with 2 eq. of 4N HCl in 1,4-dioxane for 1 hrs, followed by concentration of the reaction mixture which gave 3-(3-aminopropanamido)-N-((2S)-1-oxo-3-phenyl-1-(6-(pyridin-3-yl)-5,6-dihydropyridin-1(2H)-yl)propan-2-yl)benzamide hydrochloride as a brown solid (0.89 g, 84%) MS (ESI, pos, ion) m/z: 498.2 (M+1).

Proceeding analogously as in Example 57 above, the compounds in Examples 58-59 were prepared starting with corresponding benzoic acids.

### Example 58: 3-(2-aminoacetamido)-N-((2S)-1-oxo-3-phenyl-1-(6-(pyridin-3-yl)-5,6-dihydropyridin-1(2H)-yl)propan-2-yl)benzamide hydrochloride as a beige solid (0.83 g, 83%).

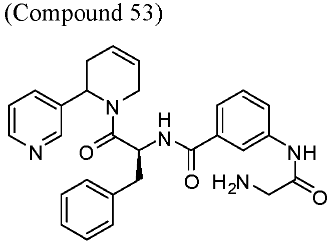

MS (ESI, pos, ion) m/z: 484.2 (M+1).

### Example 59: 3-(4-aminobutanamido)-N-((2S)-1-oxo-3-phenyl-1-(6-(pyridin-3-yl)-5,6-dihydropyridin-1(2H)-yl)propan-2-yl)benzamide hydrochloride as a beige solid (0.40 g, 36%).

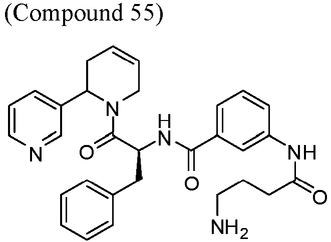

MS (ESI, pos, ion) m/z: 512.2 (M+1).

### Example 61

### Synthesis of 3-butyramido-N-((2S)-1-oxo-3-phenyl-1-(2-(pyridin-3-yl)piperidin-1-yl)propan-2-yl)benzamide (Compound 63)

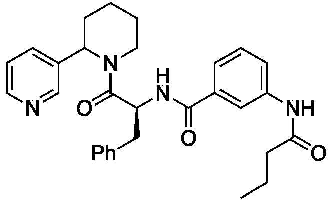

### Synthetic Scheme for 3-butyramido-N-((2S)-1-oxo-3-phenyl-1-(2-(pyridin-3-yl)piperidin-1-yl)propan-2-yl)benzamide:

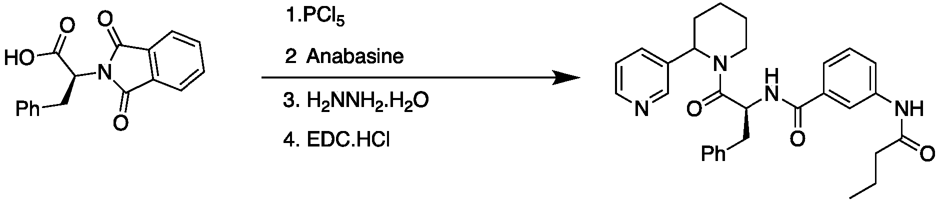

### Step 1

To a solution (S)-2-(1,3-dioxoisoindolin-2-yl)-3-phenylpropanoic acid (1.3 g, 4.4 mmol) in anhydrous DCM (20 mL) was added PCl₅ (1.0 g, 4.8 mmol) in portions. The reaction mixture was stirred for 3 hrs at rt. All volatiles were completely removed via rotovap to give (S)-2-(1,3-dioxoisoindolin-2-yl)-3-phenylpropanoyl chloride which was used in the next step without further purification.

### Step 2

To a solution of (S)-2-(1,3-dioxoisoindolin-2-yl)-3-phenylpropanoyl chloride (4.8 mmol) in anhydrous benzene (20 mL) were added (+/-)-anabasine (0.8 g, 4.9 mmol) slowly. The resulting solution was stirred at rt overnight and quenched with saturated NaHCO3 (20 mL). The layers were separated and the benzene layer was washed with brine, dried and concentrated to give 2-((2S)-1-oxo-3-phenyl-1-(2-(pyridin-3-yl)-piperidin-1-yl)propan-2-yl)isoindoline-1,3-dione as a foam (1.5 g) existing as a mixture of two diastereomers which was used in the next step without further purification.

### Step 3

To a solution of 2-((2S)-1-oxo-3-phenyl-1-(2-(pyridin-3-yl)-piperidin-1-yl)propan-2-yl)isoindoline-1,3-dione (1.5 g) in EtOH (30 mL) were added hydrazine monohydrate (0.3 mL). The reaction solution was refluxed overnight and the resulting suspension was filtered at rt. The filtrate was dissolved in a mixture of EtOAc and hexanes and was left overnight. The resulting solid was filtered off and the filtrate was concentrated to give (2S)-2-amino-3-phenyl-1-(2-(pyridin-3-yl)piperidin-1-yl)propan-1-one (0.82 g) as a mixture of two diastereomers which was used in the next step without further purification.

### Step 4

To a solution of 3-butyramidobenzoic acid (0.56 g, 2.7 mmol) and (2S)-2-amino-3-phenyl-1-(2-(pyridin-3-yl)piperidin-1-yl)propan-1-one (0.82 g, 2.7 mmol) was added EDC hydrochloride (0.78 g, 4.0 mmol) and i-Pr2NEt (0.65 mL, 4.1 mmol) in DCM (20 mL). The reaction mixture was stirred at rt overnight and quenched with H₂O (20 mL). Layers were separated and the organic layer was washed with brine, dried and concentrated to give 3-butyramido-N-((2S)-1-oxo-3-phenyl-1-(2-(pyridin-3-yl)piperidin-1-yl)propan-2-yl)benzamide as a light yellow solid (0.64 g, 32%) MS (ESI, pos. ion) m/z: 499.2 (M+1), existing as a mixture of two diastereomers after purification with CombiFlash^{®} using HP C18 Aq GOLD column with MeCN/buffered H2O as an eluting mobile phase.

### Example 62

### Synthesis of 3-butyramido-N-((2S)-3-(naphthalen-1-yl)-1-oxo-1-(6-(pyridin-3-yl)-5,6-dihydropyridin-1(2H)-yl)propan-2-yl)benzamide (Compound 64)

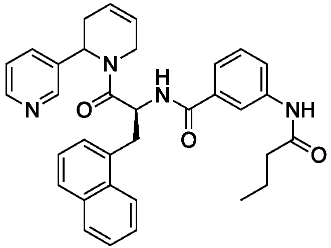

### Step 1

To a 1-necked round-bottom flask (100 mL) equipped with a condenser was charged with 3-(1-Naphthyl)-L-alanine (1.0 g, 4.6 mmol), phthalic anhydride (0.7 g, 4.6 mmol), and glacial acetic acid (10 mL) in that order. The resulting mixture was refluxed for 4 hrs. The solvent was completely removed in vacuo, and the residue was precipitated from a mixture of ethyl acetate and hexanes to give (S)-2-(1,3-dioxoisoindolin-2-yl)-3-(naphthalen-1-yl)propanoic acid (0.52 g) as a beige solid.

### Step 2

To a solution (S)-2-(1,3-dioxoisoindolin-2-yl)-3-(naphthalen-1-yl)propanoic acid (0.52 g, 1.4 mmol) in anhydrous DCM (10 mL) was added PCl₅ (0.32 g, 1.6 mmol) in portions. The reaction mixture was stirred for 3 hrs at rt. All volatiles were completely removed via rotovap to give (S)-2-(1,3-dioxoisoindolin-2-yl)-3-(naphthalen-1-yl)propanoyl chloride which was used in the next step without further purification.

### Step 3

To a solution of (S)-2-(1,3-dioxoisoindolin-2-yl)-3-(naphthalen-1-yl)propanoyl chloride (0.5 g, 1.3 mmol) in anhydrous benzene (10 mL) were added (+/-)-anatabine (0.2 g, 1.3 mmol) slowly. The resulting solution was stirred at rt overnight and quenched with saturated NaHCO₃ (10 mL). The layers were separated and the benzene layer was washed with brine, dried and concentrated to give 2-((2S)-1-oxo-3-phenyl-1-(2-(pyridin-3-yl)-piperidin-1-yl)propan-2-yl)isoindoline-1,3-dione as a brown solid (0.33 g) existing as a mixture of two diastereomers which was used in the next step without further purification.

### Step 4

To a solution of 2-((2S)-1-(3,6-dihydro-[2.3'-bipyridine]-1(2H)-yl)-3-(naphthalen-1-yl)-1-oxopropane-2-yl) isoindoline-1,3-dione (0.33g) in EtOH (10 mL) were added hydrazine monohydrate (0.1 mL). The reaction solution was refluxed for 3 hrs and the resulting suspension was filtered at rt. The filtrate was dissolved in a mixture of EtOAc and hexanes and was left overnight. The resulting solid was filtered off and the filtrate was concentrated to give (2S)-2-amino-1-(3,6-dihydro-[2,3'-bipyridin]-1(2H)-yl)-3-(naphthalen-1-yl)propan-1-one as a brown oil (0.38 g) existing as a mixture of two diastereomers which was used in the next step without further purification.

### Step 5

To a solution of 3-butyramidobenzoic acid (0.21 g, 1.0 mmol) and (2S)-2-amino-1-(3,6-dihydro-[2,3'-bipyridin]-1(2H)-yl)-3-(naphthalen-1-yl)propan-1-one (0.36 g, 1.0 mmol) was added EDC hydrochloride (0.28 g, 1.4 mmol) and i-Pr2NEt (0.23 mL, 1.4 mmol) in DCM (10 mL). The reaction mixture was stirred at rt overnight and quenched with H₂O (10 mL). The layers were separated and the organic layer was washed with brine, dried and concentrated to give 3-butyramido-N-((2S)-3-(naphthalen-1-yl)-1-oxo-1-(6-(pyridin-3-yl)-5,6-dihydropyridin-1(2H)-yl)propan-2-yl)benzamide as a yellow solid (0.19 g) MS (ESI, pos. ion) m/z: 547.2 (M+1), existing as a mixture of two diastereomers after purification with CombiFlash^{®} using HP C18 Aq GOLD column with MeCN/buffered H₂O as an eluting mobile phase.

Proceeding analogously as in Example 62 above, the compounds in Examples 63-64 were prepared starting with corresponding benzoic acids.

### Example 63: 3-butyramido-N-((2S)-3-(naphthalen-2-yl)-1-oxo-1-(6-(pyridin-3-yl)-5,6-dihydropyridin-1(2H)-yl)propan-2-yl)benzamide as a yellow solid (0.35 g). (Compound 65)

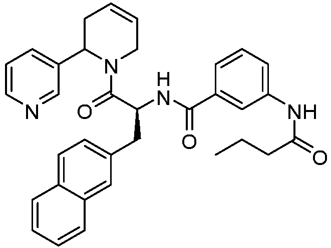

MS (ESI, pos, ion) m/z: 547.2 (M+1).

### Example 64: 3-butyramido-N-((2S)-3-cyclohexyl-1-oxo-1-(6-(pyridin-3-yl)-5,6-dihydropyridin-1(2H)-yl)propan-2-yl)benzamide as a yellow solid (0.06 g). (Compound 66)

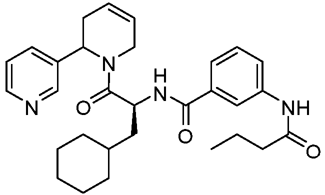

MS (ESI, pos, ion) m/z: 503.3 (M+1).

### Biological Examples

### Example 1

### Determination of inhibition of NF-kB activation induced by TNFα... in vitro assay

The ability of a compound disclosed herein to inhibit NFkB activation induced by TNFαwas determined using the TNFα Luciferase assay. The HEK293/NF-kB luciferase cell line was obtained by cotransfection of pNFkB-luc vector with pHyg (Panomics, CA, USA) followed by hygromycin selection. HEK293/NFkB Luciferase cells were grown in DMEM medium supplemented with 10% fetal bovine serum, 100U/ml of penicillin, 100 µg/ml of streptomycin/ Fungizone, and 100µg/ml of hygromycin B as the selecting agent. HEK293 NFkB Luciferase cells were grown in T75 flasks (Nunc^{™} Cell Culture Treated EasYFlasks^{™}) in a cell culture incubator at 37°C and 5%CO₂.

Confluent T75 cell culture flasks (approximately 8-10 million of cells per flask) were washed with 10 ml of sterile PBS (without calcium and magnesium) under a biosafety cabinet. After aspiration of the PBS with a sterile glass pipette, the layer of cells (for each T75 flask) was covered with 1 ml of TrypLE^{™} Express without phenol red (Gibco) at room temperature. After one minute of incubation, HEK293 were mechanically resuspended in the TrypLE by tapping the side of the flask. The cells were then resuspended with 10 ml of pre-warmed complete culture medium and transferred to a conical tube. Cells were centrifuged for 5 minutes at 4000 rpm (1000g) at room temperature. The cellular pellet was resuspended with 24 ml of pre-warmed cell culture media and 200 ml of cell suspension (approximately 80,000 cells per well) was plated per well in 96-well cell culture plates (Costar #3599, Corning, NY, USA) using a multichannel pipette and reversed pipetting to prevent air bubble formation. After plating, the cells were maintained overnight in the cell culture incubator before being treated. For each 96 well cell culture plate, 8 wells were used as "control wells" (only culture medium added) and to 8 wells only TNFα was added. The NF-kB Luciferase reporter cell line was challenged with 25 ng/ml of TNF-α for four hours in the presence and absence (control conditions) of a dose range of the different compounds to be tested. For each compound, a dose range of concentrations was tested in quadruplicate and used to determine an IC₅₀.

Luciferase activity was monitored using the Luc-Screen^{®} Extended-Glow Luciferase Reporter Gene Assay System by chemoluminescence according to the instruction of the manufacturer (Invitrogen) and a BioTek Synergy HT plate reader (BioTek Instruments, VT, USA). The IC₅₀, concentration at which NF-kB activation was reduced by 50%, for a representative number of compounds is provided in Table 3 below.

**Table 3**

| **Cpd No. (see Cpd Table 1 above)** | **IC₅₀ (µM)** | **Cpd No. (see Cpd Table 1 above)** | **IC₅₀ (µM)** | **Cpd No. (see Cpd Table 1 above)** | **IC₅₀ (µM)** |
|---|---|---|---|---|---|
| 1 | 375 | 24 | 4.0 | 46 | 0.05855 |
| 2 | 425 | 25 | 2.5 | 47 | 0.1587 |
| 3 | 750 | 26 | 2.8 | 48 | 0.1139 |
| 4 | 550 | 27 | 4.0 | 49 | 0.1741 |
| 5 | 925 | 28 | 6.5 | 50 | 0.1308 |
| 6 | 750 | 29 | 8.0 | 51 | 0.1138 |
| 7 | 225 | 30 | 4.3 | 52 | 0.1339 |
| 8 | 550 | 31 | 1.7 | 53 | 0.2124 |
| 9 | 200 | 32 | 8.6 | 54 | 0.1862 |
| 10 | 125 | 33 | 1.8 | 55 | 0.1699 |
| 11 | 250 | 34 | 2.5 | 56 | 0.9675 |
| 12 | 470 | 35 | 1.6 | 57 | 0.1208 |
| 13 | 276 | 36 | 0.15 | 58 | 0.1851 |
| 14 | 170 | 37 | 0.125 | 59 | 0.2541 |
| 15 | 4.3 | 38 | 0.25 | 60 | 0.2165 |
| 16 | 2.0 | 39 | 0.21 | 61 | 0.7082 |
| 17 | 140 | 40 | 0.04 | 63 | 0.0584 |
| 18 | 7.0 | 41 | 0.25 | 64 | 0.285 |
| 19 | 5.4 | 42 | 0.0869 | 65 | 0.7036 |
| 20 | 1.5 | 43 | 0.1288 | 66 | 0.3457 |
| 21 | 4.5 | 44 | 0.0802 | 67 | 1.7 |
| 23 | 6.5 | 45 | 0.0754 | 68 | 0.85 |

### Example 2

### Determination of Anti-inflammatory Activity using Acute LPS Model of Inflammation in Mice... in vivo model

The ability of the compounds disclosed herein to reduce inflammation was determined *in vivo* by using the above mouse model. The impact of the compounds on the production of proinflammatory cytokines induced by LPS can be evaluated in various tissues (e.g., plasma, brain, intestine, spleen, lung, etc). Adult C57Bl6/J wild-type mice were used to assess the impact of a compound disclosed herein ("test" compound) on cytokines production induced by an intraperitoneal injection of LPS (lipopolysaccharide) [LPS from Escherichia coli O111:B4, Sigma-Aldrich # L4391]. Prior to treatment with LPS (1mg/Kg (intraperitoneal injection) dissolved in sterile PBS), mice were randomized into a placebo/control group receiving an intraperitoneal (IP) injection of the vehicle used to dissolve the test compound (50% PEG4000/50% DMSO) and into a treatment group (receiving 20 mg/Kg of the test compound IP). Mice were injected with the test compound or the vehicle 15 minutes prior to the LPS injection.

Mice were then humanely euthanatized four hours after the intraperitoneal injection of LPS. Following euthanasia, blood was collected by an intracardiac puncture using EDTA as an anticoagulant. Blood was immediately centrifuged at 1500g for 4 minutes and the plasma collected and snap frozen in liquid nitrogen. All the other tissues were rapidly dissected out and snap frozen in liquid nitrogen. Samples were stored at -80°C.

Tissue homogenates were prepared by sonication in ice-cold M-PER Reagent (Pierce Biotechnology, Rockford, IL, USA) containing 1mM phenylmethanesulfonyl fluoride, 1X of protease cocktail inhibitor (Roche, Inc., USA) and 1mM sodium orthovanadate (Sigma-Aldrich, MO, USA). Cytokines were quantified by electrochemiluminescence using MULTI-SPOT plates from a V-Plex assay kit Pro-Inflammatory Panel 1 (mouse) kit (Mesoscale discovery, USA). All LPS treated samples were diluted 10X with diluent 41 from the kit and control samples were assayed without dilution. The amount of proinflammatory cytokines interferon-gamma, IL-1β, and TNFα produced was less in the treatment group versus the control group.

### Formulation Examples

The following are representative pharmaceutical formulations containing a compound of the present disclosure.

### Tablet Formulation

The following ingredients are mixed intimately and pressed into single scored tablets.

| Ingredient | Quantity per tablet mg | |
|---|---|---|
| compound of this disclosure | 400 | |
| cornstarch | 50 | |
| croscarmellose sodium | 25 | |
| lactose | 120 | |
| magnesium stearate | 5 | |

### Capsule Formulation

The following ingredients are mixed intimately and loaded into a hard-shell gelatin capsule.

| Ingredient | Quantity per capsule mg | |
|---|---|---|
| compound of this disclosure | 200 | |
| lactose spray dried | 148 | |
| magnesium stearate | 2 | |

### Injectable Formulation

Compound of the disclosure (e.g., compound 1) in 2% HPMC, 1% Tween 80 in DI water, pH 2.2 with MSA, q.s. to at least 20 mg/mL

### Inhalation Composition

To prepare a pharmaceutical composition for inhalation delivery, 20 mg of a compound disclosed herein is mixed with 50 mg of anhydrous citric acid and 100 mL of 0.9% sodium chloride solution. The mixture is incorporated into an inhalation delivery unit, such as a nebulizer, which is suitable for inhalation administration.

### Topical Gel Composition

To prepare a pharmaceutical topical gel composition, 100 mg of a compound disclosed herein is mixed with 1.75 g of hydroxypropyl cellulose, 10 mL of propylene glycol, 10 mL of isopropyl myristate and 100 mL of purified alcohol USP. The resulting gel mixture is then incorporated into containers, such as tubes, which are suitable for topical administration.

### Ophthalmic Solution Composition

To prepare a pharmaceutical ophthalmic solution composition, 100 mg of a compound disclosed herein is mixed with 0.9 g of NaCl in 100 mL of purified water and filtered using a 0.2 micron filter. The resulting isotonic solution is then incorporated into ophthalmic delivery units, such as eye drop containers, which are suitable for ophthalmic administration.

### Nasal spray solution

To prepare a pharmaceutical nasal spray solution, 10 g of a compound disclosed herein is mixed with 30 mL of a 0.05M phosphate buffer solution (pH 4.4). The solution is placed in a nasal administrator designed to deliver 100 ul of spray for each application.

## Claims

1. A compound of Formula (I): 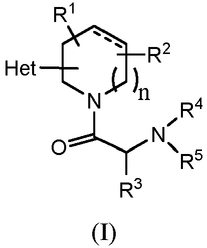 wherein:
n is 0, 1, or 2;
dashed line is an optional bond;
Het is pyridin-2-yl or pyridin-3-yl;
R¹ is hydrogen or alkyl;
R² is hydrogen or alkyl;
R³ is hydrogen, alkyl, cycloalkyl, cycloalkylalkyl, hydroxyalkyl, alkoxyalkyl, thioalkyl, alkylthioalkyl, aminoalkyl, acylaminoalkyl, carboxyalkyl, alkoxycarbonylalkyl, aminocarbonylalkyl, aryl, aralkyl, heteroaryl, or heteroaralkyl, wherein aryl or heteroaryl by itself or as part of aralkyl or heteroaralkyl is optionally substituted with R^{d}, R^{e}, and/or Rfindependently selected from alkyl, alkoxy, hydroxy, halo, haloalkyl, haloalkoxy, cyano, nitro, carboxy, alkoxycarbonyl, amino, alkylamino, and dialkylamino;
R⁴ is hydrogen or alkyl; and
R⁵ is -C(O)R⁶ where R⁶ is alkyl, aryl, aralkyl, heteroaryl, heteroaralkyl, cycloalkyl, cycloalkylalkyl, heterocycloalkyl, or heterocycloalkylalkyl wherein aryl or heteroaryl by itself or as part of aralkyl or heteroaralkyl is optionally substituted with R^{g}, R^{h}, and/or Rⁱ wherein R^{g}, R^{h}, and/or Rⁱ are each independently selected from alkyl, alkoxy, aminoalkoxy, hydroxyalkoxy, alkoxyalkoxy, cycloalkyloxy, cycloalkylalkyloxy, optionally substituted aryloxy, optionally substituted aralkyloxy, optionally substituted heteroaryloxy, optionally substituted heteroaralkyloxy, optionally substituted heterocycloalkyloxy, optionally substituted heterocycloalkylalkyloxy, halo, haloalkyl, haloalkoxy, cyano, nitro, hydroxy, carboxy, alkoxycarbonyl, amino, alkylamino, dialkylamino, acylamino, and sulfonylamino; or
R⁴ and R⁵ together with the nitrogen atom to which they are attached form a 5 to 7 membered heterocycloamino ring; or
a pharmaceutically acceptable salt thereof;
wherein "alkyl" means a linear saturated monovalent hydrocarbon radical of one to six carbon atoms or a branched saturated monovalent hydrocarbon radical of three to six carbon atoms;
wherein "heteroaryl" means a monovalent monocyclic or bicyclic aromatic radical of 5 to 10 ring atoms, where one or more ring atoms are heteroatom selected from N, O, or S, the remaining ring atoms being carbon;
wherein "optionally substituted aryloxy" means -O-alkylene-R radical where R is optionally substituted aryl;
wherein "aryl" means a monocyclic or bicyclic aromatic hydrocarbon radical of 6 to 10 ring atoms;
wherein "optionally substituted aryl" means aryl as defined above that is optionally substituted with one, two, or three substituents independently selected from alkyl, hydroxyl, cycloalkyl, carboxy, alkoxycarbonyl, hydroxy, alkoxy, alkylthio, alkylsulfonyl, amino, alkylamino, dialkylamino, halo, haloalkyl, haloalkoxy, and cyano;
wherein "optionally substituted aralkyloxy" means -O-alkylene-R radical where R is optionally substituted aryl as defined above;
wherein "optionally substituted heteroaryloxy" means -O-R radical where R is optionally substituted heteroaryl;
wherein "optionally substituted heteroaryl" means heteroaryl as defined above that is optionally substituted with one, two, or three substituents independently selected from alkyl, alkylthio, alkylsulfonyl, hydroxyl, cycloalkyl, carboxy, alkoxycarbonyl, hydroxy, alkoxy, halo, haloalkyl, haloalkoxy, amino, alkylamino, dialkylamino, and cyano;
wherein "optionally substituted heteroaralkyloxy" means -O-alkylene-R radical where R is optionally substituted heteroaryl as defined above;
wherein "optionally substituted heterocycloalkyloxy" means -O-R radical where R is optionally substituted heterocycloalkyl;
wherein "optionally substituted heterocycloalkyl" means heterocycloalkyl that is optionally substituted with one, two, or three substituents independently selected from alkyl, alkylthio, alkylsulfonyl, hydroxyl, cycloalkyl, carboxy, alkoxycarbonyl, hydroxy, hydroxyalkyl, alkoxy, alkoxyalkyl, aminoalkyl, halo, haloalkyl, haloalkoxy, and cyano;
wherein"heterocycloalkyl" means a saturated or unsaturated monovalent monocyclic group of 4 to 8 ring atoms in which one, two, or three ring atoms are heteroatom selected from N, O, or S(O)ₙ, where n is an integer from 0 to 2, the remaining ring atoms being C; wherein one or two ring carbon atoms in the heterocyclyl ring can optionally be replaced by a -CO- group and heterocycloalkyl is optionally fused to phenyl or 5- or 6-membered heteroaryl as defined above;
wherein "optionally substituted heterocycloalkylalkyloxy" means -O-alkylene-R radical where R is optionally substituted heterocycloalkyl as defined above.

2. The compound of claim 1 wherein Het is pyridin-3-yl.

3. The compound of claim 1 or claim 2 wherein the compound has structure (IA):

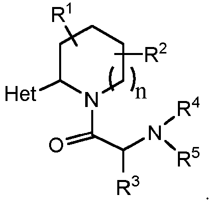

4. The compound of claim 1 or claim 2 wherein the compound has structure (IB):

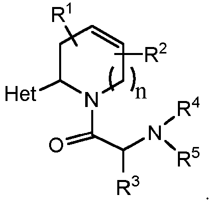

5. The compound of any one of claims 1-4 wherein (i) n is 1 or 2;
(ii) n is1; (iii) n is 0 or 2; (iv) n is 0 or; (v) n is 2.

6. The compound of any one of claims 1-5 wherein R⁴ is hydrogen or alkyl and R⁵ is -C(O)R⁶ where R⁶ is alkyl, aryl, aralkyl, heteroaryl, heteroaralkyl, cycloalkyl, or heterocycloalkyl wherein aryl or heteroaryl by itself or as part of aralkyl or heteroaralkyl are optionally substituted with R^{g}, R^{h}, and/or Rⁱ independently selected from alkyl, alkoxy, aminoalkoxy, hydroxyalkoxy, alkoxyalkoxy, cycloalkyloxy, cycloalkylalkyloxy, optionally substituted aralkyloxy, optionally substituted heteroaryloxy, optionally substituted heterocycloalkyloxy, optionally substituted heterocycloalkylalkyloxy, halo, haloalkyl, haloalkoxy, cyano, nitro, hydroxy, carboxy, alkoxycarbonyl, amino, alkylamino, dialkylamino, acylamino, and sulfonylamino.

7. The compound of claim 6 wherein:
(i) R⁵ is aryl or heteroaryl optionally substituted with R^{g}, R^{h}, and/or Rⁱ; or
(ii) R⁵ is aralkyl or heteroaralkyl optionally substituted with R^{g}, R^{h}, and/or Rⁱ; or
(iii) R⁵ is phenyl optionally substituted with R^{g}, R^{h}, and/or Rⁱ.

8. The compound of any one of claims 1 to 7 wherein:
(i) R^{g} is alkyl, alkoxy, halo, haloalkyl, haloalkoxy, hydroxy, or cyano and R^{h} and Rⁱ are independently selected from alkyl, alkoxy, aminoalkoxy, hydroxyalkoxy, alkoxyalkoxy, cycloalkyloxy, cycloalkylalkyloxy, optionally substituted aralkyloxy, optionally substituted heteroaryloxy, optionally substituted heterocycloalkyloxy, optionally substituted heterocycloalkylalkyloxy, halo, haloalkyl, haloalkoxy, cyano, hydroxy, carboxy, alkoxycarbonyl, amino, alkylamino, dialkylamino, acylamino, or sulfonylamino; or
(ii) R^{g}, R^{h} and Rⁱ are independently selected from alkyl, alkoxy, halo, haloalkyl, haloalkoxy, cyano, hydroxy, amino, acylamino, preferably, methyl, ethyl, methoxy, ethoxy, chloro, fluoro, trifluoromethyl, trifluoromethoxy, hydroxy, acetylamino, butanoylamino, and pentanoylamino.

9. The compound of any one of claims 1 to 5 wherein R⁴ and R⁵ together with the nitrogen atom to which they are attached form a 5 to 7 membered heterocycloamino ring.

10. The compound of any one of claims 1 to 9 wherein R¹ and R² are independently hydrogen or methyl, optionally hydrogen.

11. The compound of any one of claims 1 to 10 wherein when both R¹ and R² are alkyl, they are not bound to the same ring carbon.

12. The compound of any one of claims 1 to 11 wherein:
(i) R³ is hydrogen or alkyl, preferably methyl, ethyl, propyl, isopropyl, *sec*-propyl, *n-, sec-, iso-,* or *tert*-butyl;
(ii) R³ is aralkyl optionally substituted with R^{d}, R^{e}, and/or R^{f}, preferably R³ is benzyl or phenethyl, more preferably benzyl optionally substituted with R^{d}, R^{e}, and/or R^{f}, even more preferably R³ is benzyl;
(iii) R³ is cycloalkylalkyl optionally substituted with R^{d}, R^{e}, and/or R^{f}, preferably R³ is cyclopropylmethyl, cyclobutylmethyl, cyclopentylmethyl, or cyclohexylmethyl optionally substituted with R^{d}, R^{e}, and/or R^{f};
(iv) R³ is heteroaralkyl (e.g., thienylmethyl, furanylmethyl, pyridinylmethyl, quinolinylmethyl, isoquinolinylmethyl, indolylmethyl, or indazolylmethyl) optionally substituted with R^{d}, R^{e}, and/or R^{f}; or
(v) R³ is hydroxyalkyl, alkoxyalkyl, aminoalkyl, preferably R³ is hydroxymethyl, hydroxyethyl, methoxymethyl, methoxyethyl, aminomethyl, or aminobutyl.

13. The compound of any one of claims 1 to 12 wherein:
(i) the stereochemistry at carbon to which R³ is attached is (S); or
(ii) the stereochemistry at carbon to which R³ is attached is (R).

14. A pharmaceutical composition comprising a compound of any one of claims 1-13 or a pharmaceutically acceptable salt thereof, and a pharmaceutically acceptable excipient.

15. The compound or pharmaceutically acceptable salt thereof as claimed in any one of claims 1-13 or the pharmaceutical composition according to claim 14 for use in the treatment of a disease treatable by inhibiting NF-kB activation, wherein
(i) the disease is an inflammatory disease, optionally wherein said disease is selected from the goup consisting of autoimmune disease, pain, allergies, asthma, chronic obstructive pulmonary disease and sepsis; or
(ii) the disease is selected from the group consisting of rheumatoid arthritis, osteoarthritis, atherosclerosis, multiple sclerosis, asthma, inflammatory bowel disease, diabetes, Parkinson's disease, Alzheimer's disease, amyotrophic lateral sclerosis, osteoporosis, systemic lupus erythematosus, chronic obstructive pulmonary disease, cystic fibrosis, stroke, acute kidney injury, glomerulonephritis, psoriasis, atopic dermatitis, Behcet's disease, tuberculosis, Crohn's disease, colitis, Pagett's disease, pancreatitis, periodonitis, inflammatory lung disease, and lupus nephritis.

## Patentansprüche

1. Eine Verbindung der Formel (I): 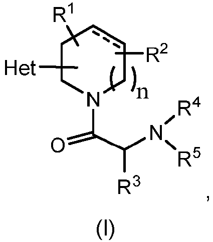 wobei:
n 0, 1 oder 2 ist;
die gestrichelte Linie eine optionale Bindung ist;
Het Pyridin-2-yl oder Pyridin-3-yl ist;
R¹ Wasserstoff oder Alkyl ist;
R² Wasserstoff oder Alkyl ist;
R³ Wasserstoff, Alkyl, Cycloalkyl, Cycloalkylalkyl, Hydroxyalkyl, Alkoxyalkyl, Thioalkyl, Alkylthioalkyl, Aminoalkyl, Acylaminoalkyl, Carboxyalkyl, Alkoxycarbonylalkyl, Aminocarbonylalkyl, Aryl, Aralkyl, Heteroaryl oder Heteroaralkyl ist, wobei Aryl oder Heteroaryl für sich allein oder als Teil von Aralkyl oder Heteroaralkyl optional mit R^{d}, R^{e} und/oder R^{f} substituiert ist, die unabhängig aus Alkyl, Alkoxy, Hydroxy, Halogen, Halogenalkyl, Halogenalkoxy, Cyan, Nitro, Carboxy, Alkoxycarbonyl, Amino, Alkylamino und Dialkylamino ausgewählt sind;
R⁴ Wasserstoff oder Alkyl ist; und
R⁵ -C(O)R⁶ ist, wobei R⁶ Alkyl, Aryl, Aralkyl, Heteroaryl, Heteroaralkyl, Cycloalkyl, Cycloalkylalkyl, Heterocycloalkyl oder Heterocycloalkylalkyl ist, wobei Aryl oder Heteroaryl für sich allein oder als Teil von Aralkyl oder Heteroaralkyl optional mit R^{g}, R^{h} und/oder Rⁱ substituiert ist, wobei R^{g}, R^{h} und/oder Rⁱ jeweils unabhängig aus Alkyl, Alkoxy, Aminoalkoxy, Hydroxyalkoxy, Alkoxyalkoxy, Cycloalkyloxy, Cycloalkylalkyloxy, optional substituiertem Aryloxy, optional substituiertem Aralkyloxy, optional substituiertem Heteroaryloxy, optional substituiertem Heteroaralkyloxy, optional substituiertem Heterocycloalkyloxy, optional substituiertem Heterocycloalkylalkyloxy, Halogen, Halogenalkyl, Halogenalkoxy, Cyan, Nitro, Hydroxy, Carboxy, Alkoxycarbonyl, Amino, Alkylamino, Dialkylamino, Acylamino und Sulfonylamino ausgewählt sind; oder
R⁴ und R⁵ zusammen mit dem Stickstoffatom, mit dem sie verbunden sind, einen 5- bis 7-gliedrigen Heterocycloamino-Ring bilden; oder
ein pharmazeutisch annehmbares Salz davon;
wobei "Alkyl" ein lineares gesättigtes einwertiges Kohlenwasserstoffradikal mit einem bis sechs Kohlenstoffatomen oder ein verzweigtes gesättigtes einwertiges Kohlenwasserstoffradikal mit drei bis sechs Kohlenstoffatomen bedeutet;
wobei "Heteroaryl" ein einwertiges monozyklisches oder bizyklisches aromatisches Radikal mit 5 bis 10 Ringatomen bedeutet, wobei ein oder mehrere Ringatome ein aus N, O und S ausgewähltes Heteroatom sind, wobei die übrigen Ringatome Kohlenstoff sind;
wobei "optional substituiertes Aryloxy" ein -O-Alkylen-R-Radikal bedeutet, wobei R ein optional substituiertes Aryl ist;
wobei "Aryl" ein monozyklisches oder bizyklisches aromatisches Kohlenwasserstoffradikal mit 6 bis 10 Ringatomen bedeutet;
wobei "optional substituiertes Aryl" ein Aryl wie oben definiert bedeutet, das optional mit einem, zwei oder drei Substituenten substituiert ist, die unabhängig aus Alkyl, Hydroxyl, Cycloalkyl, Carboxy, Alkoxycarbonyl, Hydroxy, Alkoxy, Alkylthio, Alkylsulfonyl, Amino, Alkylamino, Dialkylamino, Halogen, Halogenalkyl, Halogenalkoxy und Cyan ausgewählt sind;
wobei "optional substituiertes Aralkyloxy" ein -O-Alkylen-R-Radikal bedeutet, wobei R ein optional substituiertes Aryl wie oben definiert ist;
wobei "optional substituiertes Heteroaryloxy" ein -O-R-Radikal bedeutet, wobei R ein optional substituiertes Heteroaryl ist;
wobei "optional substituiertes Heteroaryl" ein Heteroaryl wie oben definiert bedeutet, das optional mit einem, zwei oder drei Substituenten substituiert ist, die unabhängig aus Alkyl, Alkylthio, Alkylsulfonyl, Hydroxyl, Cycloalkyl, Carboxy, Alkoxycarbonyl, Hydroxy, Alkoxy, Halogen, Halogenalkyl, Halogenalkoxy, Amino, Alkylamino, Dialkylamino und Cyan ausgewählt sind;
wobei "optional substituiertes Heteroaralkyloxy" ein -O-Alkylen-R-Radikal bedeutet,
wobei R ein optional substituiertes Heteroaryl wie oben definiert ist;
wobei "optional substituiertes Heterocycloalkyloxy" ein -O-R-Radikal bedeutet, wobei R ein optional substituiertes Heterocycloalkyl ist;
wobei "optional substituiertes Heterocycloalkyl" ein Heterocycloalkyl bedeutet, das optional mit einem, zwei oder drei Substituenten substituiert ist, die unabhängig aus Alkyl, Alkylthio, Alkylsulfonyl, Hydroxyl, Cycloalkyl, Carboxy, Alkoxycarbonyl, Hydroxy, Hydroxyalkyl, Alkoxy, Alkoxyalkyl, Aminoalkyl, Halogen, Halogenalkyl, Halogenalkoxy und Cyan ausgewählt sind;
wobei "Heterocycloalkyl" eine gesättigte oder ungesättigte einwertige monozyklische Gruppe von 4 bis 8 Ringatomen bedeutet, in der ein, zwei oder drei Ringatome ein aus N, O und S(O)ₙ ausgewähltes Heteroatom sind, wobei n eine ganze Zahl von 0 bis 2 ist, wobei die übrigen Ringatome C sind; wobei ein oder zwei Ringkohlenstoffatome in dem Heterocyclyl-Ring optional durch eine -CO-Gruppe ersetzt sein können und Heterocycloalkyl optional an Phenyl oder 5- oder 6-gliedriges Heteroaryl wie oben definiert kondensiert ist;
wobei "optional substituiertes Heterocycloalkylalkyloxy" ein -O-Alkylen-R-Radikal bedeutet, wobei R ein optional substituiertes Heterocycloalkyl wie oben definiert ist.

2. Verbindung gemäß Anspruch 1, wobei Het Pyridin-3-yl ist.

3. Verbindung gemäß Anspruch 1 oder Anspruch 2, wobei die Verbindung die Struktur (IA) aufweist:

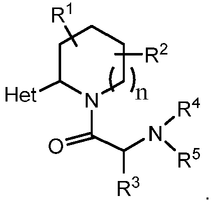

4. Verbindung gemäß Anspruch 1 oder Anspruch 2, wobei die Verbindung die Struktur (IB) aufweist:

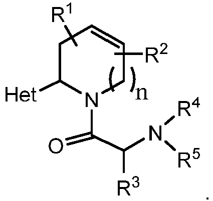

5. Verbindung gemäß einem der Ansprüche 1-4, wobei (i) n 1 oder 2 ist;
(ii) n 1 ist; (iii) n 0 oder 2 ist; (iv) n 0 ist oder (v) n 2 ist.

6. Verbindung gemäß einem der Ansprüche 1-5, wobei R⁴ Wasserstoff oder Alkyl ist und R⁶ -C(O)R⁶ ist, wobei R⁶ Alkyl, Aryl, Aralkyl, Heteroaryl, Heteroaralkyl, Cycloalkyl oder Heterocycloalkyl ist, wobei Aryl oder Heteroaryl für sich allein oder als Teil von Aralkyl oder Heteroaralkyl optional mit R^{g}, R^{h} und/oder Rⁱ substituiert sind, die unabhängig aus Alkyl, Alkoxy, Aminoalkoxy, Hydroxyalkoxy, Alkoxyalkoxy, Cycloalkyloxy, Cycloalkylalkyloxy, optional substituiertem Aralkyloxy, optional substituiertem Heteroaryloxy, optional substituiertem Heterocycloalkyloxy, optional substituiertem Heterocycloalkylalkyloxy, Halogen, Halogenalkyl, Halogenalkoxy, Cyan, Nitro, Hydroxy, Carboxy, Alkoxycarbonyl, Amino, Alkylamino, Dialkylamino, Acylamino und Sulfonylamino ausgewählt sind.

7. Verbindung gemäß Anspruch 6, wobei:
(i) R⁵ Aryl oder Heteroaryl ist, das optional mit R^{g}, R^{h} und/oder Rⁱ substituiert ist; oder
(ii) R⁵ Aralkyl oder Heteroaralkyl ist, das optional mit R^{g}, R^{h} und/oder Rⁱ substituiert ist; oder
(iii) R⁵ Phenyl ist, das optional mit R^{g}, R^{h} und/oder Rⁱ substituiert ist.

8. Verbindung gemäß einem der Ansprüche 1 bis 7, wobei:
(i) R⁹ Alkyl, Alkoxy, Halogen, Halogenalkyl, Halogenalkoxy, Hydroxy oder Cyan ist und R^{h} und Rⁱ unabhängig aus Alkyl, Alkoxy, Aminoalkoxy, Hydroxyalkoxy, Alkoxyalkoxy, Cycloalkyloxy, Cycloalkylalkyloxy, optional substituiertem Aralkyloxy, optional substituiertem Heteroaryloxy, optional substituiertem Heterocycloalkyloxy, optional substituiertem Heterocycloalkylalkyloxy, Halogen, Halogenalkyl, Halogenalkoxy, Cyan, Hydroxy, Carboxy, Alkoxycarbonyl, Amino, Alkylamino, Dialkylamino, Acylamino oder Sulfonylamino ausgewählt sind; oder
(ii) R^{g}, R^{h} und Rⁱ unabhängig aus Alkyl, Alkoxy, Halogen, Halogenalkyl, Halogenalkoxy, Cyan, Hydroxy, Amino, Acylamino, vorzugsweise, Methyl, Ethyl, Methoxy, Ethoxy, Chlor, Fluor, Trifluormethyl, Trifluormethoxy, Hydroxy, Acetylamino, Butanoylamino und Pentanoylamino ausgewählt sind.

9. Verbindung gemäß einem der Ansprüche 1 bis 5, wobei R⁴ und R⁵ zusammen mit dem Stickstoffatom, mit dem sie verbunden sind, einen 5- bis 7-gliedrigen Heterocycloamino-Ring bilden.

10. Verbindung gemäß einem der Ansprüche 1 bis 9, wobei R¹ und R² unabhängig Wasserstoff oder Methyl, optional Wasserstoff sind.

11. Verbindung gemäß einem der Ansprüche 1 bis 10, wobei, wenn sowohl R¹ als auch R² Alkyl sind, sie nicht an denselben Ringkohlenstoff gebunden sind.

12. Verbindung gemäß einem der Ansprüche 1 bis 11, wobei:
(i) R³ Wasserstoff oder Alkyl, vorzugsweise Methyl, Ethyl, Propyl, Isopropyl, sec-Propyl, *n-, sec-, iso-* oder *tert*-Butyl ist;
(ii) R³ Aralkyl ist, das optional mit R^{d}, R^{e} und/oder R^{f} substituiert ist, R³ vorzugsweise Benzyl oder Phenethyl, bevorzugter optional mit R^{d}, R^{e} und/oder R^{f} substituiertes Benzyl ist, R³ noch bevorzugter Benzyl ist;
(iii) R³ Cycloalkylalkyl ist, das optional mit R^{d}, R^{e} und/oder R^{f} substituiert ist, R³ vorzugsweise optional mit R^{d}, R^{e} und R^{f} substituiertes Cyclopropylmethyl, Cyclobutylmethyl, Cyclopentylmethyl oder Cyclohexylmethyl ist;
(iv) R³ Heteroaralkyl (z. B. Thienylmethyl, Furanylmethyl, Pyridinylmethyl, Chinolinylmethyl, Isochinolinylmethyl, Indolylmethyl oder Indazolylmethyl) ist, das optional mit R^{d}, R^{e} und/oder R^{f} substituiert ist; oder
(v) R³ Hydroxyalkyl, Alkoxyalkyl, Aminoalkyl ist, R³ vorzugsweise Hydroxymethyl, Hydroxyethyl, Methoxymethyl, Methoxyethyl, Aminomethyl oder Aminobutyl ist.

13. Verbindung gemäß einem der Ansprüche 1 bis 12, wobei:
(i) die Stereochemie an dem Kohlenstoff, mit dem R³ verbunden ist, (S) ist; oder
(ii) die Stereochemie an dem Kohlenstoff, mit dem R³ verbunden ist, (R) ist.

14. Eine pharmazeutische Zusammensetzung, beinhaltend eine Verbindung gemäß einem der Ansprüche 1-13 oder ein pharmazeutisch annehmbares Salz davon und einen pharmazeutisch annehmbaren Hilfsstoff.

15. Verbindung oder pharmazeutisch annehmbares Salz davon gemäß einem der Ansprüche 1-13 oder pharmazeutische Zusammensetzung gemäß Anspruch 14 zur Verwendung bei der Behandlung einer Krankheit, die durch Hemmen der NF-kB-Aktivierung behandelbar ist, wobei
(i) die Krankheit eine entzündliche Krankheit ist, wobei die Krankheit optional aus der Gruppe ausgewählt ist, die aus Autoimmunkrankheit, Schmerzen, Allergien, Asthma, chronisch obstruktiver Lungenkrankheit und Sepsis besteht; oder
(ii) die Krankheit aus der Gruppe ausgewählt ist, die aus rheumatoider Arthritis, Osteoarthritis, Atherosklerose, Multipler Sklerose, Asthma, entzündlicher Darmkrankheit, Diabetes, Morbus Parkinson, Morbus Alzheimer, amyotropher Lateralsklerose, Osteoporose, systemischem Lupus erythematodes, chronisch obstruktiver Lungenkrankheit, Mukoviszidose, Schlaganfall, akutem Nierenversagen, Glomerulonephritis, Psoriasis, atopischer Dermatitis, Morbus Behçet, Tuberkulose, Morbus Crohn, Colitis, Morbus Paget, Pankreatitis, Periodontitis, entzündlicher Lungenkrankheit und Lupus-Nephritis besteht.

## Revendications

1. Un composé de Formule (I) : 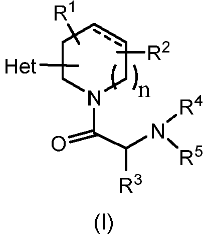 dans lequel :
n vaut 0, 1, ou 2 ;
la ligne en pointillés est une liaison facultative ;
Het est un pyridin-2-yle ou un pyridin-3-yle ;
R¹ est l'hydrogène ou un alkyle ;
R² est l'hydrogène ou un alkyle ;
R³ est l'hydrogène, un alkyle, un cycloalkyle, un cycloalkylalkyle, un hydroxyalkyle, un alcoxyalkyle, un thioalkyle, un alkylthioalkyle, un aminoalkyle, un acylaminoalkyle, un carboxyalkyle, un alcoxycarbonylalkyle, un aminocarbonylalkyle, un aryle, un aralkyle, un hétéroaryle, ou un hétéroaralkyle, dans lequel un aryle ou un hétéroaryle en lui-même ou en tant que partie d'un aralkyle ou d'un hétéroaralkyle est facultativement substitué par R^{d}, R^{e}, et/ou R^{f} indépendamment sélectionnés parmi un alkyle, un alcoxy, un hydroxy, un halo, un haloalkyle, un haloalcoxy, un cyano, un nitro, un carboxy, un alcoxycarbonyle, un amino, un alkylamino, et un dialkylamino ;
R⁴ est l'hydrogène ou un alkyle ; et
R⁵ est -C(O)R⁶ où R⁶ est un alkyle, un aryle, un aralkyle, un hétéroaryle, un hétéroaralkyle, un cycloalkyle, un cycloalkylalkyle, un hétérocycloalkyle, ou un hétérocycloalkylalkyle, dans lequel un aryle ou un hétéroaryle en lui-même ou en tant que partie d'un aralkyle ou d'un hétéroaralkyle est facultativement substitué par R^{g}, R^{h}, et/ou Rⁱ, dans lequel R^{g}, R^{h},et/ou Rⁱ sont chacun indépendamment sélectionnés parmi un alkyle, un alcoxy, un aminoalcoxy, un hydroxyalcoxy, un alcoxyalcoxy, un cycloalkyloxy, un cycloalkylalkyloxy, un aryloxy facultativement substitué, un aralkyloxy facultativement substitué, un hétéroaryloxy facultativement substitué, un hétéroaralkyloxy facultativement substitué, un hétérocycloalkyloxy facultativement substitué, un hétérocycloalkylalkyloxy facultativement substitué, un halo, un haloalkyle, un haloalcoxy, un cyano, un nitro, un hydroxy, un carboxy, un alcoxycarbonyle, un amino, un alkylamino, un dialkylamino, un acylamino, et un sulfonylamino ; ou
R⁴ et R⁵ conjointement avec l'atome d'azote auquel ils sont attachés forment un anneau hétérocycloamino de 5 à 7 chaînons ; ou
un sel pharmaceutiquement acceptable de celui-ci ;
dans lequel un « alkyle » désigne un radical hydrocarbure monovalent saturé linéaire de un à six atomes de carbone ou un radical hydrocarbure monovalent saturé ramifié de trois à six atomes de carbone ;
dans lequel un « hétéroaryle » désigne un radical aromatique monocyclique ou bicyclique monovalent de 5 à 10 atomes d'anneau, où un ou plusieurs atomes d'anneau sont un hétéroatome sélectionné parmi N, O, ou S, les atomes d'anneau restants étant le carbone ;
dans lequel un « aryloxy facultativement substitué » désigne un radical -O-alkylène-R où R est un aryle facultativement substitué ;
dans lequel un « aryle » désigne un radical hydrocarbure aromatique monocyclique ou bicyclique de 6 à 10 atomes d'anneau ;
dans lequel un « aryle facultativement substitué » désigne un aryle tel que défini ci-dessus qui est facultativement substitué par un, deux, ou trois substituants indépendamment sélectionnés parmi un alkyle, un hydroxyle, un cycloalkyle, un carboxy, un alcoxycarbonyle, un hydroxy, un alcoxy, un alkylthio, un alkylsulfonyle, un amino, un alkylamino, un dialkylamino, un halo, un haloalkyle, un haloalcoxy, et un cyano ;
dans lequel un « aralkyloxy facultativement substitué » désigne un radical -O-alkylène-R où R est un aryle facultativement substitué tel que défini ci-dessus ;
dans lequel un « hétéroaryloxy facultativement substitué » désigne un radical -O-R où R est un hétéroaryle facultativement substitué ;
dans lequel un « hétéroaryle facultativement substitué » désigne un hétéroaryle tel que défini ci-dessus qui est facultativement substitué par un, deux, ou trois substituants indépendamment sélectionnés parmi un alkyle, un alkylthio, un alkylsulfonyle, un hydroxyle, un cycloalkyle, un carboxy, un alcoxycarbonyle, un hydroxy, un alcoxy, un halo, un haloalkyle, un haloalcoxy, un amino, un alkylamino, un dialkylamino, et un cyano ;
dans lequel un « hétéroaralkyloxy facultativement substitué » désigne un radical -O-alkylène-R où R est un hétéroaryle facultativement substitué tel que défini ci-dessus ;
dans lequel un « hétérocycloalkyloxy facultativement substitué » désigne un radical -O-R où R est un hétérocycloalkyle facultativement substitué ;
dans lequel un « hétérocycloalkyle facultativement substitué » désigne un hétérocycloalkyle qui est facultativement substitué par un, deux, ou trois substituants indépendamment sélectionnés parmi un alkyle, un alkylthio, un alkylsulfonyle, un hydroxyle, un cycloalkyle, un carboxy, un alcoxycarbonyle, un hydroxy, un hydroxyalkyle, un alcoxy, un alcoxyalkyle, un aminoalkyle, un halo, un haloalkyle, un haloalcoxy, et un cyano ;
dans lequel un « hétérocycloalkyle » désigne un groupe monocyclique monovalent saturé ou insaturé de 4 à 8 atomes d'anneau dans lequel un, deux, ou trois atomes d'anneau sont un hétéroatome sélectionné parmi N, O, ou S(O)ₙ, où n est un nombre entier allant de 0 à 2, les atomes d'anneau restants étant C ; dans lequel un ou deux atomes de carbone d'anneau dans l'anneau hétérocyclyle peuvent facultativement être remplacés par un groupe -CO- et un hétérocycloalkyle est facultativement condensé en un phényle ou un hétéroaryle de 5 à 6 chaînons tel que défini ci-dessus ;
dans lequel un « hétérocycloalkylalkyloxy facultativement substitué » désigne un radical -O-alkylène-R où R est un hétérocycloalkyle facultativement substitué tel que défini ci-dessus.

2. Le composé de la revendication 1 dans lequel Het est un pyridin-3-yle.

3. Le composé de la revendication 1 ou de la revendication 2, le composé ayant la structure (IA) :

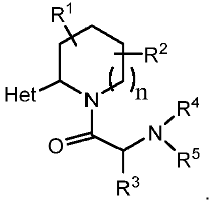

4. Le composé de la revendication 1 ou de la revendication 2, le composé ayant la structure (IB) :

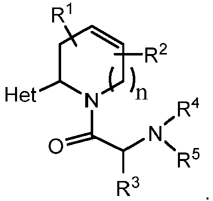

5. Le composé de l'une quelconque des revendications 1 à 4 dans lequel (i) n vaut 1 ou 2 ;
(ii) n vaut 1 ; (iii) n vaut 0 ou 2 ; (iv) n vaut 0 ou ; (v) n vaut 2.

6. Le composé de l'une quelconque des revendications 1 à 5 dans lequel R⁴ est l'hydrogène ou un alkyle et R⁵ est -C(O)R⁶ où R⁶ est un alkyle, un aryle, un aralkyle, un hétéroaryle, un hétéroaralkyle, un cycloalkyle, ou un hétérocycloalkyle, dans lequel un aryle ou un hétéroaryle en lui-même ou en tant que partie d'un aralkyle ou d'un hétéroaralkyle est facultativement substitué par R^{g}, R^{h},et/ou Rⁱ indépendamment sélectionnés parmi un alkyle, un alcoxy, un aminoalcoxy, un hydroxyalcoxy, un alcoxyalcoxy, un cycloalkyloxy, un cycloalkylalkyloxy, un aralkyloxy facultativement substitué, un hétéroaryloxy facultativement substitué, un hétérocycloalkyloxy facultativement substitué, un hétérocycloalkylalkyloxy facultativement substitué, un halo, un haloalkyle, un haloalcoxy, un cyano, un nitro, un hydroxy, un carboxy, un alcoxycarbonyle, un amino, un alkylamino, un dialkylamino, un acylamino, et un sulfonylamino.

7. Le composé de la revendication 6 dans lequel :
(i) R⁵ est un aryle ou un hétéroaryle facultativement substitué par R^{g}, R^{h}, et/ou Rⁱ ; ou
(ii) R⁵ est un aralkyle ou un hétéroaralkyle facultativement substitué par R^{g}, R^{h}, et/ou Rⁱ; ou
(iii) R⁵ est un phényle facultativement substitué par R^{g}, R^{h},et/ou Rⁱ.

8. Le composé de l'une quelconque des revendications 1 à 7 dans lequel :
(i) R⁹ est un alkyle, un alcoxy, un halo, un haloalkyle, un haloalcoxy, un hydroxy, ou un cyano et R^{h} et Rⁱ sont indépendamment sélectionnés parmi un alkyle, un alcoxy, un aminoalcoxy, un hydroxyalcoxy, un alcoxyalcoxy, un cycloalkyloxy, un cycloalkylalkyloxy, un aralkyloxy facultativement substitué, un hétéroaryloxy facultativement substitué, un hétérocycloalkyloxy facultativement substitué, un hétérocycloalkylalkyloxy facultativement substitué, un halo, un haloalkyle, un haloalcoxy, un cyano, un hydroxy, un carboxy, un alcoxycarbonyle, un amino, un alkylamino, un dialkylamino, un acylamino, ou un sulfonylamino ; ou
(ii) R^{g}, R^{h} et Rⁱ sont indépendamment sélectionnés parmi un alkyle, un alcoxy, un halo, un haloalkyle, un haloalcoxy, un cyano, un hydroxy, un amino, un acylamino, de préférence, un méthyle, un éthyle, un méthoxy, un éthoxy, un chloro, un fluoro, un trifluorométhyle, un trifluorométhoxy, un hydroxy, un acétylamino, un butanoylamino, et un pentanoylamino.

9. Le composé de l'une quelconque des revendications 1 à 5 dans lequel R⁴ et R⁵ conjointement avec l'atome d'azote auquel ils sont attachés forment un anneau hétérocycloamino de 5 à 7 chaînons.

10. Le composé de l'une quelconque des revendications 1 à 9 dans lequel R¹ et R² sont indépendamment l'hydrogène ou un méthyle, facultativement l'hydrogène.

11. Le composé de l'une quelconque des revendications 1 à 10 dans lequel lorsque R¹ et R² sont tous les deux un alkyle, ils ne sont pas liés au même carbone d'anneau.

12. Le composé de l'une quelconque des revendications 1 à 11 dans lequel :
(i) R³ est l'hydrogène ou un alkyle, de préférence un méthyle, un éthyle, un propyle, un isopropyle, un sec-propyle, un *n-, sec-, iso-,* ou *tert*-butyle ;
(ii) R³ est un aralkyle facultativement substitué par R^{d}, R^{e}, et/ou R^{f}, de préférence R³ est un benzyle ou un phénéthyle, plus préférablement un benzyle facultativement substitué par R^{d}, R^{e}, et/ou R^{f}, encore plus préférablement R³ est un benzyle ;
(iii) R³ est un cycloalkylalkyle facultativement substitué par R^{d}, R^{e}, et/ou R^{f}, de préférence R³ est un cyclopropylméthyle, un cyclobutylméthyle, un cyclopentylméthyle, ou un cyclohexylméthyle facultativement substitué par R^{d}, R^{e}, et/ou R^{f} ;
(iv) R³ est un hétéroaralkyle (par exemple, un thiénylméthyle, un furanylméthyle, un pyridinylméthyle, un quinoléinylméthyle, un isoquinoléinylméthyle, un indolylméthyle, ou un indazolylméthyle) facultativement substitué par R^{d}, R^{e}, et/ou R^{f} ; ou
(v) R³ est un hydroxyalkyle, un alcoxyalkyle, un aminoalkyle, de préférence R³ est un hydroxyméthyle, un hydroxyéthyle, un méthoxyméthyle, un méthoxyéthyle, un aminométhyle, ou un aminobutyle.

13. Le composé de l'une quelconque des revendications 1 à 12 dans lequel :
(i) la stéréochimie au niveau du carbone auquel R³ est attaché est (S) ; ou
(ii) la stéréochimie au niveau du carbone auquel R³ est attaché est (R).

14. Une composition pharmaceutique comprenant un composé de l'une quelconque des revendications 1 à 13 ou un sel pharmaceutiquement acceptable de celui-ci, et un excipient pharmaceutiquement acceptable.

15. Le composé ou le sel pharmaceutiquement acceptable de celui-ci tel que revendiqué dans l'une quelconque des revendications 1 à 13 ou la composition pharmaceutique selon la revendication 14 destiné(e) à être utilisé(e) dans le traitement d'une maladie traitable par inhibition de l'activation de NF-kB, dans lequel ou laquelle
(i) la maladie est une maladie inflammatoire, ladite maladie étant facultativement sélectionnée dans le groupe constitué d'une maladie auto-immune, d'une douleur, d'allergies, de l'asthme, de la maladie pulmonaire obstructive chronique et d'une septicémie ; ou
(ii) la maladie est sélectionnée dans le groupe constitué de l'arthrite rhumatoïde, de l'ostéoarthrite, de l'athérosclérose, de la sclérose en plaques, de l'asthme, de la maladie inflammatoire de l'intestin, du diabète, de la maladie de Parkinson, de la maladie d'Alzheimer, de la sclérose latérale amyotrophique, de l'ostéoporose, du lupus érythémateux systémique, de la maladie pulmonaire obstructive chronique, de la fibrose kystique, d'un accident vasculaire cérébral, de l'insuffisance rénale aiguë, de la glomérulonéphrite, du psoriasis, de la dermatite atopique, de la maladie de Behçet, de la tuberculose, de la maladie de Crohn, de la colite, de la maladie de Paget, de la pancréatite, de la parodontite, de la maladie inflammatoire du poumon, et de la néphrite lupique.
